# FASCICULE DE BREVET EUROPEEN

(11) **EP 1 122 254 B1**
(45) Date de publication et mention de la délivrance du brevet: **27.04.2005**
(21) Numéro de dépôt: 01400225.7
(22) Date de dépôt: 30.01.2001
(51) Int. Cl.: C07D 405/14, A61K 31/4525, A61K 31/4709, A61K 31/4725, C07D 401/12, C07D 405/06, C07D 493/04, C07D 495/04, C07D 413/14, C07D 471/04, C07D 417/14, A61P 9/08, A61P 9/12

(54) **Dérivés de 4-sulfonamides pipéridine, leur procédé de préparation et les compositions pharmaceutiques qui les contiennent**
4-Sulfonamid-Piperidin-Derivate, Verfahren zu ihrer Herstellung und sie enthaltende pharmazeutische Zubereitungen
4-Sulfonamide piperidine derivatives, method for their preparation and phamaceutical compositions containing them

(30) Priorité: 31.01.2000 FR 0001171
(43) Date de publication de la demande: 08.08.2001
(73) Titulaire: LES LABORATOIRES SERVIER, 92200 Neuilly sur Seine (FR)
(72) Inventeur: Peglion, Jean-Louis, 78110 Le Vesinet (FR); Dessinges, Aimée, 92500 Rueil Malmaison (FR); Poitevin, Christophe, 75019 Paris (FR); Vilaine, Jean-Paul, 92290 Chatenay Malabry (FR); Villeneuve, Nicole, 92500 Rueil Malmaison (FR); Thollon, Catherine, 75011 Paris (FR); Bourguignon, Marie-Pierre, 78400 Chatou (FR)

(56) Documents cités:
- EP-A- 0 526 342
- WO-A-94/13659
- WO-A-98/24766

## Description

La présente invention concerne de nouveaux dérivés de 4-sulfonamide pipéridine, leur procédé de préparation et les compositions pharmaceutiques qui les contiennent.

Les composés de la présente invention sont utiles dans le traitement des maladies ou des conditions pathologiques dans lesquelles une dysfonction endothéliale est connue comme étant un mécanisme pathogène et/ou aggravant. Ces pathologies sont : l'athérosclérose, l'existence de facteurs de risque vasculaire (dyslipidémie, diabète, hypertension artérielle systémique), les différentes formes cliniques d'ischémie myocardique ou périphérique, l'insuffisance cardiaque et les différentes formes d'hypertension artérielle pulmonaire. Ces composés sont également utiles pour le traitement de patients qui subissent une transplantation cardiaque ou une reperméabilisation vasculaire telle qu'un pontage, une thrombolyse ou une dilatation artérielle avec ou sans stent.

Une diminution de la disponibilité vasculaire en monoxyde d'azote (NO) représente le mécanisme majeur de la dysfonction endothéliale observée dans les maladies et conditions pathologiques précédemment citées et explique son rôle pathogène (*Cardiovasc. Res.,* **1999,** 43, 572 ; *Coronary*. *Art. Dis.* **1999,** 10, 277 ; *Coronary. Art. Dis.,* **1999,** 10, 301 ; *Coronary. Art. Dis.,* **1999,** 10, 287 ; *Coronary. Art. Dis*., **1999,** 10, 295).

En effet, dans ces conditions pathologiques, la dysfonction endothéliale peut résulter de deux mécanismes principaux: 1) une insuffisance de production de NO liée à l'inhibition de la NO synthase endothéliale par des inhibiteurs endogènes tel l'ADMA (asymétrique diméthyle arginine) dont la concentration plasmatique augmente chez des patients présentant des facteurs de risque cardiovasculaire (*Cardiovasc. Res.,* **1999,** 43, 542 ; *Hypertension,* **1997,** 29, 242 ; *Circulation,* **1997,** 95, 2068), 2) une inactivation du NO par l'anion superoxyde (O₂⁻) dont la production est accrue dans des conditions pathologiques (*Cardiovasc. Res.,* **1999,** 43, 562 ; *Eur.J Biochem.* **1997,** 245, 541 ; *J. Clin. Invest.,* **1993,** 91 2546).

Le NO exerce dans les conditions normales des effets majeurs tels que: 1) la régulation de la vasomotricité artérielle par son effet vasodilatateur (*N Engl. J Med*., **1993,** 329, 2002 ; *Nature,* **1980,** 288, 373), 2) la limitation de l'adhésion et de l'agrégation plaquettaire (*Trends Pharmacol. Sci*., **1991,** 12, 87), 3) le contrôle de l'adhésion aux cellules endothéliales de leucocytes et de monocytes (*Proc. Natl Acad. Sci. USA*, **1991,** 88, 4651), 4) l'inhibition de la prolifération des cellules musculaires lisses vasculaires (*Cardiovasc. Res.,* **1999,** 43, 580, *Circulation,* **1993,** 87 V51).
Ceci explique que la déficience en NO au niveau de la paroi artérielle favorise des phénomènes pathologiques comme la vasoconstriction, la thrombose, l'accumulation lipidique et la prolifération des cellules musculaires lisses vasculaires.

Des expériences *in vitro* ont permis de démontrer que les composés de la présente invention permettent de limiter la dysfonction endothéliale et la diminution de disponibilité vasculaire en NO qui ont été induites par des tests impliquant les deux mécanismes physiopathologiques déjà cités : l'inhibition de la NO synthase endothéliale et un stress oxydatif par production d'O₂.

Ainsi, grâce à leur activité pharmacologique spécifique, capable de limiter le développement de la dysfonction endothéliale, les composés de la présente invention, outre le fait qu'ils soient nouveaux, sont utiles pour prévenir le développement, l'extension et les complications des lésions athéroscléreuses notamment chez les patients présentant un facteur de risque vasculaire (dyslipidémie, diabète, hypertension artérielle), pour traiter les différentes formes cliniques d'ischémie myocardique ou périphérique, l'insuffisance cardiaque, les différentes formes d'hypertension artérielle pulmonaire. Ces composés sont également utilisés pour prévenir les complications vasculaires (spasme, thrombose, resténose, athérosclérose accélérée) chez les patients qui subissent un pontage, une dilatation vasculaire avec ou sans stent ou d'autres formes de reperméabilisation vasculaire ainsi qu'une transplantation cardiaque.

Des composés de structure voisine ont été décrits dans la littérature. C'est le cas plus particulièrement de la demande de brevet WO 94/13659 qui revendique notamment des dérivés de benzofuranyl pipéridine, inhibiteurs 5-HT_{1A}. Ces composés sont utiles dans le traitement de désordres psychiques et neurologiques. Ils se distinguent nettement des composés de la présente invention, tant par leur structure chimique et notamment par l'absence de fonction sulfonamide, que par leur propriété pharmacologique.

Le brevet EP 0 526 342 décrit quant à lui des nouvelles (isoquinoléin-5-yl)sulfonamides et revendique ces composés pour leur utilité dans le traitement et la prévention des affections dues à des phénomènes de souffrance tissulaire.

Plus particulièrement, la présente invention concerne les composés de formule (I) : 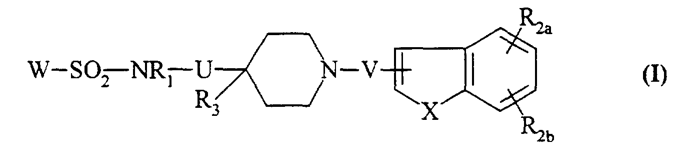 dans laquelle :
- **R**_{**1**}: représente un atome d'hydrogène ou un groupement alkyle (C₁-C₆) linéaire ou ramifié,
- **R**_{**2a**}**, R**_{**2b**}**,**: identiques ou différents, indépendamment l'un de l'autre, représentent chacun un groupement choisi parmi atome d'hydrogène, atome d'halogène, groupement alkyle (C₁-C₆) linéaire ou ramifié, hydroxy, alkoxy (C₁-C₆) linéaire ou ramifié, trihalogénoalkyle (C₁-C₆) linéaire ou ramifié, cyano, nitro, amino, alkylamino (C₁-C₆) linéaire ou ramifié, et dialkylamino (C₁-C₆) linéaire ou ramifié,
- **R**_{**3**}: représente un atome d'hydrogène ou un groupement hydroxy,
- **X**: représente un atome d'oxygène ou un groupement méthylène,
- **V**: représente une chaîne alkylène (C₁-C₆) linéaire ou ramifiée comportant éventuellement une ou plusieurs insaturations et étant éventuellement substituée par un ou plusieurs groupements, identiques ou différents, choisis parmi atomes d'halogène, groupements hydroxy et alkoxy (C₁-C₆) linéaire ou ramifié,
- **U**: représente une liaison ou une chaîne alkylène (C₁-C₆) linéaire ou ramifiée,
- **W**: représente un groupement choisi parmi aryle et hétéroaryle, chacun de ces groupements étant éventuellement substitué par un ou plusieurs groupements, identiques ou différents, choisis parmi atomes d'halogène, groupements alkyle (C₁-C₆) linéaire ou ramifié, hydroxy, alkoxy (C₁-C₆) linéaire ou ramifié, trihalogénoalkyle (C₁-C₆) linéaire ou ramifié, oxo, cyano, nitro, amino, alkylamino (C₁-C₆) linéaire ou ramifié, dialkylamino (C₁-C₆) linéaire ou ramifié, pyridinyle, alkylcarbonyle (C₁-C₆) linéaire ou ramifié, aminocarbonyle (la partie amino étant éventuellement substituée par un ou deux groupements, identiques ou différents, alkyle (C₁-C₆) linéaire ou ramifié), alkoxycarbonyle (C₁-C₆) linéaire ou ramifié, trihalogénoalkylcarbonyle (C₁-C₆) linéaire ou ramifié, alkylsulfonyle (C₁-C₆) linéaire ou ramifié, et trihalogénoalkylsulfonyle (C₁-C₆) linéaire ou ramifié,
leurs isomères, leurs hydrates, leurs solvates ainsi que leurs sels d'addition à un acide ou à une base pharmaceutiquement acceptable,
étant entendu que :
- par groupement aryle, on comprend un groupement choisi parmi phényle, biphényle, naphtyle, dihydronaphtyle, tétrahydronaphtyle, indanyle et indényle,
- par groupement hétéroaryle, on comprend un système monocyclique aromatique ou bicyclique, de 5 à 12 chaînons, comportant de 1 à 3 hétéroatomes, identiques ou différents, choisis parmi oxygène, azote et soufre, et dont l'un des cycles, dans le cas d'un système bicyclique possède un caractère aromatique, l'autre cycle pouvant être aromatique ou partiellement hydrogéné.

Parmi les groupements hétéroaryles, on peut citer, à titre indicatif et non limitatif, le groupement pyridinyle, pyrazolyle, isoxazolyle, dihydroisoxazolyle, quinolyle, isoquinolyle, tétrahydroisoquinolyle, benzofurazannyle, pyrazinyle, pyrimidinyle, pyridazinyle, indolyle, 2,1,3-benzoxodiazolyle, 2,1,3-benzothiadiazolyle, thiéno[2,3-c]pyridinyle ou furo[2,3-c] pyridinyle.

Parmi les bases pharmaceutiquement acceptables, on peut citer à titre non limitatif l'hydroxyde de sodium, l'hydroxyde de potassium, la triéthylamine ou la *tert*butylamine.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutamique, fumarique, tartrique, dibenzoyltartrique, maléïque, citrique, ascorbique, oxalique ou méthane sulfonique, camphorique.

Le substituant X préféré selon l'invention est l'atome d'oxygène.

Les substituants R₁ préférés selon l'invention sont l'atome d'hydrogène et le groupement méthyle.

Selon une variante avantageuse, les composés préférés de l'invention sont les composés de formule **(I/A)** : 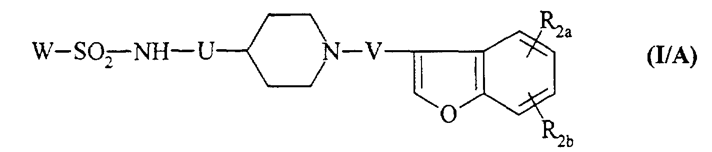 dans laquelle W, U, V, R₂ₐ et R_{2b} sont tels que définis dans la formule (I).

D'une façon avantageuse, le substituant W préféré selon l'invention est le groupement isoquinolin-5-yl. Selon une autre variante de l'invention, le substituant W préféré est le groupement 1,2,3,4-tétrahydro-isoquinolin-5-yl éventuellement substitué en position 2 par un groupement de formule -C(O)-A dans laquelle A représente un groupement choisi parmi alkyle (C₁-C₆) linéaire ou ramifié, amino (lui-même éventuellement substitué par un ou deux groupements, identiques ou différents, alkyle (C₁-C₆) linéaire ou ramifié), alkoxy (C₁-C₆) linéaire ou ramifié, trifluorométhyle, alkylsulfonyle (C₁-C₆) linéaire ou ramifié, et trifluorométhylsulfonyle.

D'une façon particulièrement avantageuse, les composés préférés de l'invention sont les composés de formule (I/A) telle que définie précédemment dans laquelle U, V, R₂ₐ et R_{2b} sont tels que définis dans la formule (I) et W représente un groupement isoquinolin-5-yl.

Selon une autre variante particulièrement avantageuse, les composés préférés de l'invention sont les composés de formule (I/A) telle que définie précédemment dans laquelle U, V, R₂ₐ et R_{2b} sont tels que définis dans la formule (I) et W représente un groupement 1,2,3,4-tétrahydroisoquinolin-5-yl éventuellement substitué en position 2 par un groupement de formule -C(O)A dans laquelle A est tel que défini précédemment.

Les composés préférés de l'invention sont :
- le *N*-{1-[2-(benzofuran-3-yl)éthyl]-4-pipéridinyl}(isoquinolin-5-yl)sulfonamide,
- le 5-{[({{1-[2-(benzofuran-3-yl)éthyl]-4-pipéridinyl}méthyl)-(méthyl)amino]sulfonyl}-3,4-dihydro-2(1*H*)-isoquinolinecarboxylate d'éthyle,
- le *N*-({2-[2-benzofuran-3-yl)éthyl]-4-hydroxy-4-pipéridinyl}éthyl)-5-isoquinoline-sulfonamide,
- le *N*-({1-[2-(benzofuran-3-yl)éthyl]-4-hydroxy-4-pipéridinyl}méthyl)-*N*-méthyl-2,1,3-benzoxadiazole-4-sulfonamide,
- et le 5-{[({1-[2-(benzofuran-3-yl)éthyl]-4-pipéridinyl}méthyl)-(méthyl)amino]sulfonyl}-3,4-dihydro-2(1*H*)-isoquinolinecarboxylate d'isopropyle.

Les isomères ainsi que les hydrates, les solvates et les sels d'addition à un acide ou à une base pharmaceutiquement acceptable des composés préférés font partie intégrante de l'invention.

L'invention s'étend également au procédé de préparation des composés de formule (I) caractérisé en ce qu'on utilise comme produit de départ un composé de formule **(II) :** 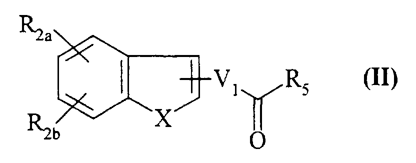 dans laquelle X, R₂ₐ et R_{2b} sont tels que définis dans la formule (I), V₁ représente une liaison ou une chaîne alkylène (C₁-C₅) linéaire ou ramifié comportant éventuellement une ou plusieurs insaturations, R₅ représente un atome d'hydrogène, de chlore, un groupement hydroxy ou alkoxy (C₁-C₆) linéaire ou ramifié,
composés de formule (II) que l'on fait réagir :
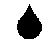 ***soit*** avec un composé de formule **(III/A)** : 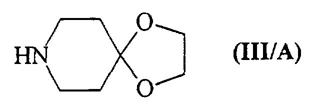 pour conduire aux composés de formule **(IV)** : 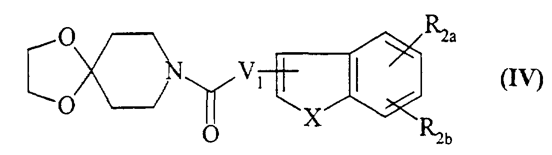 dans laquelle R₂ₐ, R_{2b}, X et V₁ sont tels que définis précédemment,
   composés de formule (IV) dont on déprotège la fonction cétal, selon des techniques classiques de la synthèse organique, puis que l'on fait réagir dans des conditions d'amination réductrice avec un composé de formule **(V)** :

   R'₁ - NH₂ **(V)**

   dans laquelle R'₁ représente un groupement alkyle (C₁-C₆) linéaire ou ramifié,
   pour conduire aux composés de formule **(VI/A)** : 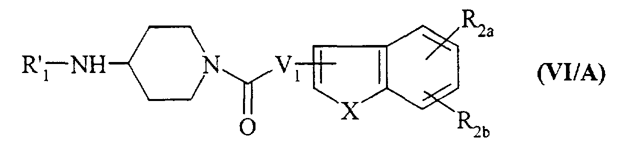 dans laquelle R'₁, R₂ₐ, R_{2b}, X et V₁ ont les mêmes significations que précédemment,
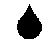 ***soit*** avec un composé de formule **(III/B)** : 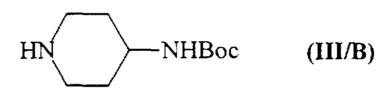 dans laquelle Boc représente un groupement *t*-butyloxycarbonyle,
   pour conduire aux composés de formule **(VII)** : 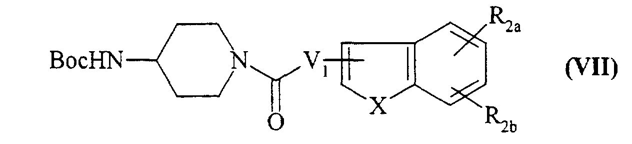 dans laquelle Boc, R₂ₐ, R_{2b}, X et V₁ sont tels que définis précédemment, composés de formule (VII) dont on déprotège la fonction amine terminale pour conduire aux composés de formule **(VI/B)** : 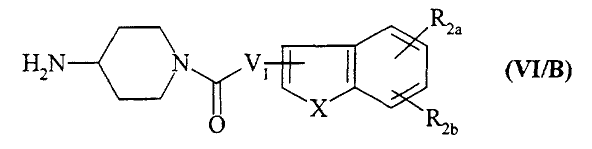 dans laquelle R₂ₐ, R_{2b}, V₁ et X sont tels que définis précédemment, l'ensemble des composés de formule (VI/A) et (VI/B) forment les composés de formule **(VI)** : 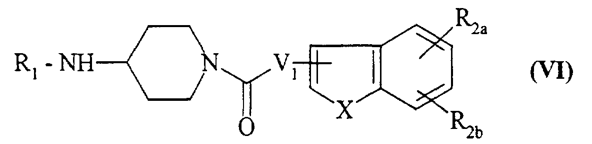 dans laquelle R₁ est tel que défini dans la formule (I) et R₂ₐ, R_{2b}, X et V₁ sont tels que définis précédemment,
   composés de formule (VI) que l'on traite par l'un des agents réducteurs utilisés couramment en synthèse organique pour réduire les fonctions amides, pour conduire aux composés de formule **(VIII)** : 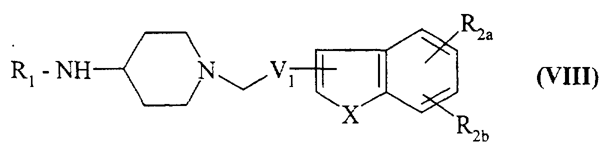 dans laquelle R₁, V₁, X, R₂ₐ et R_{2b} sont tels que définis précédemment,
   composés de formule (VIII) que l'on place en présence d'un composé de formule **(IX)** :

   W - SO₂Cl **(IX)**

   dans laquelle W a la même signification que dans la formule (I), pour conduire aux composés de formule **(I/a)**, cas particulier des composés de formule (I) : 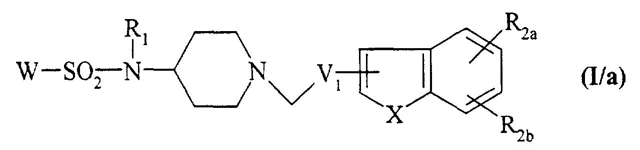 dans laquelle W, R₁, V₁, X, R₂ₐ et R_{2b} sont tels que définis précédemment,
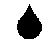 ***soit*** avec un composé de formule **(III/C)** : 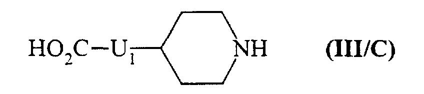 dans laquelle U₁ représente une liaison ou une chaîne alkylène (C₁-C₅) linéaire ou ramifiée, pour conduire aux composés de formule **(X)** : 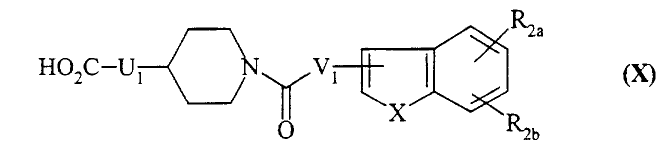 dans laquelle U₁, V₁, X, R₂ₐ et R_{2b} sont tels que définis précédemment,
   composés de formule (X) que l'on traite avec de l'ammoniaque ou avec un dérivé de formule (V) telle que définie précédemment en présence d'un agent de couplage tel que du dicyclohexylcarbodiimide, du carbonyldiimidazole ou du 1,1,1,3,3,3-hexaméthyl-disylazane, pour conduire aux composés de formule **(XI)** : 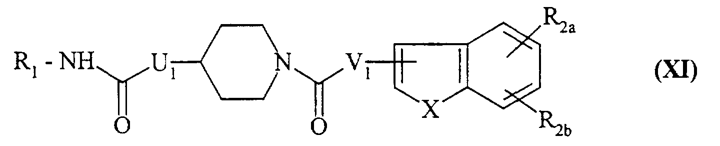 dans laquelle R₁ est tel que défini dans la formule (I), U₁, V₁, X, R₂ₐ et R_{2b} sont tels que définis précédemment,
   composés de formule (XI) que l'on réduit selon des conditions classiques de la synthèse organique, pour conduire aux composés de formule **(XII)** : 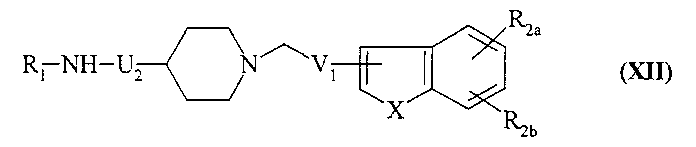 dans laquelle U₂ représente une chaîne alkylène (C₁-C₆) linéaire ou ramifié, R₁, V₁, X, R₂ₐ et R_{2b} sont tels que définis précédemment,
   composés de formule (XII) qui est mis à réagir avec un composé de formule (IX), tel que décrit précédemment, pour conduire aux composés de formule **(I/b)**, cas particulier des composés de formule (I) : 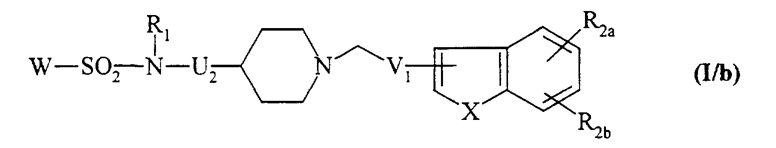 dans laquelle W, R₁, U₂, V₁, X, R₂ₐ et R_{2b} sont tels que définis précédemment,
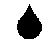 ***ou*** composés de formule (II) dont on réduit le groupement carbonyle terminal substitué par un groupement R'₅ prenant les définitions de R₅ tel que défini précédemment, exceptées la valeur atome de chlore, puis dont on substitue le groupement hydroxy obtenu, par un atome d'halogène tel que Cl, Br, ou I, en utilisant les techniques usuelles de la chimie organique, pour conduire aux composés de formule **(II/B)** : 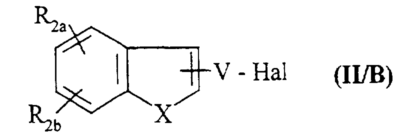 dans laquelle X et V sont tels que définis dans la formule (I) et Hal représente un atome d'halogène,
   composé de formule (II/B) que l'on additionne sur un composé de formule **(III/D)** : 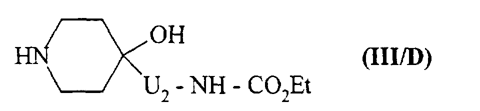 dans laquelle U₂ est tel que défini précédemment,
   pour conduire aux composés de formule **(XIII)** : 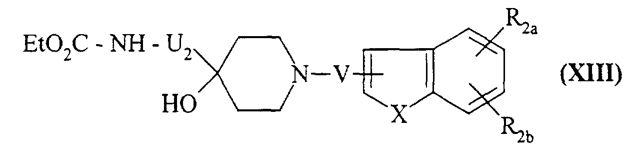 dans laquelle U₂, V, X, R₂ₐ et R_{2b} ont les mêmes significations que précédemment,
   composés de formule (XIII) que l'on traite soit par un agent réducteur, soit par une base forte alcaline, pour conduire aux composés de formule **(XIV)** : 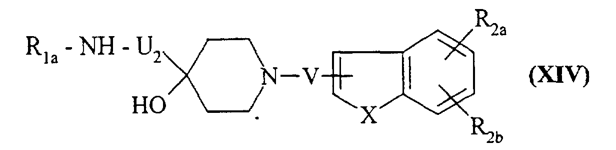 dans laquelle R₁ₐ représente un atome d'hydrogène ou un groupement méthyle, U₂, V, X, R₂ₐ et R_{2b} sont tels que définis précédemment,
   composés de formule (XIV) que l'on met en présence d'un composé de formule (IX), pour conduire aux composés de formule **(I/c)**, cas particulier des composés de formule (I) : 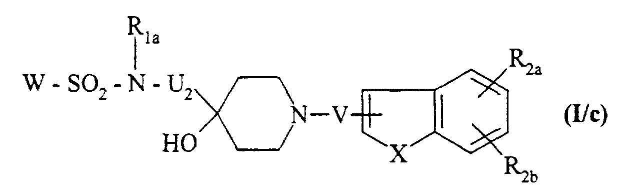 dans laquelle W, R₁ₐ, U₂, V, X, R₂ₐ et R_{2b} ont les mêmes significations que précédemment,
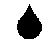 ***ou*** composé de formule (II), dans le cas particulier où R₅ représente un atome d'hydrogène et V₁ prend la valeur V'₁ représentant une chaîne alkylène (C₁-C₄) linéaire ou ramifiée, que l'on traite par de l'iodure de triméthylsulfoxonium en présence d'hydrure de sodium dans le diméthylsulfoxyde, pour conduire aux composés de formule **(II/C)** : 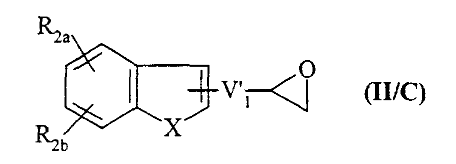 dans laquelle R₂ₐ, R_{2b}, X et V'₁ sont tels que définis précédemment,
   composés de formule (II/C) que l'on fait réagir avec l'un quelconque des composés de formule (III/A), (III/B) ou (III/C), les intermédiaires obtenus étant alors déprotégés et fonctionnalisés selon les méthodes respectives décrites précédemment, pour conduire aux composés de formule **(XV)** : 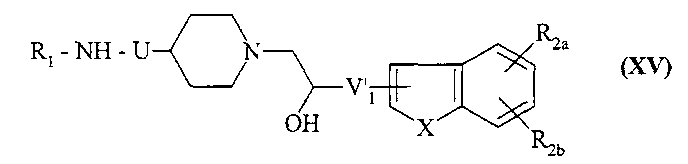 dans laquelle R₁, U, X, R₂ₐ et R_{2b} sont tels que définis précédemment et V'₁ prend la signification décrite précédemment,
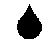 ***ou*** composé de formule (II), dans le cas particulier où il représente un composé de formule **(II/D)** : 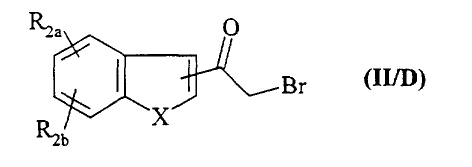 dans laquelle R₂ₐ, R_{2b} et X sont tels que définis dans la formule (I),
   que l'on fait réagir avec l'un quelconque des composés de formule (III/A), (III/B) ou (III/C), les intermédiaires obtenus étant alors déprotégés et fonctionnalisés selon les méthodes respectives décrites précédemment,
   pour conduire aux composés de formule **(XVI)** : 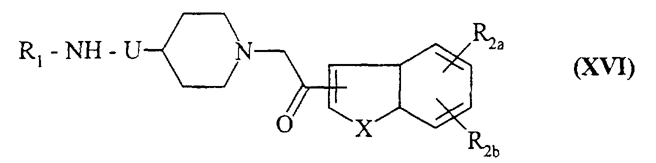 dans laquelle R₁, U, X, R₂ₐ et R_{2b} sont tels que définis précédemment,
   composés de formule (XVI) que l'on réduit de façon classique pour conduire aux composés de formule **(XVII)** : 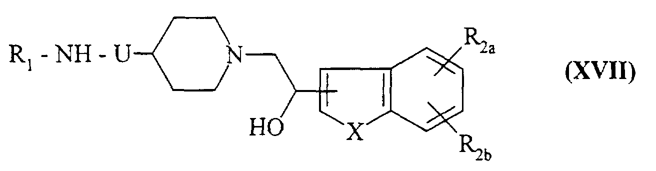 l'ensemble des composés de formule (XV) et (XVII) formant les composés de formule **(XVIII)** : 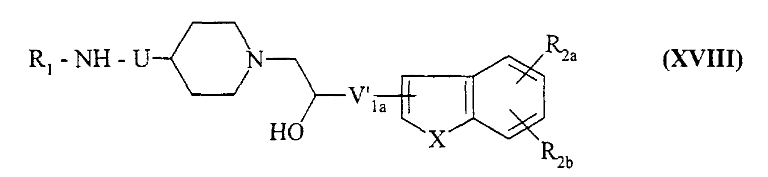 dans laquelle R₁, U, X, R₂ₐ et R_{2b} sont tels que définis précédemment, et V'₁ₐ représente une liaison ou une chaîne alkylène (C₁-C₄) linéaire ou ramifiée,
   composés de formule (XVIII) dont on substitue, si on le souhaite la fonction hydroxy par un atome d'halogène, selon des conditions classiques de la synthèse organique, pour conduire aux composés de formule (XIX) : 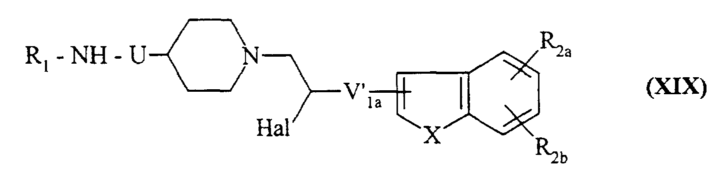 dans laquelle Hal représente un atome d'halogène, et R₁, U, V'₁ₐ, X, R₂ₐ et R_{2b} sont tels que définis précédemment,
   l'ensemble des composés de formule (XVIII) et (XIX) étant mis à réagir avec un composé de formule (IX) tel que décrit précédemment, pour conduire aux composés de formule **(I/d)**, cas particulier des composés de formule (I): 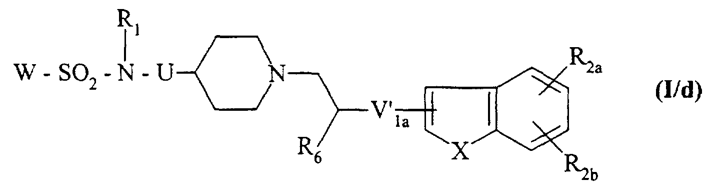 dans laquelle W, R₁, U, X, R₂ₐ et R_{2b} sont tels que définis dans la formule (I), V'₁ₐ est tel que défini précédemment et R₆ représente un groupement hydroxy ou un atome d'halogène,
   composés de formule (I/d), dans le cas particulier où R₆ représente un groupement hydroxy, que l'on soumet à l'action d'un agent alkylant selon des conditions classiques de la chimie organique, pour conduire aux composés de formule (I/e), cas particulier des composés de formule (I) : 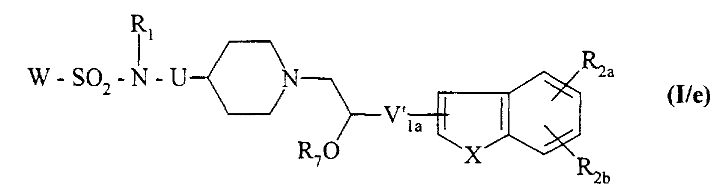 dans laquelle W, R₁, U, V'₁ₐ, X, R₂ₐ et R_{2b} sont tels que définis précédemment et R₇ représente un groupement alkyle (C₁-C₆) linéaire ou ramifié,
   les composés de formule (I/a) à (I/e) formant l'ensemble des composés de l'invention, que l'on purifie, le cas échéant, selon des techniques classiques de purification, qui peuvent, si on le désire, être séparés en leurs différents isomères, selon une technique classique de séparation, dont on module les substituants R₂ₐ, R_{2b} et ceux du groupement W selon des méthodes classiques de la synthèse organique utilisées dans le domaine de la chimie aromatique dont on réduit, si on le souhaite, le groupement W dans le cas où celui-ci est un système bicyclique, pour conduire à des groupements W bicycliques dont l'un des cycles est partiellement hydrogéné, celui-ci pouvant ensuite être substitué selon des conditions classiques de la chimie organique, et que l'on transforme, si on le souhaite, en leurs sels d'addition à un acide ou à une base pharmaceutiquement acceptable, et qui peuvent exister éventuellement sous formes d'hydrates ou de solvates.

Les composés de formule (II), (III/A), (III/B), (III/C), (III/D), (V) et (IX) sont soit des composés commerciaux, soit obtenus selon des méthodes connues et classiques de la synthèse organique.

Les composés de la présente invention sont utiles dans le traitement des maladies ou des conditions pathologiques dans lesquelles une dysfonction endothéliale est connue. De ce fait, grâce à leur activité pharmacologique spécifique, les composés de l'invention sont utiles pour prévenir le développement, l'extension et les complications des lésions athéroscléreuses, dans le traitement de l'ischémie myocardique ou périphérique, de l'insuffisance cardiaque, de l'hypertension artérielle pulmonaire, pour la prévention des complications vasculaires après pontage vasculaire, dilatation vasculaire, reperméabilisation vasculaire et transplantation cardiaque.

La présente invention a également pour objet les compositions pharmaceutiques contenant comme principe actif au moins un composé de formule (I), ses isomères optiques, ses hydrates, ses solvates, ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, ou les gouttes oculaires ou nasales.

La posologie utile varie selon l'âge et le poids du patient, la voie d'administration, la nature et la sévérité de l'affection, et la prise de traitements éventuels associés et s'échelonne de 1 mg à 200 mg en une ou plusieurs prises par jour.

Les exemples suivants illustrent l'invention mais ne la limitent en aucune façon. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire ou spectrométrie de masse).

Les points de fusion ont été déterminés soit à la platine chauffante de Kofler (K.), soit à la platine chauffante sous microscope (M.K.). Lorsque le composé existe sous forme de sel, le point de fusion donné correspond à celui du produit salifié.

### PREPARATION 1 :

### N-({1-[2-(Benzofuran-3-yl)éthyl]-4-pipéridinyl}méthyl)-N-méthylamine

### Stade A : 1-(Benzofuran-3-ylacétyl)-4-pipéridinecarboxylate d'éthyle

A 23,9 g d'acide benzofuran-3-yl acétique dans 250 ml de dichlorométhane sont ajoutés par portions 22 g de carbonyldiimidazole. A la fin du dégagement gazeux, la réaction est agitée 1 heure puis 21 ml de pipéridin-4-yl carboxylate d'éthyle sont ajoutés goutte à goutte. Après 12 heures d'agitation à température ambiante, le milieu réactionnel est lavé à la soude 1N puis à l'acide chlorhydrique 1N. Après décantation et séchage, une concentration sous pression réduite permet d'isoler le produit attendu.

### Stade B : Acide 1-(benzofuran-3-ylacétyl)-4-pipéridinecarboxylique

A une solution de 42,1 g du produit obtenu au stade A dans 200 ml d'éthanol sont ajoutés 200 ml de soude 1N. Après 8 heures d'agitation à température ambiante, l'éthanol est évaporé, le milieu est lavé à l'éther, et la phase aqueuse est acidifiée par de l'acide chlorhydrique 1N. Après extraction au dichlorométhane, décantation et séchage, une concentration sous pression réduite permet d'isoler le produit attendu.

### Stade C : 1-(Benzofuran-3-ylacétyl)-N-méthyl-4-pipéridinecarboxamide

A 33 g du produit obtenu au stade B dans 350 ml de dichlorométhane sont additionnés par portions 19 g de carbonyldiimidazole. A la fin du dégagement gazeux, le milieu réactionnel est agité 1 heure à température ambiante puis on y fait circuler un courant de méthylamine. Après 12 heures d'agitation, le milieu est dilué à l'eau, décanté, lavé à la soude 1N puis à l'acide chlorhydrique 1N, décanté, séché et évaporé permettant d'isoler le produit attendu.

### Stade D : N-({1-[2-(benzofuran-3-yl)éthyl]-4-pipéridinyl}méthyl)-N-méthylamine

A une solution de 28,7 g du produit obtenu au stade C dans 1,5 l de tétrahydrofurane portée à 60°C, sont ajoutés par portions 7,6 g d'hydrure de lithium et d'aluminium. Après 18 heures à reflux, le milieu réactionnel est hydrolysé par addition de 5,24 ml d'eau, 4,2 ml de soude à 20 % puis 19,2 ml d'eau. Après filtration, séchage et concentration sous pression réduite, le produit attendu est isolé. Son chlorhydrate recristallise du méthanol.
***Point de fusion (M.K.) : 270-280°C (décomposition)***

### PREPARATION 2 :

### N-({1-[2-(Benzofuran-2-yl)éthyl]-4-pipéridinyl}méthyl)-N-méthylamine

Le produit est obtenu selon le procédé de la préparation 1, en utilisant au stade A, comme substrat, l'acide benzofuran-2-ylacétique.

### PREPARATION 3 :

### {1-[2-(Benzofuran-3-yl)éthyl]-4-pipéridinyl}-méthylamine

Le produit est obtenu selon le procédé de la préparation 1, en utilisant au stade C un courant d'ammoniac à la place d'un courant de méthylamine.

### PREPARATION 4 :

### N-({1-[2-(1-H-Indèn-3-yl)éthyl]-4-pipéridinyl}méthyl)-N-méthylamine

Le produit est obtenu selon le procédé de la préparation 1, en utilisant au stade A, comme substrat, l'acide 1*H*-indène-3-ylacétique.

### PREPARATION 5 :

### N-({1-[(2E)-3-(Benzofuran-2-yl)-2-propènyl]-4-pipéridinyl}méthyl)-N-méthylamine

### Stade A : Acide 3-(benzofuran-2-yl)acrylique

Un mélange composé de 20 g de benzofuran-2-yl carbaldéhyde, 14,25 g d'acide malonique dans 13,8 ml de pyridine est porté à 100°C jusqu'à la fin du dégagement gazeux. Après refroidissement, le produit attendu est précipité par l'intermédiaire d'acide chlorhydrique 1N. Après filtration, lavage à l'acide chlorhydrique puis à l'eau, et séchage, on isole le produit attendu.

### Stade B : 1-[(2E)-3-(Benzofuran-2-yl)-2-propènoyl]-4-pipéridinecarboxylate d'éthyle

25 g du chlorure d'acide du produit obtenu au stade A (obtenu par action du chlorure de thionyle) sont lentement additionnés à température ambiante sur une solution de 19 g de pipéridine-4-yl carboxylate d'éthyle, 9,75 ml de pyridine dans 250 ml d'acétonitrile. Après 1 heure d'agitation, le milieu réactionnel est concentré, repris à l'acétate d'éthyle, lavé à l'acide chlorhydrique 1N puis à la soude 0,1N, séché et filtré. Une concentration sous pression réduite permet d'isoler le produit attendu.

### Stade C : N-({1-[(2E)-3-(Benzofuran-2-yl)-2-propènyl]-4-pipéridinyl}méthyl)-N méthylamine

Le produit est obtenu selon le procédé de la préparation 1, des stades B à D, en utilisant au stade B, comme substrat, le produit isolé au stade B précédent.

### PREPARATION 6 :

### 1-[2-(Benzofuran-3-yl)éthyl]-N- méthyl-4-pipéridinamine

### Stade A : 8-(Benzofuran-3-ylacétyl)-1,4-dioxa-8-azaspiro[4.5]décane

Le produit est obtenu selon le stade A de la préparation 1 en utilisant comme réactif le 1,4-dioxa-8-azaspiro[4.5]décane.

### Stade B : 1-(Benzofuran-3-ylacétyl)-pipéridine-4-one

A une solution de 5 g du produit obtenu au stade A dans 200 ml d'acétone sont ajoutés 50 ml d'acide chlorhydrique 1N. Après 1 h 30 d'agitation à température ambiante, 50 ml d'acide chlorhydrique 1N sont à nouveau ajoutés et le milieu est chauffé 2 heures à 50°C. Après refroidissement, le milieu réactionnel est neutralisé par addition de 100 ml d'une solution à 10 % de bicarbonate de sodium, puis concentré, repris au dichlorométhane, lavé à l'eau, décanté et séché. Une concentration sous pression réduite permet d'isoler le produit attendu.

### Stade C : 1-(Benzofuran-3-ylacétyl)-N-méthyl-4-pipéridinamine

A une solution de 13,7 g du produit obtenu au stade B dans 130 ml d'isopropanol est ajouté goutte à goutte une solution de méthylamine à 33 % dans l'éthanol. Après 2 heures à 10°C, 3,5 g de soude sont additionnés et l'agitation est maintenu pendant 1 heure à température ambiante. La réaction est alors refroidie à 10°C et 2,8 g de borohydrure de sodium sont additionnés. Après 12 heures d'agitation à température ambiante, le milieu réactionnel est concentré, repris à l'eau et au dichlorométhane, décanté, séché et évaporé. Une chromatographie sur gel de silice (CH₂Cl₂/MeOH/NH₄OH : 95/5/0,5) permet d'isoler le produit attendu.

### Stade D : 1-[2-(Benzofuran-3-yl)éthyl]-N- méthyl-4-pipéridinamine

7,2 g du produit obtenu au stade C sont additionnés sur une suspension de 2 g de LiAlH₄ dans 210 ml de tétrahydrofurane. Après 48 heures à température ambiante, le milieu réactionnel est hydrolysé avec 1,4 ml d'eau, 1,1 ml de soude à 20 % et 5 ml d'eau. Après filtration et concentration, une chromatographie sur gel de silice permet d'isoler le produit attendu.
***Point de fusion (M.K.) :* < *50°C***

### PREPARATION 7 :

### 1-[2-(Benzofuran-3-yl)éthyl]-4-pipéridinamine

### Stade A : 1-[2-(Benzofuran-3-yl)éthyl]-4-pipéridinylcarbamate de tert-butyle

A une solution de 33 g de 2-(benzofuran-3-yl)acétaldéhyde, 41,3 g de 4-pipéridinylcarbamate de *tert*-butyle dans 1,4 l de dichlorométhane sont additionnés successivement 13 ml d'acide acétique, et 61 g de triacétoxyborohydrure de sodium. Après 24 heures d'agitation à température ambiante, une solution de soude à 20 % est ajoutée. Après 10 minutes, le milieu réactionnel est décanté et la phase organique est lavée, séchée et concentrée. Une chromatographie sur gel de silice (cyclohexane/acétate d'éthyle : 30/70) permet d'isoler le produit attendu.

### Stade B : 1-[2-(Benzofuran-3-yl)éthyl]-4-pipéridinamine

A une solution de 21 g du produit obtenu au stade A dans 786 ml d'éthanol sont ajoutés 235 ml d'une solution 2,9 N d'éthanol chlorhydrique. Après 3 heures à 70°C puis 16 heures à température ambiante, le milieu réactionnel est concentré sous pression réduite. Le résidu est repris par 150 ml d'eau. Le produit attendu est précipité par addition de soude de 20 %.
***Point de fusion (K.) : 64-66°C***

### PREPARATION 8 :

### 1-[2-(1H-Indèn-3-yl)éthyl]-4-pipéridinamine

### Stade A : 1-(1H-Indèn-3-ylacétyl)pipéridin-4-one

Le produit est obtenu selon le procédé de la préparation 6, des stades A à B, en utilisant au stade A comme substrat l'acide 1H-indèn-3-ylacétique.

### Stade B : Oxime de 1-(1H-indèn-3-ylacétyl)-4-pipéridinone

Un mélange composé de 11,5 g du produit obtenu au stade A, 12,5 g de chlorhydrate d'hydroxylamine, 13,3 g d'acétate de sodium et 100 ml d'éthanol est porté à reflux 1 heure. Le milieu réactionnel est ensuite filtré, concentré, repris à l'eau, extrait au dichlorométhane. La phase organique est alors séchée puis évaporée permettant d'isoler le produit attendu.
***Point de fusion : 190-192°C***

### Stade C : 1-[2-(1H-Indèn-3-yl)éthyl]-4-pipéridinamine

Sur une suspension de 4 g d'hydrure de lithium et d'aluminium dans 12 ml de tétrahydrofurane est additionnée à température ambiante une solution de 6,8 g du produit obtenu au stade B dans 123 ml de tétrahydrofurane. Après 12 heures d'agitation à température ambiante, on hydrolyse par 2,8 ml d'eau, 2,2 ml de soude à 20 % et 10,1 ml d'eau, puis le milieu réactionnel est filtré et concentré sous pression réduite permettant d'isoler le produit attendu.

### PREPARATION 9 :

### 1-[2-(5-Méthoxy-1H-indèn-3-yl)éthyl]-4-pipéridinamine

Le produit est obtenu selon le procédé de la préparation 8 en utilisant comme substrat au stade A l'acide (5-méthoxy-1*H*-indèn-3-yl)acétique.

### PREPARATION 10 :

### 1-[2-(6-Fluoro-1H-indèn-3-yl)éthyl]-4-pipéridinamine

Le produit est obtenu selon le procédé de la préparation 8 en utilisant comme substrat au stade A l'acide (6-fluoro-1*H*-indèn-3-yl)acétique.

### PREPARATION 11 :

### 1-[3-(Benzofuran-2-yl)propyl]-N- méthyl-4-pipéridinamine

### Stade A : 8-[3-(Benzofuran-2-yl)propyl]-1,4-dioxa-8-azaspiro[4.5]décane

Une solution de 5 g de 3-(benzofuran-2-yl)-1-bromopropane, 3 g de 1,4-dioxa-8-azaspiro[4.5]décane, 5,8 g de carbonate de potassium dans 200 ml d'acétone est portée 24 heures à reflux, puis évaporée à sec. Le résidu est repris à l'acétate d'éthyle, lavé à l'eau, séché et évaporé pour conduire au produit attendu.

### Stade B : 1-[3-(Benzofuran-2-y)propyl]-4-pipéridinone

Une solution de 6 g du produit du stade A, 60 ml d'acide sulfurique à 10 % et 30 ml de tétrahydrofurane est portée à 50°C pendant 12 heures, puis évaporée. Le résidu est repris à l'éther, basifié par de la soude à 20 %, extrait à l'acétate d'éthyle, séché et évaporé permettant d'isoler le produit attendu.

### Stade C : 1-[3-(Benzofuran-2-yl)propyl]-N- méthyl-4-pipéridinamine

Le produit est obtenu selon le procédé du stade C de la préparation 6 en utilisant comme substrat le produit obtenu au stade B précédent.

### PREPARATION 12 :

### 1-[3-(Benzofuran-3-yl)propyl]-4-pipéridinamine

Le produit est obtenu en quatre étapes en utilisant dans la première étape le procédé du stade A de la préparation 11 avec comme substrat le 3-(benzofuran-3-yl)-1-bromopropane, puis le procédé du stade B de la préparation 6, puis le procédé des stades C et D de la préparation 8.

### PREPARATION 13 :

### 1-[(4-Méthoxybenzofuran-3-yl)méthyl]-4-pipéridinamine

### Stade A : 1-(4-Méthoxybenzofuran-3-carbonyl)-4-pipéridinylcarbamate de tert-butyle

A une solution de 4,3 ml de diisopropyléthylamine et 2,75 g de *N*-(4-pipéridinyl)carbamate de *tert*-butyle dans 43 ml de dichlorométhane est additionnée, à 0°C, une solution de 2,9 g de chlorure d'acide 4-méthoxybenzofuran-3-yl carboxylique dans 4 ml de dichlorométhane. Après 12 heures d'agitation à température ambiante, le milieu réactionnel est lavé à l'eau, décanté, séché puis évaporé permettant d'isoler le produit attendu.

### Stade B : 1-(4-Méthoxybenzofuran-3-carbonyl)-4-pipéridinamine

Le produit est obtenu selon le procédé du stade B de la préparation 7 en utilisant comme substrat le produit obtenu au stade A précédent.

### Stade C : 1-[(4- Méthoxybenzofuran-3-yl)-méthyl]-4-pipéridinamine

Le produit est obtenu selon le procédé du stade D de la préparation 8 en utilisant comme substrat le produit obtenu au stade B précédent.

### PREPARATION 14 :

### 1-[(Benzofuran-3-yl)méthyl]-4-pipéridinamine

Le produit est obtenu selon le procédé de la préparation 13, des stades A à C, en utilisant comme substrat au stade A le chlorure d'acide de benzofuran-3-ylcarboxylique.

### PREPARATION 15 :

### 1-[2-(6-Méthoxy-benzofuran-3-yl)éthyl]-4-pipéridinamine

Le produit est obtenu selon le procédé de la préparation 13, des stades A à C, en utilisant comme substrat au stade A l'acide (6-méthoxy-benzofuran-3-yl)acétique.

### PREPARATION 16 :

### 1-[2-(Benzofuran-3-yl)éthyl]-4-[(méthylamino)méthyl]-4-pipéridinol

### Stade A : N-(1-Benzyl-4-hydroxy-4-pipéridinyl)méthyl carbamate d'éthyle

A une solution de 30 g de 1-benzyl-4-aminométhyl-pipéridin-4-ol, 13,8 g de triéthylamine dans 250 ml de dichlorométhane est ajoutée, à 0°C, une solution de 14,75 g de chloroformiate d'éthyle dans 125 ml de dichlorométhane. Après 10 minutes de contact, le milieu réactionnel est lavé à la soude 0,1 N puis séché sur MgSO₄. Une concentration sous pression réduite permet d'isoler le produit attendu.

### Stade B : N-(4-Hydroxy-4-pipéridinyl)méthyl carbamate d'éthyle

Une solution de 47,7 g du produit obtenu au stade A, 51,4 g de formiate d'ammonium, 9,5 g de Palladium sur charbon à 10 % et 1600 ml de méthanol est portée à reflux pendant 1 heure. Après refroidissement et filtration, une concentration sous pression réduite permet d'isoler le produit attendu.

### Stade C : N-({1-[2-(Benzofuran-3-yl)éthyl]-4-hydroxy-4-pipéridinyl}méthyl) carbamate d'éthyle

Un mélange composé de 15 g de 2-(benzofuran-3-yl)-l-bromoéthane, 13,5 g du produit obtenu au stade B, 73,5 g de carbamate de potassium, 21,5 g de bromure de tétrabutylammonium, et 150 ml d'acétonitrile est vigoureusement agité pendant 4 jours à température ambiante. Le milieu réactionnel est ensuite concentré, repris à l'acétate d'éthyle, lavé à l'eau, séché sur MgSO4 puis évaporé. Une chromatographie sur gel de silice du résidu (CH₂Cl₂/EtOH/NH₄OH : 95/5/0,5) permet d'isoler le produit attendu.

### Stade D : 1-[2-(Benzofuran-3-yl)éthyl]-4-[(méthylamino)méthyl]-4-pipéridinol

Sur une suspension de 3,63 g d'hydrure de lithium et d'aluminium dans 20 ml de tétrahydrofurane est ajoutée, à une température du milieu réactionnel de 20°C, une solution de 13,4 g du produit obtenu au stade C dans 130 ml de tétrahydrofurane. Après 9 heures à reflux puis 12 heures à température ambiante, le milieu réactionnel est hydrolysé, filtré puis concentré sous pression réduite permettant d'isoler le produit attendu.

### PREPARATION 17 :

### 4-(Aminométhyl)-1-[2-(benzofuran-3-yl)éthyl]-4-pipéridinol

Une solution composée de 2 g du produit obtenu au stade C de la préparation 16, 0,65 g de potasse, 23 ml d'éthanol et 17 ml d'eau est portée à reflux. Après 24 heures, 0,65 g de potasse est additionnée et le reflux est maintenu pendant 3 jours. Le milieu réactionnel est alors concentré, puis dilué au dichlorométhane, lavé à l'eau, neutralisé, séché puis concentré sous pression réduite permettant d'isoler le produit attendu.
***Point de fusion : 78°C***

### PREPARATION 18 :

### 1-[2-(5-Fluoro-benzofuran-3-yl)éthyl]-4-[(méthylamino)méthyl]-4-pipéridinol

Le produit est obtenu selon le procédé de la préparation 16, des stades A à D, en utilisant au stade C le 2-(5-fluoro-benzofuran-3-yl)-1-bromoéthane.

### PREPARATION 19 :

### 4-(Aminométhyl)-1-[2-(5-fluoro-benzofuran-3-yl)éthyl]-4-pipéridinol

Le produit est obtenu selon le procédé de la préparation 17, en utilisant comme substrat le N-{1-[2-(5-fluoro-benzofuran-3-yl)éthyl]4-hydroxy-4-pipéridinylméthyl}carbarnate d'éthyle qui est préparé comme le produit du stade C de la préparation 16 en partant du 2-(5-fluoro-benzofuran-3-yl)-1-bromoéthane.

### PREPARATION 20 :

### 1-[2-(7-Méthoxy-benzofuran-3-yl)éthyl]-4-[(méthylamino)méthyl]-4-pipéridinol

Le produit est obtenu selon le procédé de la préparation 16, des stades A à D, en utilisant au stade C le 2-(7-méthoxy-benzofuran-3-yl)-1-bromoéthane.

### PREPARATION 21 :

### 4-(Aminométhyl)-1-[2-(5-méthoxy-benzofuran-3-yl)éthyl]-4-pipéridinol

Le produit est obtenu selon le procédé de la préparation 17, en utilisant comme substrat le N-({1-[2-(5-méthoxy-benzofuran-3-yl)éthyl]-4-hydroxy-4-pipéridinyl}méthyl) carbamate d'éthyle qui est préparé selon le procédé de la préparation 16, des stades A à C, en utilisant au stade C le 2-(5-méthyloxy-benzofuran-3-yl)-1-bromoéthane.

### PREPARATION 22 :

### 4-(Aminométhyl)-1-[2-(1H-indèn-3-yl)éthyl]-4-pipéridinol

Le produit est obtenu selon le procédé de la préparation 17, en utilisant comme substrat le N-({1-[2-(1*H*-indèn-3-yl)éthyl]-4-hydroxy-4-pipéridinyl}méthyl) carbamate d'éthyle qui est préparé selon le procédé de la préparation 16, des stades A à C, en utilisant au stade C le 2-(1*H*-indèn-3-yl)-1-bromoéthane.

### PREPARATION 23 :

### 1-[2-(5-Méthoxy-benzofuran-3-yl)éthyl]-4-[(méthylamino)méthyl]-4-pipéridinol

Le produit est obtenu selon le procédé de la préparation 16, des stades A à D, en utilisant au stade C comme substrat le 2-(5-méthoxy-benzofuran-3-yl)-1-bromoéthane.

### PREPARATION 24 :

### 4-(Aminométhyl)-1-[2-(7-méthoxy-benzofuran-3-yl)éthyl]-4-pipéridinol

Le produit est obtenu selon le procédé de la préparation 17, en utilisant comme substrat le produit obtenu au stade C de la préparation 20.

### PREPARATION 25 :

### 1-[2-(1H-Indèn-3-yl)éthyl]-4-[(méthylamino)méthyl]-4-pipéridinol

Le produit est obtenu selon le procédé de la préparation 16, des stades A à D, en utilisant au stade C comme substrat le 2-(1*H*-indèn-3-yl)-1-bromoéthane.

### PREPARATION 26 :

### 4-(2-Aminoéthyl)-1-[2-(benzofuran-3-yl)éthyl]-4-pipéridinol

Le produit est obtenu selon le procédé de la préparation 16, des stades A à D, en utilisant comme substrat au stade A le 4-(2-aminoéthyl)-1-benzyl-4-pipéridinol, puis en appliquant au produit le procédé de la préparation 17.

### PREPARATION 27 :

### 1-[2-(5-Hydroxy-benzofuran-3-yl)éthyl]-4-[(méthylamino)méthyl]-4-pipéridinol

A une solution de 3,23 mmol du produit de la préparation 23 dans 14,3 ml de chloroforme sont additionnés, à une température de -5 à -10°C, 6,46 ml d'une solution 1M de tribromure de bore dans le dichlorométhane. Après 12 heures de réaction à température ambiante, le milieu réactionnel est hydrolysé, basifié avec du bicarbonate de sodium en poudre. Après extraction et purification, le produit attendu est isolé.

### PREPARATION 28 :

### 2-(4-Amino-1-pipéridinyl)-1-(benzofuran-3-yl)-éthanol

### Stade A : 1-[2-(Benzofuran-3-yl)-2-oxoéthyl]-4-pipéridinylcarbamate de tert-butyle

A une suspension de 8,4 g de *N*-(pipérid-4-yl)carbamate de *tert-*butyle, 17,3 g de carbonate de potassium et 80 ml d'acétonitrile sont additionnés 10 g de 1-(benzofuran-3-yl)-2-bromo-éthanone. Après 1 heure 45 de réaction à température ambiante, le milieu réactionnel est dilué à l'eau, extrait à l'acétate d'éthyle, puis séché sur MgSO₄ et concentré sous pression réduite permettant d'obtenir le produit attendu.

### Stade B : 1-[2-(Benzofuran-3-yl)-2-hydroxyéthyl]-4-pipéridinylcarbamate de tert-butyle

A une solution de 7,7 g du produit obtenu au stade A dans 100 ml d'éthanol sont additionnés, par portions, 1,2 g de borohydrure de sodium. Après 1 heure de réaction, la solution est évaporée, reprise à l'eau, extraite à l'acétate d'éthyle, séchée puis concentrée sous pression réduite. Une chromatographie sur gel de silice (dichlorométhane/éthanol : 95/5) permet d'isoler le produit attendu.

### Stade C : 2-(4-Amino-1-pipéridinyl)-1-(benzofuran-3-yl)-éthanol

Le produit est obtenu selon le procédé de la préparation 7 en utilisant comme substrat le produit obtenu au stade B précédent.

### PREPARATION 29 :

### 1-(4-Amino-1-pipéridinyl)-3-(benzofuran-3-yl)-2-propanol

### Stade A : 1-[3-(Benzofuran-3-yl)-2-hydroxypropyl]-4-pipéridinyl carbamate de tert-butyle

Une solution de 2,9 g de 3-(2-oxiranylméthyl)benzofurane, 4 g de 4-pipéridinyl carbamate de tert-butyle et 35 ml d'isopropanol est portée à 80°C pendant 5 heures. Après évaporation à sec, une chromatographie sur gel de silice permet d'isoler le produit attendu.
***Point de fusion (K.) : 118-120°C***

### Stade B : 1-(4-Amino-1-pipéridinyl)-3-(benzofuran-3-yl)-2-propanol

Le produit est obtenu selon le stade B de la préparation 7, en utilisant comme substrat le produit du stade A précédent.

### PREPARATION 30 :

### 1-[3-(Benzofuran-3-yl)-2-fluoropropyl]-4-pipéridinamine

### Stade A : 1-[3-(Benzofuran-3-yl)-2-fluoropropyl]-4-pipéridinylcarbamate de tert-butyle

A une solution de 2 g du produit obtenu au stade A de la préparation 29 dans 25 ml de dichlorométhane est ajoutée, sous argon et à 0°C, goutte à goutte, une solution de 0,85 ml de trifluorure de diéthylaminosulfure dans 10 ml de dichlorométhane. Après 2 heures de réaction à 0°C, 1 heure à température ambiante, le milieu réactionnel est hydrolysé à 0°C par une solution aqueuse de NaHCO₃ à 10 %. Après décantation, séchage et évaporation, une chromatographie sur gel silice (dichlorométhane/éthanol : 95/5) permet d'isoler le produit attendu.
***Point de fusion : 118-120°C***

### Stade B : 1-[3-(Benzofuran-3-yl)-2-fluoropropyl]-4-pipéridinamine

Un courant d'acide chlorhydrique gazeux est mis à circuler au travers d'une solution de 3,64 g du produit obtenu au stade A dans 70 ml d'éthanol. Après 2 heures d'agitation à 50°C, le milieu réactionnel est concentré sous pression réduite. Le résidu est repris à l'eau et à la soude à 20 %, extrait au dichlorométhane, séché puis évaporé permettant d'isoler le produit attendu.

### PREPARATION 31 :

### 1-[2-(Benzofuran-3-yl)-1-(fluorométhyl)éthyl]-4-pipéridinamine

Le produit est obtenu selon le procédé du stade B de la préparation 30 en utilisant comme substrat le premier produit isolé lors de la chromatographie sur gel de silice réalisée au stade A de la préparation 30.

### PREPARATION 32 :

### Chlorure de 7-méthoxy-1H-pyrrolo[2,3-c]pyridine-3-sulfonyle

### Stade A : (2-Méthoxy-3-nitro-4-pyridinyl)acétonitrile

A une solution de 40 g de 2-méthoxy-3-nitropyridine et 48,8 g de 4-chlorophénoxyacétonitrile dans 350 ml de diméthylformamide est additionnée à une température inférieure à -15°C, et en 45 minutes, une solution refroidie à -30°C de 68,8 g de tertiobutylate de potassium dans 250 ml de diméthylformamide. Après 1 h 30 de réaction à une température comprise entre -20 et -10°C, le milieu réactionnel est versé sur 2 1 d'acide chlorhydrique 1N puis la solution est agité 30 minutes à -10°C. Le précipité obtenu est filtré, séché et recristallisé de l'éthanol anhydre permettant d'isoler le produit attendu.
***Point de fusion (K.) : 110-114°C***

### Stade B : 7-Méthoxy-1H-pyrrolo[2,3-c]pyridine

A une solution de 30,8 g du produit obtenu au stade A dans 600 ml d'éthanol anhydre et 150 ml d'acide acétique sont additionnés 3,1 g de Pd/C à 10 %. Le milieu est hydrogéné à température ambiante, sous 4 bars, pendant 6 heures, puis filtré et concentré sous pression réduite. Le résidu est repris par 50 ml d'une solution saturée de bicarbonate de sodium, dilué à l'acétate d'éthyle, amené à pH = 8 par addition de bicarbonate de sodium, décanté, lavé à l'eau, séché, puis concentré sous pression réduite permettant d'obtenir le produit attendu.
***Point de fusion (K.) : 129-132°C***

### Stade C : 3-Bromo-7-méthoxy-1H-pyrrolo[2,3-c]pyridine

A une solution de 16,5 g de produit obtenu au stade B dans 340 ml d'éthanol anhydre sont ajoutés, en 5 minutes, 19,7 g de N-bromo-succinimide. Après 12 heures d'agitation à température ambiante, le milieu réactionnel est concentré sous vide, repris à l'eau et à l'éther, lavé, séché et concentré. Une chromatographie sur gel de silice (dichlorométhane/cyclohexane: 80/20 à 100/0) permet d'isoler le produit attendu.
***Point de fusion (K.) : 130-134°C***

### Stade D : 3-Bromo-1-(tert-butyldiméthyl)silyl-7-méthoxy-1H-pyrrolo[2,3-c] pyridine

A une solution refroidie à -76°C de 7,5 g du produit obtenu au stade C dans 200 ml de tétrahydrofurane sont additionnés 22,7 ml d'une solution 1,6 M de *n*-butyllithium dans le tétrahydrofurane. Après 20 minutes d'agitation à cette température, 5,5 g de chlorure de tert-butyldiméthylsilyle dans 50 ml de tétrahydrofurane sont ajoutés et l'agitation est maintenue 3 heures à -76°C. Après retour à température ambiante, 15 ml d'eau, 200 ml d'une solution saturée de chlorure de sodium et 600 ml d'éther sont additionnés. La solution est décantée, séchée, puis concentrée sous pression réduite. Une chromatographie sur gel de silice (dichlorométhane : 100 %) permet d'isoler le produit attendu.

### Stade E : Chlorure de 7-méthoxy-1H-pyrrolo[2,3-c]pyridine-3-sulfonyle

A une solution refroidie à -80°C de 3 g du produit obtenu au stade D dans 80 ml d'éther sont additionnés 11 ml de *t*-butyllithium. Après 30 minutes d'agitation à cette température, on fait passer un courant de dioxyde de soufre au sein du milieu réactionnel pendant 10 minutes, puis 2,3 g de N-chlorosuccinimide dans 40 ml de tétrahydrofurane sont ajoutés et la réaction est ramenée à température ambiante. Après concentration sous pression réduite et purification du résidu, le produit attendu est isolé.

### PREPARATION 33 :

### Chlorure de 4-bromo-5-isoquinolinesulfonyle

### Stade 1 : 4-Bromo-5-nitro-isoquinoline

Ajouter 11 g de 4-bromoisoquinoline dans 45 ml d'acide sulfurique, puis, au goutte à goutte, une solution de 9,1 g de nitrate de potassium dans 45 ml d'acide sulfurique tout en refroidissant au bain de glace. Le milieu réactionnel est agité à température ambiante pendant 2 h 30, avant d'être versé sur de la glace. Après basification de la solution résultante par de l'ammoniaque, les cristaux sont filtrés, lavés et séchés pour conduire au produit attendu.
***Point de fusion (K) : 176-178°C***

### Stade 2 : 5-Amino-4-bromoisoquinoline

100 ml d'acide chlorhydrique concentré sont ajoutés à une suspension de 16 g du composé obtenu au stade 1 dans 55 ml d'éthanol. Le mélange est refroidi par de la glace et agité pendant que l'on introduit une solution de 62,6 g de SnCl₂.2H₂O dans 100 ml d'éthanol, puis encore pendant 3 heures. Après évaporation du solvant, le résidu est dilué avec 140 ml d'eau glacée et basifié par de la soude 2N. La phase aqueuse résultante est extraite par 3 fois 350 ml de chlorure de méthylène. Les phases organiques réunies sont séchées sur MgSO₄ et évaporées. Le résidu est purifié par chromatographie flash (CH₂Cl₂/MeOH : 98/2) pour conduire au produit attendu.
***Point de fusion (K) : 151-153°C***

### Stade 3 : Chlorure de 4-bromo-5-isoquinolinesulfonyle

Sur une suspension de 5,4 g du produit obtenu au stade 2 dans 66 ml d'acide chlorhydrique concentré, on ajoute, au goutte à goutte à -5°C, une solution de 3,45 g de nitrite de sodium dans 14 ml d'eau et la solution est agitée 30 minutes à température ambiante (solution A). Séparément, on prépare une solution aqueuse (8,5 ml) de 1,95 g de CuCl₂.2H₂O que l'on introduit dans 42 ml d'une solution d'acide acétique saturée en anhydride sulfureux.
La solution résultante est coulée au goutte à goutte dans la solution A, puis le milieu réactionnel est agité une heure à température ambiante avant d'être tiédi par un bain-marie à 30°C pendant 30 minutes. Après extraction par du chloroforme, décantation, lavage par une solution saturée de bicarbonate de sodium et traitement habituel, on isole le produit attendu.
***Point de fusion (K) : 84-86°C***

### PREPARATION 34 :

### Chlorure de 4-fluoro-5-isoquinolinesulfonyle

Le produit est obtenu selon le procédé de la préparation 33, des stades 1 à 3, mais en utilisant au stade 1 de cette préparation la 4-fluoroisoquinoline dont la préparation est décrite dans J. Am. Chem. Soc., *1951,* pp. 687-688, à la place de la 4-bromoisoquinoline.
***Point de fusion (K) : 88-89°C***

### EXEMPLE 1 :

### N-({1-[2-(Benzofuran-3-yl)éthyl]-4-pipéridinyl}méthyl)-N-méthyl-5-isoquinolinesulfonamide et son dichlorhydrate

A une solution refroidie à 0°C de 5,26 g du produit de la préparation 1, 5,5 g de diisopropyléthylamine dans 750 ml de dichlorométhane sont ajoutés par fraction 5,1 g du chlorhydrate du chlorure de 5-isoquinolinesulfonyle. Après 6 heures de réaction à température ambiante, le milieu est concentré sous pression réduite, repris à l'acétate d'éthyle, lavé à l'eau, séché et évaporé. Une chromatographie sur gel de silice (dichlorométhane/méthanol : 95/5) permet d'isoler le produit attendu qui est transformé en dichlorhydrate par l'intermédiaire d'une solution d'éther chlorhydrique.
***Point de fusion : 237-239°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***57,77*** | ***5,79*** | ***7,99*** | ***13,68*** | ***5,65*** |
| ***% calculé*** | ***58,21*** | ***5,82*** | ***7,83*** | ***13,22*** | ***5,98*** |

### EXEMPLE 2 :

### 2-Acétyl-8-chloro-N-({1-[2-(benzofuran-3-yl)éthyl]-4-pipéridinyl}méthyl)-N-méthyl-1,2,3,4-tétrahydro-5-isoquinolinesulfonamide et son fumarate

Le produit est obtenu selon le procédé de l'exemple 1 en utilisant comme réactif le chlorure de 2-acétyl-8-chloro-1,2,3,4-tétrahydro-5-isoquinolinesulfonyle. Le produit obtenu est transformé en son fumarate par l'intermédiaire d'une solution 0,172 M d'acide fumarique dans l'éthanol.
***Point de fusion (M.K.) : 168-170°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***57,77*** | ***5,86*** | ***6,21*** | ***5,35*** | ***4,36*** |
| ***% calculé*** | ***58,22*** | ***5,80*** | ***6,36*** | ***5,37*** | ***4,86*** |

### EXEMPLE 3 :

### N-({1-[2-(Benzofuran-2-yl)éthyl]-4-pipéridinyl}méthyl)-N-méthyl-5-isoquinolinesulfonamide

Le produit est obtenu selon le procédé de l'exemple 1 en utilisant comme substrat le composé de la préparation 2 au lieu de celui de la préparation 1.
***Point de fusion (M.K.) : 105-108°C***

| ***Microanalyse élémentaire :*** | | | | |
|---|---|---|---|---|
| | ***C*** | ***H*** | ***N*** | ***S*** |
| ***% trouvé*** | ***67,48*** | ***6,47*** | ***9,14*** | ***6,68*** |
| ***% calculé*** | ***67,36*** | ***6,30*** | ***9,06*** | ***6,92*** |

### EXEMPLE 4 :

### N-({1-[2-(Benzofuran-3-yl)éthyl]-4-pipéridinyl}méthyl)-5-isoquinolinesulfonamide et son fumarate

Le produit est obtenu selon le procédé de l'exemple 1 en utilisant comme substrat le composé de la préparation 3 au lieu de celui de la préparation 1. Le produit obtenu est transformé en son fumarate.
***Point de fusion (M.K.) : 140-155°C***

| ***Microanalyse élémentaire :*** | | | | |
|---|---|---|---|---|
| | ***C*** | ***H*** | ***N*** | ***S*** |
| ***% trouvé*** | ***61,29*** | ***5,63*** | ***7,27*** | ***5,31*** |
| ***% calculé*** | ***61,58*** | ***5,52*** | ***7,43*** | ***5,67*** |

### EXEMPLE 5 :

### N-({1-[2-(1H-Indèn-3-yl)éthyl]-4-pipéridinyl}méthyl)-N-méthyl-5-isoquinolinesulfonamide

Le produit est obtenu selon le procédé de l'exemple 1 en utilisant comme substrat le composé de la préparation 4 au lieu de celui de la préparation 1. Le produit est cristallisé de l'éther isopropylique.
***Point de fusion (M.K.) : 92-95°C***

| ***Microanalyse élémentaire :*** | | | | |
|---|---|---|---|---|
| | ***C*** | ***H*** | ***N*** | ***S*** |
| ***% trouvé*** | ***70*,*30*** | ***6*,*70*** | ***9*,*17*** | ***6,45*** |
| ***% calculé*** | ***70*,*25*** | ***6*,*77*** | ***9*,*10*** | ***6,95*** |

### EXEMPLE 6 :

### N-({1-[(2 E)-3-(Benzofuran-2-yl)-2-propényl]-4-pipéridinyl}méthyl)-N-méthyl-5-isoquinolinesulfonamide

Le produit est obtenu selon le procédé de l'exemple 1 en utilisant comme substrat le composé de la préparation 5 au lieu de celui de la préparation 1. Le produit recristallise de l'acétonitrile.
***Point de fusion (K.) : 109°C***

| ***Microanalyse élémentaire :*** | | | | |
|---|---|---|---|---|
| | ***C*** | ***H*** | ***N*** | ***S*** |
| ***% trouvé*** | ***68*,*57*** | ***6*,*23*** | ***8*,*95*** | ***6,57*** |
| ***% calculé*** | ***68*,*19*** | ***6*,*15*** | ***8*,*83*** | ***6,74*** |

### EXEMPLE 7 :

### N-{1-[2-(Benzofuran-3-yl)éthyl]-4-pipéridinyl}-N-méthyl-5-isoquinolinesulfonamide et son hémi-fumarate

Le produit est obtenu selon le procédé de l'exemple 1 en utilisant comme substrat le composé de la préparation 6 au lieu de celui de la préparation 1. Le produit est transformé en son hémi-fumarate qui recristallise de l'acétate d'éthyle.
***Point de fusion (M.K.) : 122-124°C***

| ***Microanalyse élémentaire :*** | | | | |
|---|---|---|---|---|
| | ***C*** | ***H*** | ***N*** | ***S*** |
| ***% trouvé*** | ***63*,*30*** | ***5*,*71*** | ***8*,*00*** | ***6,43*** |
| ***% calculé*** | ***63*,*88*** | ***5*,*77*** | ***8*,*29*** | ***6,32*** |

### EXEMPLE 8 :

### N-{1-[2-(Benzofuran-3-yl)éthyl]-4-pipéridinyl}-5-isoquinolinesulfonamide et son fumarate

Le produit est obtenu selon le procédé de l'exemple 1 en utilisant comme substrat le composé de la préparation 7 au lieu de celui de la préparation 1. Le produit est transformé en son fumarate.
***Point de fusion (M.K.) : 230-235°C***

| ***Microanalyse élémentaire :*** | | | | |
|---|---|---|---|---|
| | ***C*** | ***H*** | ***N*** | ***S*** |
| ***% trouvé*** | ***60,90*** | ***5,17*** | ***7,50*** | ***6,08*** |
| ***% calculé*** | ***60,97*** | ***5,30*** | ***7,62*** | ***5,81*** |

### EXEMPLE 9 :

### N-{1-[2-(Benzofuran-3-yl)éthyl]-4-pipéridinyl}-2,1,3-benzoxadiazole-4-sulfonamide et son fumarate

A une solution placée à 0°C de 3,5 g du produit de la préparation 7 et 1,85 g de diisopropyléthylamine dans 35 ml de dichlorométhane sont additionnés 3,13 g du chlorure d'acide benzo[2,1,3]oxadiazole-4-sulfonique. Après 3 heures à température ambiante, le milieu réactionnel est lavé à l'eau, séché et évaporé. Le résidu est repris par une solution 0,172 M d'acide fumarique dans l'éthanol. Le précipité obtenu est filtré. Il recristallise de l'éthanol pour conduire au produit attendu.
***Point de fusion (M.K.) : 200-210°C***

| ***Microanalyse élémentaire :*** | | | | |
|---|---|---|---|---|
| | ***C*** | ***H*** | ***N*** | ***S*** |
| ***% trouvé*** | ***55,00*** | ***4,96*** | ***10,15*** | ***6,19*** |
| ***% calculé*** | ***55,34*** | ***4,83*** | ***10,33*** | ***5,91*** |

### EXEMPLE 10 :

### N-{1-[2-(Benzofuran-3-yl)éthyl]-4-pipéridinyl}-1-naphtylsulfonamide et son fumarate

Le produit est obtenu selon le procédé de l'exemple 8 en utilisant comme réactif le chlorure de naphtalène-1-sulfonyle à la place du chlorure de 5-isoquinolinesulfonyle. Le produit est transformé en son fumarate.
***Point de fusion (M.K.) : 226-230°C***

| ***Microanalyse élémentaire :*** | | | | |
|---|---|---|---|---|
| | ***C*** | ***H*** | ***N*** | ***S*** |
| ***% trouvé*** | ***63,04*** | ***5,50*** | ***5,10*** | ***5,67*** |
| ***% calculé*** | ***63,26*** | ***5,49*** | ***5,09*** | ***5,82*** |

### EXEMPLE 11 :

### N-{1-[2-(Benzofuran-3-yl)éthyl]-4-pipéridinyl}-5-chloronaphtyl-1-sulfonamide

Le produit est obtenu selon le procédé de l'exemple 8 en utilisant comme réactif le chlorure de 5-chloro-naphtalène-1-sulfonyle à la place du chlorure de 5-isoquinolinsulfonyle.
***Point de fusion (M.K.) : 107-110°C***

| ***Microanalyse élémentaire :*** | | | | | |
|---|---|---|---|---|---|
| | ***C*** | ***H*** | ***N*** | ***S*** | ***Cl*** |
| ***% trouvé*** | ***64,16*** | ***5,38*** | ***6,15*** | ***6,72*** | ***7,49*** |
| ***% calculé*** | ***64,02*** | ***5,37*** | ***5,97*** | ***6,84*** | ***7,48*** |

### EXEMPLE 12 :

### N-{1-[2-(Benzofuran-3-yl)éthyl]-4-pipéridinyl}-7-méthoxynaphtyl-1-sulfonamide

Le produit est obtenu selon le procédé de l'exemple 8 en utilisant comme réactif le chlorure de 7-métoxynaphtalène-1-sulfonyle à la place du chlorure de 5-isoquinolinesulfonyle. Le produit cristallise dans un mélange éther/heptane.
***Point de fusion (M.K.) : 140-145°C***

| ***Microanalyse élémentaire :*** | | | | |
|---|---|---|---|---|
| | ***C*** | ***H*** | ***N*** | ***S*** |
| ***% trouvé*** | ***66*,*99*** | ***6*,*24*** | ***5*,*92*** | ***6*,*73*** |
| ***% calculé*** | ***67*,*22*** | ***6*,*07*** | ***6*,*03*** | ***6,90*** |

### EXEMPLE 13 :

### N-{1-[2-(Benzofuran-3-yl)éthyl]-4-pipéridinyl}-7-chlorofuro[2,3-c]pyridine-3-sulfonamide et son fumarate

Le produit est obtenu selon le procédé de l'exemple 8 en utilisant comme réactif le chlorure de 7-chlorofuro[2,3-c]pyridine-3-sulfonyle à la place du chlorure de 5-isoquinolinesulfonyle. Le produit est transformé en son fumarate.
***Point de fusion (M.K.) : 185-195°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***54,94*** | ***4,82*** | ***7,70*** | ***6,52*** | ***5,90*** |
| ***% calculé*** | ***55,04*** | ***4,62*** | ***7,76*** | ***6,55*** | ***5,92*** |

### EXEMPLE 14 :

### 1-Chloro-N-{1-[2-(benzofuran-3-yl)éthyl]-4-pipéridinyl}-5-isoquinolinesulfonamide

Le produit est obtenu selon le procédé de l'exemple 8 en utilisant comme réactif le chlorure de 1-chloro-isoquinoline-5-sulfonyle à la place du chlorure de 5-isoquinolinesulfonyle.
***Point de fusion (M.K.) : 130-132°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***61,44*** | ***5,19*** | ***9,00*** | ***7,57*** | ***6,69*** |
| ***% calculé*** | ***61,33*** | ***5,15*** | ***8,94*** | ***7,54*** | ***6,82*** |

### EXEMPLE 15 :

### N-{1-[2-(Benzofuran-3-yl)éthyl]-4-pipéridinyl}-7-chlorothiéno[2,3-c]pyridine-3-sulfonamide et son fumarate

Le produit est obtenu selon le procédé de l'exemple 8 en utilisant comme réactif le chlorure de 7-chlorothiéno[2,3-c]pyridine-3-sulfonyle à la place du chlorure de 5-isoquinolinesulfonyle.
***Point de fusion (M.K.) : 170-173°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***52,58*** | ***4,41*** | ***6,97*** | ***6,21*** | ***11,13*** |
| ***% calculé*** | ***52,74*** | ***4,43*** | ***7,10*** | ***5,99*** | ***10,83*** |

### EXEMPLE 16 :

### 1-Ethoxy-N-{1-[2-(benzofuran-3-yl)éthyl]-4-pipéridinyl}-5-isoquinolinesulfonamide

Le produit est obtenu selon le procédé de l'exemple 8 en utilisant comme réactif le chlorure de 1-éthoxy-5-isoquinolinesulfonyle à la place du chlorure de 5-isoquinolinesulfonyle.
***Point de fusion (M.K.) : 109-114°C***

| ***Microanalyse élémentaire :*** | | | | |
|---|---|---|---|---|
| | ***C*** | ***H*** | ***N*** | ***S*** |
| ***% trouvé*** | ***65,10*** | ***6,35*** | ***8,66*** | ***6,72*** |
| ***% calculé*** | ***65,11*** | ***6,09*** | ***8,76*** | ***6,69*** |

### EXEMPLE 17 :

### 1-Méthoxy-N-{1-[2-(benzofuran-3-yl)éthyl]-4-pipéridinyl}-5-isoquinolinesulfonamide

Le produit est obtenu selon le procédé de l'exemple 8 en utilisant comme réactif le chlorure de 1-méthoxy-5-isoquinolinesulfonyle à la place du chlorure de 5-isoquinolylesulfonyle.
***Point de fusion (M.K.) : 124-128°C***

| ***Microanalyse élémentaire :*** | | | | |
|---|---|---|---|---|
| | ***C*** | ***H*** | ***N*** | ***S*** |
| ***% trouvé*** | ***64,43*** | ***6,03*** | ***9,07*** | ***6,99*** |
| ***% calculé*** | ***64,50*** | ***5,85*** | ***9,03*** | ***6,89*** |

### EXEMPLE 18 :

### N-{1-[2-(Benzofuran-3-yl)éthyl]-4-pipéridinyl}-5-chloro-1,3-diméthyl-1-H-pyrazole-4-sulfonamide et son chlorhydrate

Le produit est obtenu selon le procédé de l'exemple 8 en utilisant comme réactif le chlorure de 5-chloro-1,3-diméthyl-1*H*-pyrrazole-4-sulfonyle à la place du chlorure de 5-isoquinolinesulfonyle.
***Point de fusion (M.K.) : 205-210°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***50,57*** | ***5,61*** | ***11,50*** | ***15,40*** | ***6,95*** |
| ***% calculé*** | ***50,74*** | ***5,54*** | ***11,83*** | ***14,98*** | ***6,77*** |

### EXEMPLE 19 :

### N-{1-[2-(Benzofuran-3-yl)éthyl]-4-pipéridinyl}-3,5-diméthyl-4-isoxazolesulfonamide et son chlorhydrate

Le produit est obtenu selon le procédé de l'exemple 8 en utilisant comme réactif le chlorure de 3,5-diméthyl-4-isoxazolesulfonyle à la place du chlorure de 5-isoquinolinesulfonyle.
***Point de fusion (M.K.) : 266-268°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***54,59*** | ***6,06*** | ***9,31*** | ***8,28*** | ***7,55*** |
| ***% calculé*** | ***54,60*** | ***5,96*** | ***9,55*** | ***8,06*** | ***7,29*** |

### EXEMPLE 20 :

### N-{1-[2-(Benzofuran-3-yl)éthyl]-4-pipéridinyl}-7-oxo-6,7-dihydro-1H-pyrrolo [2,3-c]pyridine-3-sulfonamide

Le produit est obtenu selon le procédé de l'exemple 8 en utilisant comme réactif le produit de la préparation 32. à la place du chlorure de 5-isoquinolinesulfonyle.
***Point de fusion (M.K.) : 251-255°C***

| ***Microanalyse élémentaire :*** | | | | |
|---|---|---|---|---|
| | ***C*** | ***H*** | ***N*** | ***S*** |
| ***% trouvé*** | ***59,70*** | ***5,44*** | ***12,41*** | ***7,18*** |
| ***% calculé*** | ***59,98*** | ***5,49*** | ***12,72*** | ***7,28*** |

### EXEMPLE 21 :

### N-{1-[2-(1H-Indèn-3-yl)éthyl]-4-pipéridinyl}-5-isoquinolinesulfonamide

Le produit est obtenu selon le procédé de l'exemple 1 en utilisant comme substrat le composé de la préparation 8 au lieu de celui de la préparation 1.
***Point de fusion (M.K.) : 170-172°C***

| ***Microanalyse élémentaire :*** | | | | |
|---|---|---|---|---|
| | ***C*** | ***H*** | ***N*** | ***S*** |
| ***% trouvé*** | ***69*,*28*** | ***6*,*32*** | ***9*,*68*** | ***7,45*** |
| ***% calculé*** | ***69*,*26*** | ***6*,*28*** | ***9*,*69*** | ***7,40*** |

### EXEMPLE 22 :

### N-{1-[2-(5-Méthoxy-1H-indèn-3-yl)éthyl]-4-pipéridinyl}-5-isoquinolinesulfonamide et son fumarate

Le produit est obtenu selon le procédé de l'exemple 1 en utilisant comme substrat le composé de la préparation 9 au lieu de celui de la préparation 1.
***Point de fusion (M.K.) : 165-170°C***

| ***Microanalyse élémentaire :*** | | | | |
|---|---|---|---|---|
| | ***C*** | ***H*** | ***N*** | ***S*** |
| ***% trouvé*** | ***61,93*** | ***5,75*** | ***7,10*** | ***5,53*** |
| ***% calculé*** | ***62,16*** | ***5,74*** | ***7,25*** | ***5,53*** |

### EXEMPLE 23 :

### N-{1-[2-(7-Fluoro-1H-indèn-3-yl)éthyl]-4-pipéridinyl}-5-isoquinolinesulfonamide et son fumarate

Le produit est obtenu selon le procédé de l'exemple 1 en utilisant comme substrat le composé de la préparation 10 au lieu de celui de la préparation 1.
***Point de fusion (M.K.) : 215-222°C***

| ***Microanalyse élémentaire :*** | | | | |
|---|---|---|---|---|
| | ***C*** | ***H*** | ***N*** | ***S*** |
| ***% trouvé*** | ***61,36*** | ***5,43*** | ***7,18*** | ***5,61*** |
| ***% calculé*** | ***61,36*** | ***5,33*** | ***7,40*** | ***5,65*** |

### EXEMPLE 24 :

### N-{1-[3-(Benzofuran-2-yl)propyl]-4-pipéridinyl}-N-méthyl-5-isoquinolinesulfonamide et son fumarate

Le produit est obtenu selon le procédé de l'exemple 1 en utilisant comme substrat le composé de la préparation 11 au lieu de celui de la préparation 1.
***Point de fusion (M.K.) : 146-148°C***

| ***Microanalyse élémentaire :*** | | | | |
|---|---|---|---|---|
| | ***C*** | ***H*** | ***N*** | ***S*** |
| ***% trouvé*** | ***62,23*** | ***5,86*** | ***6,99*** | ***5,23*** |
| ***% calculé*** | ***62,16*** | ***5,74*** | ***7,25*** | ***5,53*** |

### EXEMPLE 25 :

### N-{1-[3-(Benzofuran-3-yl)propyl]-4-pipéridinyl}-5-isoquinolinesulfonamide

Le produit est obtenu selon le procédé de l'exemple 1 en utilisant comme substrat le composé de la préparation 12 au lieu de celui de la préparation 1.
***Point de fusion (M.K.) : 115-118°C***

| ***Microanalyse élémentaire :*** | | | | |
|---|---|---|---|---|
| | ***C*** | ***H*** | ***N*** | ***S*** |
| ***% trouvé*** | ***66,79*** | ***6,05*** | ***9,35*** | ***7,13*** |
| ***% calculé*** | ***66,20*** | ***6,27*** | ***9,28*** | ***6,91*** |

### EXEMPLE 26 :

### N-{1-[(4-Méthoxy-benzofuran-3-yl)méthyl]-4-pipéridinyl}-5-isoquinolinesulfonamide et son hémi-fumarate

Le produit est obtenu selon le procédé de l'exemple 1 en utilisant comme substrat le composé de la préparation 13 au lieu de la préparation 1.
***Point de fusion (M.K.) : 204-219°C***

| ***Microanalyse élémentaire :*** | | | | |
|---|---|---|---|---|
| | ***C*** | ***H*** | ***N*** | ***S*** |
| ***% trouvé*** | ***60,82*** | ***5,44*** | ***7,95*** | ***6,00*** |
| ***% calculé*** | ***61,28*** | ***5,35*** | ***8,25*** | ***6,29*** |

### EXEMPLE 27 :

### N-{1-[(Benzofuran-3-yl)méthyl]-4-pipéridinyl}-5-isoquinolinesulfonamide et son fumarate

Le produit est obtenu selon le procédé de l'exemple 1 en utilisant comme substrat le composé de la préparation 14 au lieu de celui de la préparation 1.
***Point de fusion (M.K.) : 203-208°C***

| ***Microanalyse élémentaire :*** | | | | |
|---|---|---|---|---|
| | ***C*** | ***H*** | ***N*** | ***S*** |
| ***% trouvé*** | ***59*,*40*** | ***5*,*12*** | ***7*,*80*** | ***5,48*** |
| ***% calculé*** | ***59*,*54*** | ***5*,*10*** | ***7*,*49*** | ***5,72*** |

### EXEMPLE 28 :

### N-{1-[2-(6-Méthoxy-benzofuran-3-yl)éthyl]-4-pipéridinyl}-5-isoquinolinesulfonamide et son fumarate

Le produit est obtenu selon le procédé de l'exemple 1 en utilisant comme substrat le composé de la préparation 15 au lieu de celui de la préparation 1.
***Point de fusion (M.K.) : 174-178°C***

| ***Microanalyse élémentaire :*** | | | | |
|---|---|---|---|---|
| | ***C%*** | ***H%*** | ***N%*** | ***S%*** |
| ***% trouvé*** | ***59*,*64*** | ***5*,*55*** | ***7*,*16*** | ***5,32*** |
| ***% calculé*** | ***59*,*89*** | ***5*,*37*** | ***7*,*22*** | ***5,51*** |

### EXEMPLE 29 :

### 1-Méthoxy-N-{1-[2-(6-méthoxy-benzofuran-3-yl)éthyl]-4-pipéridinyl}-5-isoquinolinesulfonamide et son fumarate

Le produit est obtenu selon le procédé de l'exemple 1 en utilisant comme substrat le composé de la préparation 15 au lieu de celui de la préparation 1, et comme réactif le chlorure de 1-chloroisoquinoline-5-sulfonyle. Le produit obtenu après chromatographie sur gel de silice est traité par de la potasse méthanolique pour conduire au produit attendu.
***Point de fusion (M.K.) : 194-196°C***

| ***Microanalyse élémentaire :*** | | | | |
|---|---|---|---|---|
| | ***C*** | ***H*** | ***N*** | ***S*** |
| ***% trouvé*** | ***58*,*99*** | ***5*,*54*** | ***6*,*95*** | ***5,08*** |
| ***% calculé*** | ***58*,*91*** | ***5*,*44*** | ***6*,*87*** | ***5,24*** |

### EXEMPLE 30 :

### N-({1-[2-(Benzofuran-3-yl)éthyl]-4-hydroxy-4-pipéridinyl}méthyl)-N-méthyl-5-isoquinolinesulfonamide

Le produit est obtenu selon le procédé de l'exemple 1 en utilisant comme substrat le composé de la préparation 16 au lieu de celui de la préparation 1.
***Point de fusion (M.K.) : 115-120°C***

| ***Microanalyse élémentaire :*** | | | | |
|---|---|---|---|---|
| | ***C*** | ***H*** | ***N*** | ***S*** |
| ***% trouvé*** | ***64,76*** | ***6,03*** | ***8,60*** | ***6,85*** |
| ***% calculé*** | ***65,11*** | ***6,09*** | ***8,76*** | ***6,69*** |

### EXEMPLE 31 :

### N-({1-[2-(Benzofuran-3-yl)éthyl]-4-hydroxy-4-pipéridinyl}méthyl)-N-méthyl-2,1,3-benzothiadiazole-4-sulfonamide et son chlorhydrate

Le produit est obtenu selon le procédé de l'exemple 1 en utilisant comme substrat le composé de la préparation 16 au lieu de celui de la préparation 1, et comme réactif le chlorure de 2,1,3-benzothiadiazole-4-sulfonyle.
***Point de fusion (M.K.) : 217-220°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***53,10*** | ***5,41*** | ***10,64*** | ***6,80*** | ***12,37*** |
| ***% calculé*** | ***52*,*81*** | ***5*,*20*** | ***10*,*71*** | ***6*,*78*** | ***12,26*** |

### EXEMPLE 32 :

### N-({1-[2-(Benzofuran-3-yl)éthyl]-4-hydroxy-4-pipéridinyl}méthyl)-N-méthyl-2,1,3-benzoxadiazole-4-sulfonamide et son fumarate

Le produit est obtenu selon le procédé de l'exemple 1 en utilisant comme substrat le composé de la préparation 16 au lieu de celui de la préparation 1, et comme réactif le chlorure de 2,1,3-benzoxadiazole-4-sulfonyle.
***Point de fusion (M.K.) :* 216-223°C**

| ***Microanalyse élémentaire :*** | | | | |
|---|---|---|---|---|
| | ***C*** | ***H*** | ***N*** | ***S*** |
| ***% trouvé*** | ***55*,*38*** | ***5*,*31*** | ***9*,*60*** | ***5,30*** |
| ***% calculé*** | ***55*,*28*** | ***5*,*15*** | ***9*,*55*** | ***5,47*** |

### EXEMPLE 33 :

### N-({1-[2-(Benzofuran-3-yl)éthyl]-4-hydroxy-4-pipéridinyl}méthyl)-5-isoquinolinesulfonamide

Le produit est obtenu selon le procédé de l'exemple 1 en utilisant comme substrat le composé de la préparation 17 au lieu de celui de la préparation 1.
***Point de fusion (M.K.) : 120-130°C***

| ***Microanalyse élémentaire :*** | | | | |
|---|---|---|---|---|
| | ***C*** | ***H*** | ***N*** | ***S*** |
| ***% trouvé*** | ***64*,*68*** | ***5*,*82*** | ***9*,*00*** | ***6,47*** |
| ***% calculé*** | ***64*,*50*** | ***5*,*85*** | ***9*,*03*** | ***6,89*** |

### EXEMPLE 34 :

### N-({1-[2-(benzofuran-3-yl)éthyl]-4-hydroxy-4-pipéridinyl}méthyl)-2,1,3-benzoxadiazole-4-sulfonamide

Le produit est obtenu selon le procédé de l'exemple 1 en utilisant comme substrat le composé de la préparation 17 au lieu de celui de la préparation 1, et comme réactif le chlorure de 2,1,3-benzoxadiazole-4-sulfonyle.
***Point de fusion (M.K.) : 140-142°C***

| ***Microanalyse élémentaire :*** | | | | |
|---|---|---|---|---|
| | ***C*** | ***H*** | ***N*** | ***S*** |
| ***% trouvé*** | ***58*,*11*** | ***5*,*36*** | ***12*,*09*** | ***6,87*** |
| ***% calculé*** | ***57*,*88*** | ***5*,*30*** | ***12*,*27*** | ***7,02*** |

### EXEMPLE 35 :

### N-({1-[2-(5-Fluoro-benzofuran-3-yl)éthyl]-4-hydroxy-4-pipéridinyl}méthyl)-N-méthyl-5-isoquinolinesulfonamide et son dichlorhydrate

Le produit est obtenu selon le procédé de l'exemple 1 en utilisant comme substrat le composé de la préparation 18 au lieu de celui de la préparation 1.
***Point de fusion (M.K.) : 195-200°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***55,12*** | ***5,34*** | ***7,31*** | ***12,42*** | ***5,61*** |
| ***% calculé*** | ***54,74*** | ***5,30*** | ***7,37*** | ***12,43*** | ***5,62*** |

### EXEMPLE 36 :

### N-({1-[2-(5-Fluoro-benzofuran-3-yl)éthyl]-4-hydroxy-4-pipéridinyl}méthyl)-5-isoquinolinesulfonamide et son dichlorhydrate

Le produit est obtenu selon le procédé de l'exemple 1 en utilisant comme substrat le composé de la préparation 19 au lieu de celui de la préparation 1.
***Point de fusion (M.K.) : 220-230°C***

| ***Microanalyse élémentaire :*** | | | | | |
|---|---|---|---|---|---|
| | ***C*** | ***H*** | ***N*** | ***Cl*** | ***S*** |
| ***% trouvé*** | ***53,50*** | ***5,14*** | ***7,49*** | ***12,79*** | ***5,92*** |
| ***% calculé*** | ***53,96*** | ***5,07*** | ***7,55*** | ***12,74*** | ***5,76*** |

### EXEMPLE 37 :

### N-({1-[2-(7-Méthoxy-benzofuran-3-yl)éthyl]-4-hydroxy-4-pipéridinyl}méthyl)-N-méthyl-5-isoquinolinesulfonamide

Le produit est obtenu selon le procédé de l'exemple 1 en utilisant comme substrat le composé d la préparation 20 au lieu de celui de la préparation 1.
***Point de fusion (M.K.) : 125-135°C***

| ***Microanalyse élémentaire :*** | | | | |
|---|---|---|---|---|
| | ***C*** | ***H*** | ***N*** | ***S*** |
| ***% trouvé*** | ***63,63*** | ***6,36*** | ***8,00*** | ***6,69*** |
| ***% calculé*** | ***63,63*** | ***6,13*** | ***8,25*** | ***6,29*** |

### EXEMPLE 38 :

### N-({1-[2-(7-Méthoxy-benzofuran-3-yl)éthyl]-4-hydroxy-4-pipéridinyl}méthyl)-N-méthyl-2,1,3-benzoxadiazole-4-sulfonamide et son hémi-fumarate

Le produit est obtenu selon le procédé de l'exemple 1 en utilisant comme substrat le composé de la préparation 20 au lieu de celui de la préparation 1, et comme réactif le chlorure de 2,1,3-benzoxadiazole-4-sulfonyle.
***Point de fusion (M.K.) : 175-178°C***

| ***Microanalyse élémentaire :*** | | | | |
|---|---|---|---|---|
| | ***C*** | ***H*** | ***N*** | ***S*** |
| ***% trouvé*** | ***55,73*** | ***5,43*** | ***9,96*** | ***5,79*** |
| ***% calculé*** | ***55,90*** | ***5,41*** | ***10,03*** | ***5,74*** |

### EXEMPLE 39 :

### N-({1-[2-(5-Méthoxy-benzofuran-3-yl)éthyl]-4-hydroxy-4-pipéridinyl}méthyl)-5-isoquinolinesulfonamide

Le produit est obtenu selon le procédé de l'exemple 1 en utilisant comme substrat le composé de la préparation 21 au lieu de celui de la préparation 1.
***Point de fusion (M.K.) : 95-105°C***

### EXEMPLE 40 :

### N-({1-[2-(5-Méthoxy-benzofuran-3-yl)éthyl]-4-hydroxy-4-pipéridinyl}méthyl)-2,1,3-benzoxadiazole-4-sulfonamide et son fumarate

Le produit est obtenu selon le procédé de l'exemple 9 en utilisant comme substrat le composé de la préparation 21 au lieu de celui de la préparation 1
***Point de fusion (M.K.) : 204-208°C***

| ***Microanalyse élémentaire :*** | | | | |
|---|---|---|---|---|
| | ***C*** | ***H*** | ***N*** | ***S*** |
| ***% trouvé*** | ***54,04*** | ***5,15*** | ***9,02*** | ***5,14*** |
| ***% calculé*** | ***53,81*** | ***5,02*** | ***9,30*** | ***5,32*** |

### EXEMPLE 41 :

### N-({1-[2-(1H-indèn-3-yl)éthyl]-4-hydroxy-4-pipéridinyl}méthyl)-5-isoquinoline-sulfonamide et son sesquifumarate

Le produit est obtenu selon le procédé de l'exemple 1 en utilisant comme substrat le composé de la préparation 22 au lieu de celui de la préparation 1.
***Point de fusion (M.K.) : 200-210°C***

| ***Microanalyse élémentaire :*** | | | | |
|---|---|---|---|---|
| | ***C*** | ***H*** | ***N*** | ***S*** |
| ***% trouvé*** | ***59,92*** | ***5,74*** | ***6,53*** | ***4,81*** |
| ***% calculé*** | ***60,27*** | ***5,53*** | ***6,59*** | ***5,03*** |

### EXEMPLE 42 :

### N-({1-[2-(5-Méthoxy-benzofuran-3-yl)éthyl]-4-hydroxy-4-pipéridinyl}méthyl)-N-méthyl-5-isoquinolinesulfonamide et son fumarate

Le produit est obtenu selon le procédé de l'exemple 1 en utilisant comme substrat le composé de la préparation 23 au lieu de celui de la préparation 1.
***Point de fusion (M.K.) : 160-165°C***

| ***Microanalyse élémentaire :*** | | | | |
|---|---|---|---|---|
| | ***C*** | ***H*** | ***N*** | ***S*** |
| ***% trouvé*** | ***59*,*23*** | ***5*,*67*** | ***6*,*65*** | ***4,86*** |
| ***% calculé*** | ***59*,*51*** | ***5*,*64*** | ***6*,*72*** | ***5,12*** |

### EXEMPLE 43 :

### N-({1-[2-(7-Méthoxy-benzofuran-3-yl)éthyl]-4-hydroxy-4-pipéridinyl}méthyl)-5-isoquinolinesulfonamide et son hémi-fumarate

Le produit est obtenu selon le procédé de l'exemple 1 en utilisant comme substrat le composé de la préparation 24 au lieu de celui de la préparation 1.
***Point de fusion (M.K.) : 160-165°C***

| ***Microanalyse élémentaire :*** | | | | |
|---|---|---|---|---|
| | ***C*** | ***H*** | ***N*** | ***S*** |
| ***% trouvé*** | ***60*,*97*** | ***5*,*68*** | ***7*,*54*** | ***5,79*** |
| ***% calculé*** | ***60*,*75*** | ***5*,*64*** | ***7*,*59*** | ***5,79*** |

### EXEMPLE 44 :

### N-({1-[2-(1H-Indèn-3-yl)éthyl]-4-hydroxy-4-pipéridinyl}méthyl)-N-méthyl-5-isoquinolinesulfonamide

Le produit est obtenu selon le procédé de l'exemple 1 en utilisant comme substrat le composé de la préparation 25 au lieu de celui de la préparation 1.
***Point de fusion (M.K.) : 160-162°C***

| ***Microanalyse élémentaire :*** | | | | |
|---|---|---|---|---|
| | ***C*** | ***H*** | ***N*** | ***S*** |
| ***% trouvé*** | ***67,99*** | ***6,61*** | ***8,70*** | ***6,68*** |
| ***% calculé*** | ***67,90*** | ***6,54*** | ***8,80*** | ***6,71*** |

### EXEMPLE 45 :

### N-({2-[2-(Benzofuran-3-yl)éthyl]-4-hydroxy-4-pipéridinyl}éthyl)-5-isoquinolinesulfonamide

Le produit est obtenu selon le procédé de l'exemple 1 en utilisant comme substrat le composé de la préparation 26 au lieu de celui de la préparation 1.
***Point de fusion (M.K.) : 174-176°C***

| ***Microanalyse élémentaire:*** | | | | |
|---|---|---|---|---|
| | ***C*** | ***H*** | ***N*** | ***S*** |
| ***% trouvé*** | ***65,09*** | ***6,19*** | ***8,81*** | ***6,55*** |
| ***% calculé*** | ***65,11*** | ***6,10*** | ***8,76*** | ***6,49*** |

### EXEMPLE 46 :

### N-({1-[2-(5-Hydroxy-benzofuran-3-yl)éthyl]-4-hydroxy-4-pipéridinyl}méthyl)-N-méthyl-5-isoquinolinesulfonamide et son sel de potassium

A une solution de 1,65 g du produit de l'exemple 42 dans 14 ml de chloroforme sont additionnés, goutte à goutte à -15°C, 6,46 ml d'une solution molaire de tribromure de bore dans le dichlorométhane. Après 12 heures à température ambiante, le milieu réactionnel est hydrolysé, basifié par du bicarbonate de sodium. Le précipité formé est filtré puis chromatographié sur gel de silice (dichlorométhane/EtOH/NH₄OH : 90/10/1). Le produit attendu est ainsi isolé et transformé en son sel de potassium par de la potasse méthanolique.
***Point de fusion (M.K.) : 185-190°C***

| ***Microanalyse élémentaire :*** | | | | |
|---|---|---|---|---|
| | ***C*** | ***H*** | ***N*** | ***S*** |
| ***% trouvé*** | ***58,03*** | ***5,20*** | ***7,06*** | ***5,52*** |
| ***% calculé*** | ***58,51*** | ***5,29*** | ***7,87*** | ***6,01*** |

### EXEMPLE 47 :

### N-{1-[2-(Benzofuran-3-yl)-2-hydroxyéthyl]-4-pipéridinyl}-5-isoquinolinesulfonamide

Le produit est obtenu selon le procédé de l'exemple 1 en utilisant comme substrat le composé de la préparation 28 au lieu de celui de la préparation 1.
***Point de fusion (M.K.) : 160-165°C***

| ***Microanalyse élémentaire :*** | | | | |
|---|---|---|---|---|
| | ***C*** | ***H*** | ***N*** | ***S*** |
| ***% trouvé*** | ***63,46*** | ***5,65*** | ***9,20*** | ***7,21*** |
| ***% calculé*** | ***63,84*** | ***5,58*** | ***9,31*** | ***7,10*** |

### EXEMPLE 48 :

### N-{1-[3-(Benzofuran-3-yl)-2-hydroxypropyl]-4-pipéridinyl}-5-isoquinolinesulfonamide et son fumarate

Le produit est obtenu selon le procédé de l'exemple 1 en utilisant comme substrat le composé de la préparation 29 au lieu de celui de la préparation 1.
***Point de fusion (M.K.) : 102-109°C***

| ***Microanalyse élémentaire :*** | | | | |
|---|---|---|---|---|
| | ***C*** | ***H*** | ***N*** | ***S*** |
| ***% trouvé*** | ***58*,*94*** | ***5*,*42*** | ***6*,*62*** | ***5,06*** |
| ***% calculé*** | ***59*,*24*** | ***5*,*32*** | ***6*,*98*** | ***5,32*** |

### EXEMPLE 49 :

### N-{1-[3-(Benzofuran-3-yl)-2-hydroxypropyl]-4-pipéridinyl}-2-(3-pyridinyl) benzènesulfonamide et son fumarate

### Stade A : N-{1-[3-(Benzofuran-3-yl)-2-hydroxypropyl]-4-pipéridinyl}-2-bromobenzènesulfonamide

Le produit est obtenu selon le procédé de l'exemple 1 en utilisant comme substrat le composé de la préparation 29 au lieu de celui de la préparation 1, et comme réactif le chlorure de l'acide 2-bromobenzènesulfonique.
***Point de fusion (M.K.) : 108-110°C***

### Stade B : N-{1-[3-(Benzofuran-3-yl)-2-hydroxypropyl]-4-pipéridinyl}-2-(3-pyridinyl)benzènesulfonamide et son fumarate

A une solution de 3 g du produit obtenu au stade A, 280 mg de tétrakis (triphénylphosphine)palladium dans 50 ml de toluène sont ajoutés, à température ambiante pendant 2 heures, 20,3 ml d'une solution 2M de carbonate de sodium, puis sous argon, 1,3 g de diéthylboran-3-yl pyridine dans 25 ml d'éthanol. Le milieu réactionnel est alors porté à reflux pendant 2 jours, puis dilué à l'eau, extrait à l'acétate d'éthyle, séché sur MgSO₄ et concentré sous pression réduite. Une chromatographie sur gel de silice du résidu permet d'isoler le produit attendu.
***Point de fusion (M.K.) : 94-103°C***

| ***Microanalyse élémentaire :*** | | | | |
|---|---|---|---|---|
| | ***C*** | ***H*** | ***N*** | ***S*** |
| ***% trouvé*** | ***61,27*** | ***5,58*** | ***6,79*** | ***5,06*** |
| ***% calculé*** | ***61,27*** | ***5,47*** | ***6,91*** | ***5,28*** |

### EXEMPLE 50 :

### N-{1-[3-(Benzofuran-3-yl)-2-hydroxypropyl]-4-pipéridinyl}-2-[3-aminophényl] benzènesulfonamide

Le produit est obtenu selon le procédé de l'exemple 49 en utilisant comme réactif au stade B l'acide 3-aminophénylboronique.
***Point de fusion (M.K.) : 68-70°C***

| ***Microanalyse élémentaire :*** | | | | |
|---|---|---|---|---|
| | ***C*** | ***H*** | ***N*** | ***S*** |
| ***% trouvé*** | ***66,39*** | ***6,31*** | ***8,07*** | ***5,99*** |
| ***% calculé*** | ***66,51*** | ***6,18*** | ***8,11*** | ***6,34*** |

### EXEMPLE 51 :

### N-{1-[3-(Benzofuran-3-yl)-2-hydroxypropyl]-4-pipéridinyl}-3-[3-aminophényl] benzènesulfonamide

Le produit est obtenu selon le procédé de l'exemple 49 en utilisant comme réactifs au stade A l'acide 3-bromobenzènesulfonique et au stade B l'acide 3-aminophénylboronique.
***Point de fusion (M.K.) : 68-78°C***

| ***Microanalyse élémentaire :*** | | | | |
|---|---|---|---|---|
| | ***C*** | ***H*** | ***N*** | ***S*** |
| ***% trouvé*** | ***66,27*** | ***6,43*** | ***7,98*** | ***6,38*** |
| ***% calculé*** | ***66,51*** | ***6,18*** | ***8,31*** | ***6,34*** |

### EXEMPLE 52 :

### N-{1-[3-(Benzofuran-3-yl)-2-hydroxypropyl]-4-pipéridinyl}-3-(3-pyridinyl) benzènesulfonamide

Le produit est obtenu selon le procédé de l'exemple 49, des stades A à B, en utilisant comme réactif au stade A l'acide 3-bromobenzènesulfonique.
***Point de fusion (M.K.) : 55-63°C***

| ***Microanalyse élémentaire :*** | | | | |
|---|---|---|---|---|
| | ***C*** | ***H*** | ***N*** | ***S*** |
| ***% trouvé*** | ***66,02*** | ***6,03*** | ***8,41*** | ***6,19*** |
| ***% calculé*** | ***65,97*** | ***5,95*** | ***8,55*** | ***6,52*** |

### EXEMPLE 53 :

### N-{1-[3-(Benzofuran-3-yl)-2-fluoropropyl]-4-pipéridinyl}-5-isoquinolinesulfonamide et son difumarate

Le produit est obtenu selon le procédé de l'exemple 1 en utilisant comme substrat le produit de la préparation 30 au lieu de celui de la préparation 1.
***Point de fusion (M.K.) : 197-204°C***

| ***Microanalyse élémentaire :*** | | | | |
|---|---|---|---|---|
| | ***C*** | ***H*** | ***N*** | ***S*** |
| ***% trouvé*** | ***56,76*** | ***4,86*** | ***6,00*** | ***4,50*** |
| ***% calculé*** | ***56,15*** | ***4,86*** | ***5,81*** | ***4,43*** |

### EXEMPLE 54 :

### N-{1-[2-(Benzofuran-3-yl)-1-(fluorométhyl)éthyl]-4-pipéridinyl}-5-isoquinoline-sulfonamide et son fumarate

Le produit est obtenu selon le procédé de l'exemple 1 comme substrat le produit de la préparation 31 au lieu de celui de la préparation 1.
***Point de fusion (M.K.) : 143-153°C***

| ***Microanalyse élémentaire :*** | | | | |
|---|---|---|---|---|
| | ***C*** | ***H*** | ***N*** | ***S*** |
| ***% trouvé*** | ***59*,*72*** | ***5,48*** | ***6,82*** | ***5,00*** |
| ***% calculé*** | ***59*,*68*** | ***5*,*18*** | ***7*,*20*** | ***5,49*** |

### EXEMPLE 55 :

### N-{1-[2-(Benzofuran-3-yl)-éthyl]-4-pipéridinyl}-2-(3-pyridinyl)benzènesulfonamide et son fumarate

### Stade A : N-{1-[2-(Benzofuran-3-yl)éthyl]-4-pipéridinyl}-2-bromobenzène-sulfonamide

Le produit est obtenu selon le procédé du stade A de l'exemple 49 en utilisant comme substrat le produit de la préparation 7.
***Point de fusion (K) : 105-108°C***

### Stade B : N-{1-[2-(Benzofuran-3-yl)éthyl]-4-pipéridinyl}-2-bromo-N-[(2-méthoxyéthoxy)méthyl]benzènesulfonamide

A une solution de 15,8 g du produit obtenu au stade A dans 180 ml de tétrahydrofurane sont ajoutés, à température ambiante et sous argon, 1,9 g d'hydrure de sodium à 60 % dans l'huile. Après 30 minutes d'agitation, 5 ml de chlorure de 2-méthoxyéthoxyméthane sont ajoutés. Après 5 heures à température ambiante, le milieu réactionnel est concentré sous pression réduite, repris au dichlorométhane, lavé à l'eau, séché et concentré sous pression réduite permettant d'obtenir le produit attendu.

### Stade C : N-{1-[2-(Benzofuran-3-yl)éthyl]-4-pipéridinyl}-N-[(2-méthoxyéthoxy) méthyl]-2-(3-pyridinyl)benzènesulfonamide

Le produit est obtenu selon le procédé du stade B de l'exemple 49 en utilisant comme substrat le produit obtenu au stade B précédent.

### Stade D : N-{1-[2-(Benzofuran-3-yl)-éthyl]-4-pipéridinyl}-2-(3-pyridinyl)benzène-sulfonamide et son fumarate

Une solution de 1,7 g du produit obtenu au stade C dans 50 ml d'éthanol et 50 ml d'acide chlorhydrique 6N est mise à reflux 2 heures puis abandonnée 12 heures à température ambiante. Le milieu réactionnel est ensuite évaporé, dilué à l'eau, amené à pH = 7 par addition d'une solution aqueuse à 10 % de bicarbonate de sodium, extrait à l'acétate d'éthyle, séché, et évaporé pour conduire au produit attendu qui est transformé en son fumarate.
***Point de fusion (M.K.) : 189-191°C***

| ***Microanalyse élémentaire :*** | | | | |
|---|---|---|---|---|
| | ***C*** | ***H*** | ***N*** | ***S*** |
| ***% trouvé*** | ***62,34*** | ***5,52*** | ***7,26*** | ***5,46*** |
| ***% calculé*** | ***62,38*** | ***5,41*** | ***7,27*** | ***5,55*** |

### EXEMPLE 56 :

### N-{1-[2-(Benzofuran-3-yl)-éthyl]-4-pipéridinyl}-2-[3-aminophényl]benzène-sulfonamide et son fumarate

### Stade A : N-{1-[2-(Benzofuran-3-yl)éthyl]-4-pipéridinyl}-N-[(2-méthoxyéthoxy) méthyl]-2-(3-aminophényl)benzènesulfonamide

Le produit est obtenu selon le procédé de l'exemple 50 en utilisant comme substrat le produit obtenu au stade B de l'exemple 55.

### Stade B : N-{1-[2-(Benzofuran-3-yl)-éthyl]-4-pipéridinyl}-2-[3-amino phényl]benzènesulfonamide et son fumarate

Le produit est obtenu selon le procédé du stade D de l'exemple 55 en utilisant comme substrat le produit obtenu au stade A précédent.
***Point de fusion (M.K.) : 97-102°C***

| ***Microanalyse élémentaire :*** | | | | |
|---|---|---|---|---|
| | ***C*** | ***H*** | ***N*** | ***S*** |
| ***% trouvé*** | ***62,71*** | ***5,89*** | ***6,71*** | ***5,02*** |
| ***% calculé*** | ***62,93*** | ***5,62*** | ***7,10*** | ***5,42*** |

### EXEMPLE 57 :

### N-{1-[2-(Benzofuran-3-yl)-éthyl]-4-pipéridinyl}-3-(3-pyridinyl)benzènesulfonamide et son fumarate

Le produit est obtenu successivement selon le procédé du stade A de l'exemple 49 en utilisant comme substrat le produit de la préparation 7 et comme réactif le chlorure de 3-bromobenzènesulfonyle, puis selon le procédé du stade B de l'exemple 55, puis selon le procédé du stade B de l'exemple 49 et enfin selon le procédé du stade D de l'exemple 55.
***Point de fusion (M.K.) : 191-194°C***

| ***Microanalyse élémentaire :*** | | | | |
|---|---|---|---|---|
| | ***C*** | ***H*** | ***N*** | ***S*** |
| ***% trouvé*** | ***62*,*15*** | ***5*,*51*** | ***7*,*13*** | ***5*,*31*** |
| ***% calculé*** | ***62*,*38*** | ***5*,*41*** | ***7*,*27*** | ***5,55*** |

### EXEMPLE 58 :

### N-{1-[2-(benzofuran-3-yl)-éthyl]4-pipéridinyl}-3-[3-aminophényl] benzènesulfonamide et son dichlorhydrate

Le produit est obtenu successivement selon le procédé de l'exemple 50 en utilisant comme substrat le produit obtenu au second stade de synthèse de l'exemple 57, puis selon le procédé du stade D de l'exemple 55. Le produit obtenu est transformé en son dichlorhydrate par l'intermédiaire d'une solution 2,5 N d'éther chlorhydrique.
***Point de fusion (M.K.) : 155-167°C***

| ***Microanalyse élémentaire :*** | | | | | |
|---|---|---|---|---|---|
| | ***C*** | ***H*** | ***N*** | ***S*** | ***Cl*** |
| ***% trouvé*** | ***59,18*** | ***5,82*** | ***7,37*** | ***5,64*** | ***12,35*** |
| ***% calculé*** | ***59,12*** | ***5,70*** | ***7,66*** | ***5,85*** | ***12,93*** |

### EXEMPLE 59 :

### N-({1-[2-(Benzofuran-3-yl)éthyl]-4-pipéridinyl}méthyl)-N-méthyl-1,2,3,4-tétrahydro-5-isoquinolinesulfonamide et son dichlorhydrate

13,3 g du produit de l'exemple 1 et 1,3 g d'oxyde de platine dans 500 ml d'acide acétique sont hydrogénés sous 5 bars à température ambiante pendant 4 heures. Le milieu réactionnel est ensuite évaporé, repris à l'eau et à l'acétate d'éthyle, basifié à la soude 1N, décanté, lavé, séché et concentré sous pression réduite. Une chromatographie sur gel de silice permet d'isoler le produit attendu.
***Point de fusion (M.K.) : 162-174°C***

| ***Microanalyse élémentaire :*** | | | | | |
|---|---|---|---|---|---|
| | ***C*** | ***H*** | ***N*** | ***S*** | ***Cl*** |
| ***% trouvé*** | ***57,77*** | ***6,85*** | ***7,77*** | ***5,29*** | ***13,26*** |
| ***% calculé*** | ***57,77*** | ***6,53*** | ***7,77*** | ***5,93*** | ***13,12*** |

### EXEMPLE 60 :

### 2-Acétyl-N-({1-[2-(benzofuran-3-yl)éthyl]-4-pipéridinyl}méthyl)-N-méthyl-1,2,3,4-tétrahydro-5-isoquinolinesulfonamide et son chlorhydrate

2,2 g du produit obtenu à l'exemple 1 et 0,1 g d'oxyde de platine dans 120 ml d'acide acétique et 10 ml d'anhydride acétique sont hydrogénés sous 5 bars, à température ambiante, pendant 12 heures. Le produit attendu est isolé par chromatographie sur gel de silice (CH₂Cl₂/MeOH/NH₄OH : 98/2/0,2).
***Point de fusion (M.K.) : 108-110°C***

| ***Microanalyse élémentaire :*** | | | | | |
|---|---|---|---|---|---|
| | ***C*** | ***H*** | ***N*** | ***S*** | ***Cl*** |
| ***% trouvé*** | ***61,48*** | ***6,72*** | **7,71** | ***5,82*** | ***6,49*** |
| ***% calculé*** | ***61,58*** | ***6,64*** | ***7,69*** | ***5,87*** | ***6,49*** |

### EXEMPLE 61 :

### 5{[({1-[2-(Benzofuran-3-yl)éthyl]-4-pipéridinyl}méthyl)-(méthyl)amino]sulfonyl}-N-méthyl-3,4-dihydro-2(1H)-isoquinolinecarboxamide et son fumarate

2 g du produit de l'exemple 59 dans 4 ml de dichlorométhane sont additionnés à 10°C à une solution de 0,25 g d'isocyanate de méthyle dans 1 ml de dichlorométhane. Après 45 minutes, le milieu réactionnel est lavé, décanté, séché, et évaporé. Une chromatographie sur gel de silice (dichlorométhane/méthanol : 95/5) permet d'isoler le produit attendu.
***Point de fusion (M.K.) : 107-116°C***

| ***Microanalyse élémentaire :*** | | | | |
|---|---|---|---|---|
| | ***C*** | ***H*** | ***N*** | ***S*** |
| ***% trouvé*** | ***59,82*** | ***6,40*** | ***8,57*** | ***4,95*** |
| ***% calculé*** | ***59,98*** | ***6,29*** | ***8,74*** | ***5,00*** |

### EXEMPLE 62 :

### 5{[({1-[2-(benzofuran-3-yl)éthyl]-4-pipéridinyl}méthyl)-(méthyl)amino] sulfonyl}-3,4-dihydro-2(1H)-isoquinolinecarboxylate d'éthyle et son fumarate

A une solution, refroidie à 0°C, de 2 g du produit de l'exemple 59, 0,43 g de triéthylamine dans 7 ml de dichlorométhane est additionné 0,41 ml de chloroformiate d'éthyle. Après 2 heures à température ambiante, le milieu réactionnel est dilué à l'eau, décanté, séché et évaporé. Une chromatographie sur gel de silice (dichlorométhane/méthanol : 98/2) permet d'isoler le produit attendu.
***Point de fusion (M.K.) : 109-113°C***

| ***Microanalyse élémentaire :*** | | | | |
|---|---|---|---|---|
| | ***C*** | ***H*** | ***N*** | ***S*** |
| ***% trouvé*** | ***60,11*** | ***6,43*** | ***6,28*** | ***4,92*** |
| ***% calculé*** | ***60,44*** | ***6,30*** | ***6,41*** | ***4,89*** |

### EXEMPLE 63 :

### 5{[({1-[2-(Benzofuran-3-yl)éthyl]-4-pipéridinyl}méthyl)-(méthyl)amino]sulfonyl}-3,4-dihydro-2(1H)-isoquinolinecarboxamide et son fumarate

Une solution de 1 g du produit de l'exemple 59 dans 10 ml d'acide chlorhydrique 1N et 40 ml d'eau est amenée à pH = 3 par addition de soude à 20 %, puis une solution de 0,187 g d'isocyanate de potassium dans 0,5 ml d'eau est ajoutée à température ambiante.

Après 20 minutes, le pH du milieu s'est élevé jusqu'à pH = 6 et est ramené à pH = 3 par addition d'acide chlorhydrique 1N. Après 3 heures de réaction, l'opération précédente est répétée. Le milieu réactionnel est alors chauffé 3 heures à 50°C puis 0,187 g d'isocyanate de potassium dans 0,5 ml d'eau sont à nouveau ajoutés, et le pH du milieu est conservé à pH = 3. Après 2 heures, le milieu réactionnel est basifié à la soude, extrait au dichlorométhane. Après traitement usuel, le produit attendu est isolé.
***Point de fusion : 105-113°C***

| ***Microanalyse élémentaire :*** | | | | |
|---|---|---|---|---|
| | ***C*** | ***H*** | ***N*** | ***S*** |
| ***% trouvé*** | ***59*,*41*** | ***6*,*11*** | ***8*,*94*** | ***5,12*** |
| ***% calculé*** | ***59*,*16*** | ***5*,*95*** | ***8*,*36*** | ***4,91*** |

### EXEMPLE 64 :

### 5{[({1-[2-(Benzofuran-3-yl)éthyl]-4-pipéridinyl}méthyl)-(méthyl)amino]sulfonyl}-N,N-diméthyl-3,4-dihydro-2(1H)-isoquinolinecarboxamide et son fumarate

A une solution refroidie à 0°C de 1 g du produit de l'exemple 59, 0,24 g de triéthylmaine et 15 ml de dichlorométhane est additionné 0,22 ml de chlorure de N,N-diéthylcarbamoyle. Après 5 heures de réaction à température ambiante, puis 3 heures à reflux, 0,33 ml de triéthylamine et 0,22 ml de chlorure de N,N-diéthylcarbamoyle sont ajoutés et le reflux est maintenu 1 heure. Après traitement classique, le résidu d'évaporation est chromatographié sur gel de silice (dichlorométhane/méthanol) permettant d'isoler le produit attendu.
***Point de fusion (M.K.) : 115-120°C***

### EXEMPLE 65 :

### N-({1-[2-(Benzofuran-3-yl)éthyl]-4-pipéridinyl}méthyl)-N-méthyl-2-(méthylsulfonyl)-1,2,3,4-tétrahydro-5-isoquinolinesulfonamide et son chlorhydrate

A une solution, refroidie à 0°C, de 1,5 g du produit de l'exemple 59, 0,36 g de triéthylamine dans 15 ml de dichlorométhane est additionné 0,27 ml de chlorure d'acide méthanesulfonique. Après 10 minutes de réaction, le milieu réactionnel est traité de façon classique permettant d'obtenir le produit attendu qui est transformé en son chlorhydrate par l'intermédiaire d'une solution d'éther chlorhydrique.
***Point de fusion (M.K.) : 223-233°C***

### EXEMPLE 66 :

### N-({1-[2-(Benzofuran-3-yl)éthyl]-4-pipéridinyl}méthyl)-N-méthyl-2-[(trifluorométhyl) sulfonyl]-1,2,3,4-tétrahydro-5-isoquinolinesulfonamide et son fumarate

A une solution refroidie à -5°C de 1 g du produit de l'exemple 59 dans 20 ml de dichlorométhane est additionnée 0,36 ml d'anhydride d'acide trifluorométhanesulfonique, puis après 1 heure 0,36 g de triéthylamine et 0,36 ml d'anhydride d'acide trifluorométhanesulfonique. Après 12 heures de réaction à température ambiante et traitement usuel, une chromatographie sur gel de silice (dichlorométhane/méthanol : 98/2) permet d'isoler le produit attendu.
***Point de fusion (M.K.) : 198-200°C***

| ***Microanalyse élémentaire :*** | | | | |
|---|---|---|---|---|
| | ***C*** | ***H*** | ***N*** | ***S*** |
| ***% trouvé*** | ***52,77*** | ***5,16*** | ***5,82*** | ***8,99*** |
| ***% calculé*** | ***52,02*** | ***5,07*** | ***5,87*** | ***8,96*** |

### EXEMPLE 67 :

### Méthyl-5{[({1-[2-(benzofuran-3-yl)éthyl]-4-pipéridinyl}méthyl)-(méthyl)amino] sulfonyl}-3,4-dihydro-2(1H)-isoquinolinecarboxylate et son fumarate

Le produit est obtenu selon le procédé de l'exemple 62 en utilisant comme réactif le chloroformiate de méthyle.
***Point de fusion (M.K.) : 110-115°C***

| ***Microanalyse élémentaire :*** | | | | |
|---|---|---|---|---|
| | ***C*** | ***H*** | ***N*** | ***S*** |
| ***% trouvé*** | ***59*,*39*** | ***5*,*96*** | ***6*,*46*** | ***4*,*73*** |
| ***% calculé*** | ***59*,*89*** | ***6*,*13*** | ***6*,*55*** | ***5,00*** |

### EXEMPLE 68 :

### Isopropyl-5{[({1-[2-(benzofuran-3-yl)éthyl]-4-pipéridinyl}méthyl)-(méthyl)amino] sulfonyl}-3,4-dihydro-2(1H)-isoquinolinecarboxylate et son fumarate

Le produit est obtenu selon le procédé de l'exemple 62 en utilisant comme réactif le chloroformiate d'isopropyle.
***Point de fusion (M.K.) : 175-180°C***

| ***Microanalyse élémentaire :*** | | | | |
|---|---|---|---|---|
| | ***C*** | ***H*** | ***N*** | ***S*** |
| ***% trouvé*** | ***61,17*** | ***6,51*** | ***6,36*** | ***4,49*** |
| ***% calculé*** | ***60,97*** | ***6,47*** | ***6,27*** | ***4,79*** |

### EXEMPLE 69 :

### tert-Butyl-5{[({1-[2-(benzofuran-3-yl)éthyl]-4-pipéridinyl}méthyl)-(méthyl)amino] sulfonyl}-3,4-dihydro-2(1H)-isoquinolinecarboxylate et son fumarate

Le produit est obtenu selon le procédé de l'exemple 62 en utilisant comme réactif le chloroformiate de tertiobutyle.
***Point de fusion (M.K.) : 171-175°C***

| ***Microanalyse élémentaire :*** | | | | |
|---|---|---|---|---|
| | ***C*** | ***H*** | ***N*** | ***S*** |
| ***% trouvé*** | ***61*,*40*** | ***6*,*75*** | ***6*,*10*** | ***4,54*** |
| ***% calculé*** | ***61*,*48*** | ***6*,*63*** | ***6*,*14*** | ***4,69*** |

### EXEMPLE 70 :

### N-({1-[2-(Benzofuran-3-yl)éthyl]-4-pipéridinyl}méthyl)-1,2,3,4-tétrahydro-5-isoquinolinesulfonamide et son dichlorhydrate

Une solution de 6,6 g du composé de l'exemple 4, 150 ml de méthanol, 3 ml d'acide chlorhydrique concentré et 0,7 g de PtO₂ est hydrogéné sous 5 bars pendant 5 heures. Après traitement classique, le résidu obtenu est repris au méthanol et le dichlorhydrate est précipité par une solution de méthanol chlorhydrique.
***Point de fusion (M.K.) : 125-135°C***

| ***Microanalyse élémentaire :*** | | | | | |
|---|---|---|---|---|---|
| | ***C*** | ***H*** | ***N*** | ***S*** | ***Cl*** |
| ***% trouvé*** | ***57,01*** | ***6,39*** | ***7,78*** | ***5,78*** | ***13,39*** |
| ***% calculé*** | ***57,03*** | ***6,32*** | ***7,98*** | ***6,09*** | ***13,47*** |

### EXEMPLE 71 :

### 2-Acétyl-N-({1-[2-(benzofuran-3-yl)éthyl]-4-pipéridinyl}méthyl)-1,2,3,4-tétrahydro-5-isoquinolinesulfonamide et son fumarate

A une solution, refroidie à 0°C, de 2 g du produit de l'exemple 70 et 2,8 ml de diisopropyléthylamine est ajouté 0,32 ml de chlorure d'acide acétique. Après 10 minutes d'agitation à température ambiante, le milieu réactionnel est traité de façon classique. Une chromatographie sur gel de silice (dichlorométhane/méthanol : 95/5) permet d'isoler le produit attendu.
***Point de fusion (M.K.) : 136-140°C***

| ***Microanalyse élémentaire :*** | | | | |
|---|---|---|---|---|
| | ***C*** | ***H*** | ***N*** | ***S*** |
| ***% trouvé*** | ***60,96*** | ***6,15*** | ***6,86*** | ***5,02*** |
| ***% calculé*** | ***60,87*** | ***6,10*** | ***6,87*** | ***5,24*** |

### EXEMPLE 72 :

### N-{1-[2-(Benzofuran-3-yl)éthyl]-4-pipéridinyl}-N-méthyl-1,2,3,4-tétrahydro-5-isoquinolinesulfonamide

Le produit est obtenu selon le procédé de l'exemple 70 en utilisant comme substrat le produit de l'exemple 7.
***Point de fusion (M.K.) : 134-136°C***

| ***Microanalyse élémentaire :*** | | | | |
|---|---|---|---|---|
| | ***C*** | ***H*** | ***N*** | ***S*** |
| ***% trouvé*** | ***66,16*** | ***6,84*** | ***9,31*** | ***6,83*** |
| ***% calculé*** | ***66,20*** | ***6,89*** | ***9,26*** | ***7,07*** |

### EXEMPLE 73 :

### 2-Acétyl-N-{1-[2-(benzofuran-3-yl)éthyl]-4-pipéridinyl}-N-méthyl-1,2,3,4-tétrahydro-5-isoquinolinesulfonamide

Le produit est obtenu selon le procédé de l'exemple 71 en utilisant comme substrat le produit de l'exemple 72.
***Point de fusion (M.K.) : 118-120°C***

| ***Microanalyse élémentaire :*** | | | | |
|---|---|---|---|---|
| | ***C*** | ***H*** | ***N*** | ***S*** |
| ***% trouvé*** | ***65*,*31*** | ***6*,*70*** | ***8*,*31*** | ***6,14*** |
| ***% calculé*** | ***65*,*43*** | ***6*,*71*** | ***8*,*48*** | ***6,47*** |

### EXEMPLE 74 :

### 2-Trifluoroacétyl-N-({1-[2-(benzofuran-3-yl)éthyl]-4-pipéridinyl}méthyl)-N-méthyl-1,2,3,4-tétrahydro-5-isoquinolinesulfonamide et son fumarate

Le produit est obtenu selon le procédé de l'exemple 66 en utilisant comme substrat le produit de l'exemple 72, et comme réactif l'anhydride de l'acide trifluorométhylacétique.
***Point de fusion (M.K.) : 102-106°C***

| ***Microanalyse élémentaire :*** | | | | |
|---|---|---|---|---|
| | ***C*** | ***H*** | ***N*** | ***S*** |
| ***% trouvé*** | ***56,71*** | ***5,32*** | ***6,27*** | ***4,59*** |
| ***% calculé*** | ***56,55*** | ***5,34*** | ***6,18*** | ***4,72*** |

### EXEMPLE 75 :

### N-{1-[2-(Benzofuran-3-yl)éthyl]-4-pipéridinyl}-1,2,3,4-tétrahydro-5-isoquinolinesulfonamide et son hémi-fumarate

Le produit est obtenu selon le procédé de l'exemple 70 en utilisant comme substrat le produit de l'exemple 8.
***Point de fusion (M.K.) : 286-288°C***

| ***Microanalyse élémentaire :*** | | | | |
|---|---|---|---|---|
| | ***C*** | ***H*** | ***N*** | ***S*** |
| ***% trouvé*** | ***62,73*** | ***6,36*** | ***8,28*** | ***6,33*** |
| ***% calculé*** | ***62,76*** | ***6,28*** | ***8,44*** | ***6,44*** |

### EXEMPLE 76 :

### 2-Acétyl-N-{1-[2-(benzofuran-3-yl)éthyl]-4-pipéridinyl}-1,2,3,4-tétrahydro-5-isoquinolinesulfonamide

Le produit est obtenu selon le procédé de l'exemple 71 en utilisant comme substrat le produit de l'exemple 75.
***Point de fusion (M.K.) : 70-80°C***

| ***Microanalyse élémentaire :*** | | | | |
|---|---|---|---|---|
| | ***C*** | ***H*** | ***N*** | ***S*** |
| ***% trouvé*** | ***64,87*** | ***6,20*** | ***8,65*** | ***6,40*** |
| ***% calculé*** | ***64,84*** | ***6,49*** | ***8,72*** | ***6,66*** |

### EXEMPLE 77 :

### 2-Ethyl-N-({1-[2-(benzofuran-3-yl)éthyl]-4-pipéridinyl}méthyl)-N-méthyl-1,2,3,4-tétrahydro-5-isoquinolinesulfonamide et son camphosulfonate

Sur une suspension de 0,15 g d'hydrure de lithium et d'aluminium dans 25 ml de tétrahydrofurane à reflux est additionnée une solution d'1 g de produit de l'exemple 60 dans 60 ml de tétrahydrofurane. Après 1 heure à reflux puis refroidissement, le milieu réactionnel est hydrolysé par 0,1 ml d'eau, 0,08 ml de soude à 20 % et 0,37 ml d'eau. Après traitement classique, une chromatographie sur gel de silice (dichlorométhane/méthanol : 90/10) permet d'isoler le produit attendu.
***Point de fusion (M.K.) : 70-80°C***

### EXEMPLE 78 :

### 5{[({1-[2-(5-fluoro-benzofuran-3-yl)éthyl]-4-pipéridinyl}méthyl)-(méthyl)amino]sulfonyl}-3,4-dihydro-2(1H)-isoquinolinecarboxylate de méthyle et son fumarate

### Stade A : N-[(1-Benzyl-4-pipéridinyl)méthyl]-N-méthyl-5-isoquinolinesulfonamide

Le produit est obtenu selon le procédé de l'exemple 1 en utilisant le *N*-[(1-benzyl-4-pipéridinyl)méthyl]-*N*-méthylamine à la place du produit de la préparation 1.

### Stade B : N-[(1-Benzyl-4-pipéridinyl)méthyl]-N-méthyl-1,2,3,4-tétrahydro-5-isoquinolinesulfonamide

Le produit est obtenu selon le procédé de l'exemple 70 en utilisant le produit obtenu au stade A précédent.

### Stade C : 5-{[[(1-Benzyl-4-pipéridinyl)méthyl](méthyl)amino]sulfonyl}-3,4-dihydro-2(1H)-isoquinolinecarboxylate de méthyle

Le produit est obtenu selon le procédé de l'exemple 60 en utilisant le produit obtenu au stade B précédent, et comme réactif le chloroformiate de méthyle.

### Stade D : 5-{[[(4-Pipéridinyl)méthyl](méthyl)amino]sulfonyl}-3,4-dihydro-2(1H)-isoquinolinecarboxylate de méthyle

Un mélange composé de 5,6 g du produit obtenu au stade C, 3,7 g de formiate d'ammonium et 0,56 g de Pd/C à 10 % dans 120 ml de méthanol est porté 3 heures à reflux, puis filtré sur célite et évaporé à sec permettant d'isoler le produit attendu.

### Stade E : 5{[({1-[2-(5-fluoro-benzofuran-3-yl)éthyl]-4-pipéridinyl} méthyl)-(méthyl)amino]sulfonyl}-3,4-dihydro-2(1H)-isoquinolinecarboxylate de méthyle et son fumarate

Un mélange composé de 2 g du produit obtenu au stade D, 1,26 g de 2-(5-fluorobenzofuran-3-yl)-1-bromoéthane, 1,4 g de carbonate de potassium et 30 ml d'acétonitrile est porté à 50°C pendant 2 heures. Après évaporation à sec, le résidu est repris à l'eau et à l'acétate d'éthyle et subit un traitement usuel. Une chromatographie sur gel de silice (acétate d'éthyle) permet d'isoler le produit attendu qui est transformé en son fumarate.
***Point de fusion (M.K.) : 110-115°C***

| ***Microanalyse élémentaire :*** | | | | |
|---|---|---|---|---|
| | ***C*** | ***H*** | ***N*** | ***S*** |
| ***% trouvé*** | ***58,31*** | ***5,65*** | ***6,35*** | ***4,58*** |
| ***% calculé*** | ***58,26*** | ***5,81*** | ***6,37*** | ***4,86*** |

### EXEMPLE 79 :

### 5{[({1-[2-(5-fluoro-benzofuran-3-yl)éthyl]-4-pipéridinyl}méthyl)-(méthyl) amino]sulfonyl}-3,4-dihydro-2(1H)-isoquinolinecarboxylate d'éthyle et son fumarate

Le produit est obtenu selon le procédé de l'exemple 78 des stades A à E, en utilisant comme réactif au stade C, le chloroformiate d'éthyle.
***Point de fusion (M.K.) : 115-120°C***

| ***Microanalyse élémentaire :*** | | | | |
|---|---|---|---|---|
| | ***C*** | ***H*** | ***N*** | ***S*** |
| ***% trouvé*** | ***58,79*** | ***6,13*** | ***6,12*** | ***4,51*** |
| ***% calculé*** | ***58,82*** | ***6,00*** | ***6,24*** | ***4,76*** |

### EXEMPLE 80 :

### 5{[({1-[2-(5-fluoro-benzofuran-3-yl)éthyl]-4-pipéridinyl}méthyl)-(méthyl)amino]sulfonyl}-3,4-dihydro-2-(H)-isoquinolinecarboxylate d'isopropyle et son fumarate

Le produit est obtenu selon le procédé de l'exemple 78 des stades A à E, en utilisant comme réactif au stade C, le chloroformiate d'isopropyle.
***Point de fusion (M.K.) : 135-145°C***

| ***Microanalyse élémentaire :*** | | | | |
|---|---|---|---|---|
| | ***C*** | ***H*** | ***N*** | ***S*** |
| ***% trouvé*** | ***59,30*** | ***6,06*** | ***6,14*** | ***4,47*** |
| ***% calculé*** | ***59,38*** | ***6,15*** | ***6,11*** | ***4,66*** |

### EXEMPLE 81 :

### 5{[({1-[2-(benzofuran-3-yl)éthyl]4-hyroxy-4-pipéridinyl} éthyl)amino]sulfonyl}-3,4-dihydro-2(1H)-isoquinolinecarboxylate d'éthyle et son fumarate

Le produit est obtenu selon le procédé de l'exemple 78 des stades A à E, en utilisant comme substrat au stade A le 4-aminométhyl-1-benzylpipéridin-4-ol, au stade C le chloroformiate d'éthyle, et au stade E le mésylate du 2-(benzofuran-3-yl)éthanol.
***Point de fusion (M.K.) : 180-185°C***

| ***Microanalyse élémentaire :*** | | | | |
|---|---|---|---|---|
| | ***C*** | ***H*** | ***N*** | ***S*** |
| ***% trouvé*** | ***58,17*** | ***5,99*** | ***6,30*** | ***4,56*** |
| ***% calculé*** | ***58,44*** | ***5,98*** | ***6,39*** | ***4,87*** |

### EXEMPLE 82 :

### 5{[({1-[2-(5-fluoro-benzofuran-3-yl)éthyl]4-hydroxy-4-pipéridinyl} méthyl)amino]sulfonyl}-3,4-dihydro-2(1H)-isoquinolinecarboxylate d'éthyle et son fumarate

Le produit est obtenu selon le procédé de l'exemple 81, en utilisant comme réactif au stade E le mésylate du 2-(5-fluorobenzofuran-3-yl)éthanol.
***Point de fusion (M.K.) : 165-175°C***

| ***Microanalyse élémentaire :*** | | | | |
|---|---|---|---|---|
| | ***C*** | ***H*** | ***N*** | ***S*** |
| ***% trouvé*** | ***56,82*** | ***5,69*** | ***6,13*** | ***4,40*** |
| ***% calculé*** | ***56,88*** | ***5,67*** | ***6,22*** | ***4,75*** |

### EXEMPLE 83 :

### 2-Acétyl-N-{(1-[2-(benzofuran-3-yl)éthyl]-4-pipéridinyl-4-hydroxy)méthyl}-1,2,3,4-tétrahydro-5-isoquinolinesulfonamide et son mésylate

Le produit est obtenu selon le procédé de l'exemple 81, en utilisant comme réactif au stade C le chlorure d'acétyle à la place du chloroformiate d'éthyle.
***Point de fusion (M.K.) : 201-206°C***

| ***Microanalyse élémentaire :*** | | | | |
|---|---|---|---|---|
| | ***C*** | ***H*** | ***N*** | ***S*** |
| ***% trouvé*** | ***55,20*** | ***6,17*** | ***6,73*** | ***10,91*** |
| ***% calculé*** | ***55,34*** | ***6,14*** | ***6,91*** | ***10,55*** |

### EXEMPLE 84 :

### 5-{[({1-[2-(benzofuran-3-yl)éthyl]-4-hydroxy-4-pipéridinyl}méthyl)-(méthyl)amino]sulfonyl}-3,4-dihydro-2-(1H)-isoquinolinecarboxylate d'éthyle

Le produit est obtenu selon le procédé de l'exemple 83, en utilisant comme substrat au stade A le 1-benzyl-4-(méthylamino)méthyl-pipéridin-4-ol.
***Point de fusion (M.K.) : 125-130°C***

| ***Microanalyse élémentaire :*** | | | | |
|---|---|---|---|---|
| | ***C*** | ***H*** | ***N*** | ***S*** |
| ***% trouvé*** | ***62,76*** | ***6,76*** | ***7,56*** | ***5,90*** |
| ***% calculé*** | ***62,68*** | ***6,71*** | ***8,56*** | ***5,77*** |

### EXEMPLE 85 :

### 5-{[{1-[2-(Benzofuran-3-yl)éthyl]-4-pipéridinyl}amino]sulfonyl}-3,4-dihydro-2(1H)-isoquinolinecarboxamide

Le produit est obtenu selon le procédé de l'exemple 63 en utilisant comme substrat le composé de l'exemple 75.
***Point de fusion (M.K.) : 198-201°C***

### EXEMPLE 86 :

### N-{1-[2-(1H-Indèn-3-yl)éthyl]-4-pipéridinyl}-N-méthyl-1,2,3,4-térahydro-5-isoquinolinesulfonamide

Le produit est obtenu selon le procédé de l'exemple 59 en utilisant comme substrat le composé de l'exemple 5.
***Point de fusion (M.K.) : 210-214°C***

### EXEMPLE 87 :

### N-{1-[3-(Benzofuran-3-yl)-2-hydroxypropyl]4-pipéridinyl}3-[2-aminophényl] benzènesulfonamide et son dichlorhydrate

Le produit est obtenu selon le procédé de l'exemple 49 en utilisant au stade A l'acide 3-bromobenzènesulfonique et au stade B l'acide 2-aminophénylboronique. Le dichlorhydrate est obtenu par l'action d'une solution d'éther chlorhydrique.
***Point de fusion (M.K.) : 145-148°C***

### EXEMPLE 88 :

### N-{1-[2-(Benzofuran-3-yl)éthyl]4-pipéridinyl}4-bromo-5-isoquinolinesulfonamide et son fumarate

Le produit est obtenu selon le procédé de l'exemple 1 en utilisant comme substrat le composé de la préparation 7 au lieu de celui de la préparation 1 et le composé de la préparation 33 à la place du chlorhydrate de chlorure de 5-isoquinolinsulfonyle. Le produit est ensuite transformé en son fumarate.
***Point de fusion (M.K.) : 234-236°C***

### EXEMPLE 89 :

### N-{1-[2-(Benzofuran-3-yl)éthyl]4-pipéridinyl}4-fluoro-5-isoquinolinesulfonamide et son fumarate

Le produit est obtenu selon le procédé de l'exemple 1 en utilisant comme substrats le composé de la préparation 7 au lieu de celui de la préparation 1 et le composé de la préparation 34 à la place du chlorhydrate du chlorure de 5-isoquinolinesulfonyle. Le produit est ensuite transformé en son fumarate.
***Point de fusion (M.K.) : 219-221°C***

### ETUDES PHARMACOLOGIQUES DES COMPOSES DE L'INVENTION

Dans des conditions standard *in vitro,* la relaxation à l'acétylcholine (ACh) d'anneaux aortiques, totalement dépendante de la présence de l'endothélium, est le reflet de la production de NO (stimulée par l'ACh) qui en diffusant au niveau des cellules musculaires lisses entraîne la relaxation artérielle (*Nature,* **1980,** 288, 373).
Les composés de l'invention ont été testés sur deux modèles mettant en jeu deux mécanismes différents impliqués dans la dysfonction endothéliale observée en pathologie :
- le premier modèle consiste à induire une inhibition de la relaxation à l'ACh par blocage de l'activité enzymatique (NOS endothéliale) responsable de la production de NO.
- le second modèle consiste à induire un stress oxydant *in vitro* par un système enzymatique générateur d'O₂⁻ (xanthine oxydase -XO et hypoxanthine - Hypo).

### EXEMPLE 90 :

### Effets protecteurs vasculaires vis à vis d'une dysfonction endothéliale induite par un inhibiteur de NOS

L'aorte thoracique de rat Wistar (325-375 g), anesthésié par voie intrapérinétonéale au pentobarbital sodique (30 mg/kg), est prélevée et disséquée en anneaux de 3 mm de longueur. Chaque anneau est suspendu à un capteur de tension isométrique connecté à un système d'acquisition et la tension initiale appliquée est de 2,5 g. La solution physiologique utilisée, thermostatée à 37°C et oxygénée (95% O₂ + 5% CO₂) est composée de (en mM) : NaCl 112,0, KCl 5,0, CaCl₂ 2,5, KH₂PO₄ 1,0, MgSO₄ 1,2, NaHCO₃ 25,0, glucose 11,5, Ca-EDTA 0,016.
Après une période de stabilisation de 90 minutes les préparations sont contractées avec de la phényléphrine (PHE 10⁻⁶ M) et relaxées par addition de 10⁻⁵ M d'acétylcholine afin de vérifier l'intégrité de la couche endothéliale. Si celle-ci est confirmée, les préparations sont rincées et une concentration de produit à tester (ou son solvant) est additionnée au milieu suivie de 3.10⁻⁷ M de N^{G}-nitro-L-arginine (LNA). Les préparations sont à nouveau contractées avec de la phényléphrine et 30 minutes après les relaxations à l'acétylcholine (ACh - 10⁻⁸M à 10⁻⁵ M) sont évaluées en présence d'indométhacine (10⁻⁵M).
Les valeurs de relaxation sont exprimées en pourcentage par rapport à la contraction maximale à la PHE. Les effets protecteurs des composés vis à vis de la dysfonction endothéliale correspondent à la différence entre les pourcentages de relaxation maximale observée en présence ou en absence de produit.
A titre d'exemple, le composé de l'exemple 8 à 3x10⁻⁹ M inhibe de 23 % la dysfonction endothéliale induite par le LNA.

### EXEMPLE 91 :

### Effets protecteurs vasculaires vis à vis d'une dysfonction endothéliale induite par un système générateur de O₂⁻

Ce protocole, réalisé sur anneaux aortiques de lapins Néo-Zélandais (2,5-3 kg) est comparable au précédent hormis pour les points suivants : la tension initiale appliquée est de 5 g et l'association XO (3 mU/ml) - Hypo (10⁻⁴ M) est utilisée à la place du LNA.
A titre d'exemple, le composé de l'exemple 8 à 3x10⁻⁹ M inhibe de 28,3 % la dysfonction endothéliale induite par l'association XO-Hypo.

### EXEMPLE 92 :

### Implication de la voie du NO dans les effets protecteurs vasculaires détectés: évaluation de la production aortique de GMPc.

En diffusant au niveau des cellules musculaires lisses, le NO produit par les cellules endothéliales active la guanylate cyclase soluble ce qui entraîne une augmentation du GMP cyclique responsable de la relaxation.
Ce médiateur a donc été dosé sur les anneaux aortiques de rat afin de démontrer que les effets protecteurs des composés vis à vis de la dysfonction endothéliale sont médiés par une augmentation de la disponibilité en NO.
Les anneaux aortiques de rat sont préparés comme précédemment. Les effets d'une incubation de 30 minutes des composés de l'invention à différentes concentrations sont évalués sur la production de GMPc stimulée par l'ACh (10⁻⁵ M - 1 minute) en présence de LNA (3x10⁻⁶ M). Ces expériences sont réalisées en présence d'isobutyl méthyl xanthine (10⁻⁵ M) afin d'éviter la dégradation du GMPc par les phosphodiestérases. Les anneaux sont congelés dans de l'azote liquide et maintenus à -80°C jusqu'au dosage. Le contenu en GMPc est évalué par radio-immunodosage et exprimé par rapport à la quantité de protéines contenues dans le tissu (dosage par la méthode de Bradford).
A titre d'exemple, le composé de l'exemple 8 à 3x10⁻⁹ M augmente la production de GMPc stimulée par l'ACh en présence de LNA de 142,7 %

### EXEMPLE 93 :

### Composition pharmaceutique - Comprimé

| | |
|---|---|
| Composé de l'exemple 8 | 10 g |
| Hydroxypropylcellulose | 2 g |
| Polyvinylpyrrolidone | 2 g |
| Amidon de blé | 10 g |
| Lactose | 100 g |
| Stéarate de magnésium | 3 g |

## Revendications

1. Composés de formule (I) : 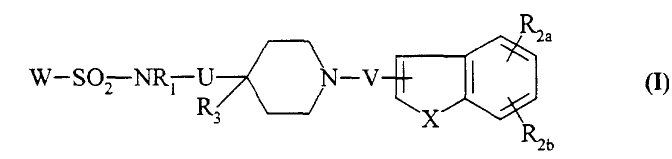 dans laquelle :
**R**_{**1**} représente un atome d'hydrogène ou un groupement alkyle (C₁-C₆) linéaire ou ramifié,
**R**_{**2a**}**, R**_{**2b**}**,** identiques ou différents, indépendamment l'un de l'autre, représentent chacun un groupement choisi parmi atome d'hydrogène, atome d'halogène, groupement alkyle (C₁-C₆) linéaire ou ramifié, hydroxy, alkoxy (C₁-C₆) linéaire ou ramifié, trihalogénoalkyle (C₁-C₆) linéaire ou ramifié, cyano, nitro, amino, alkylamino (C₁-C₆) linéaire ou ramifié, et dialkylamino (C₁-C₆) linéaire ou ramifié,
**R**_{**3**} représente un atome d'hydrogène ou un groupement hydroxy,
**X** représente un atome d'oxygène ou un groupement méthylène,
**V** représente une chaîne alkylène (C₁-C₆) linéaire ou ramifiée comportant éventuellement une ou plusieurs insaturations et étant éventuellement substituée par un ou plusieurs groupements, identiques ou différents, choisis parmi atomes d'halogène, groupements hydroxy et alkoxy (C₁-C₆) linéaire ou ramifié,
**U** représente une liaison ou une chaîne alkylène (C₁-C₆) linéaire ou ramifiée,
**W** représente un groupement choisi parmi aryle et hétéroaryle, chacun de ces groupements étant éventuellement substitué par un ou plusieurs groupements, identiques ou différents, choisis parmi atomes d'halogène, groupements alkyle (C₁-C₆) linéaire ou ramifié, hydroxy, alkoxy (C₁-C₆) linéaire ou ramifié, trihalogénoalkyle (C₁-C₆) linéaire ou ramifié, oxo, cyano, nitro, amino, alkylamino (C₁-C₆) linéaire ou ramifié, dialkylamino (C₁-C₆) linéaire ou ramifié, pyridinyle, alkylcarbonyle (C₁-C₆) linéaire ou ramifié, aminocarbonyle (la partie amino étant éventuellement substituée par un ou deux groupements, identiques ou différents, alkyle (C₁-C₆) linéaire ou ramifié), alkoxycarbonyle (C₁-C₆) linéaire ou ramifié, trihalogénoalkylcarbonyle (C₁-C₆) linéaire ou ramifié, alkylsulfonyle (C₁-C₆) linéaire ou ramifié, et trihalogénoalkylsulfonyle (C₁-C₆) linéaire ou ramifié,
leurs isomères, leurs hydrates, leurs solvates ainsi que leurs sels d'addition à un acide ou à une base pharmaceutiquement acceptable,
étant entendu que :
- par groupement aryle, on comprend un groupement choisi parmi phényle, biphényle, naphtyle, dihydronaphtyle, tétrahydronaphtyle, indanyle et indényle,
- par groupement hétéroaryle, on comprend un système monocyclique aromatique ou bicyclique, de 5 à 12 chaînons, comportant de 1 à 3 hétéroatomes, identiques ou différents, choisis parmi oxygène, azote et soufre, et dont l'un des cycles, dans le cas d'un système bicyclique possède un caractère aromatique, l'autre cycle pouvant être aromatique ou partiellement hydrogéné.

2. Composés de formule (I) selon la revendication 1 **caractérisés en ce que** X représente un atome d'oxygène, leurs isomères, leurs hydrates, leurs solvates ainsi que leurs sels d'addition à un acide pharmaceutiquement acceptable.

3. Composés de formule (I) selon la revendication 1 **caractérisés en ce que** R₁ représente l'atome d'hydrogène ou le groupement méthyle, leurs isomères, leurs hydrates, leurs solvates ainsi que leurs sels d'addition à un acide pharmaceutiquement acceptable.

4. Composés de formule (I) selon l'une quelconques des revendications 1 à 3 **caractérisés en ce qu'**ils représentent les composés de formule **(I/A)** : 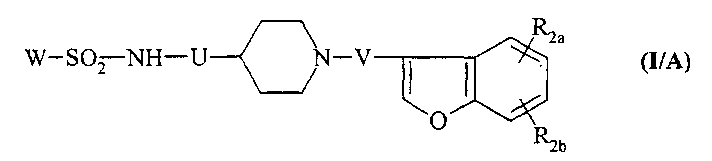 dans laquelle W, U, V, R₂ₐ et R_{2b} sont tels que définis dans la formule (I), leurs isomères, leurs hydrates, leurs solvates ainsi que leurs sels d'addition à un acide pharmaceutiquement acceptable.

5. Composés de formule (I) selon la revendication 1 **caractérisés en ce que** W représente un groupement isoquinolin-5-yl, leurs isomères, leurs hydrates, leurs solvates ainsi que leurs sels d'addition à un acide pharmaceutiquement acceptable.

6. Composés de formule (I) selon la revendication 1 **caractérisés en ce que** W représente un groupement 1,2,3,4-tétrahydro-isoquinolin-5-yl éventuellement substitué en position 2 par un groupement de formule -C(O)-A dans laquelle A représente un groupement choisi parmi alkyle (C₁-C₆) linéaire ou ramifié, amino (lui-même éventuellement substitué par un ou deux groupements, identiques ou différents, alkyle (C₁-C₆) linéaire ou ramifié), alkoxy (C₁-C₆) linéaire ou ramifié, trifluorométhyle, alkylsulfonyle (C₁-C₆) linéaire ou ramifié, et trifluorométhylsulfonyle, leurs isomères, leurs hydrates, leurs solvates ainsi que leurs sels d'addition à un acide pharmaceutiquement acceptable.

7. Composés de formule (I) selon la revendication 4 **caractérisés en ce qu'**ils représentent des composés de formule (I/A) telle que définie précédemment dans laquelle U, V, R₂ₐ et R_{2b} sont tels que définis dans la formule (I) et W représente un groupement isoquinolin-5-yl, leurs isomères, leurs hydrates, leurs solvates ainsi que leurs sels d'addition à un acide pharmaceutiquement acceptable.

8. Composés de formule (I) selon la revendication 4 **caractérisés en ce qu'**ils représentent des composés de formule (I/A) telle que définie précédemment dans laquelle U, V, R₂ₐ et R_{2b} sont tels que définis dans la formule (I) et W représente un groupement 1,2,3,4-tétrahydroisoquinolin-5-yl éventuellement substitué en position 2 par un groupement de formule -C(O)A dans laquelle A est tel que défini précédemment, leurs isomères, leurs hydrates, leurs solvates ainsi que leurs sels d'addition à un acide pharmaceutiquement acceptable.

9. Composés de formule (I) selon la revendication 1 qui sont :
- le *N*-{1-[2-(benzofuran-3-yl)éthyl]-4-pipéridinyl}(isoquinolin-5-yl)sulfonamide,
- le 5-{[({(1-[2-(benzofuran-3-yl)éthyl]-4-pipéridinyl}méthyl)-(méthyl)amino]sulfonyl}-3,4-dihydro-2(1*H*)-isoquinolinecarboxylate d'éthyle,
- le *N*-({2-[2-benzofuran-3-yl)éthyl]-4-hydroxy-4-pipéridinyl}éthyl)-5-isoquinoline-sulfonamide,
- le *N*-({1-[2-(benzofuran-3-yl)éthyl]-4-hydroxy-4-pipéridinyl}méthyl)-*N*-méthyl-2,1,3-benzoxadiazole-4-sulfonamide,
- et le 5-{[({1-[2-(benzofuran-3-yl)éthyl]-4-pipéridinyl}méthyl)-(méthyl)amino]sulfonyl}-3,4-dihydro-2(1*H*)-isoquinolinecarboxylate d'isopropyle.

10. Procédé de préparation des composés de formule (I) selon la revendication 1 **caractérisé en ce qu'**on utilise comme produit de départ un composé de formule **(II)** : 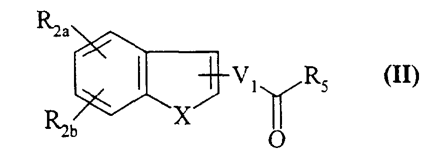 dans laquelle X, R₂ₐ et R_{2b} sont tels que définis dans la formule (I), V₁ représente une liaison ou une chaîne alkylène (C₁-C₅) linéaire ou ramifié comportant éventuellement une ou plusieurs insaturations, R₅ représente un atome d'hydrogène, de chlore, un groupement hydroxy ou alkoxy (C₁-C₆) linéaire ou ramifié,
composés de formule (II) que l'on fait réagir :
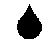 ***soit*** avec un composé de formule **(III/A)** : 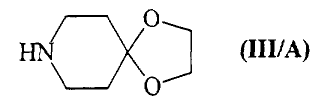 pour conduire aux composés de formule **(IV)** : 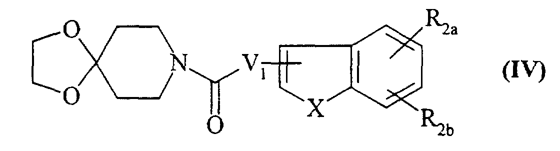 dans laquelle R₂ₐ, R_{2b}, X et V₁ sont tels que définis précédemment,
composés de formule (IV) dont on déprotège la fonction cétal, selon des techniques classiques de la synthèse organique, puis que l'on fait réagir dans des conditions d'amination réductrice avec un composé de formule **(V)** :
R'₁ - NH₂ **(V)**
dans laquelle R'₁ représente un groupement alkyle (C₁-C₆) linéaire ou ramifié, pour conduire aux composés de formule **(VI/A)** : 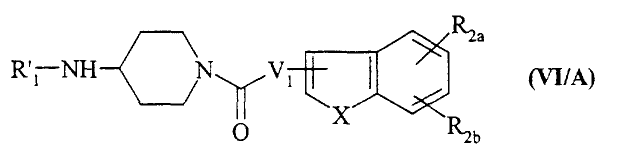 dans laquelle R'₁, R₂ₐ, R_{2b}, X et V₁ ont les mêmes significations que précédemment,
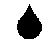 ***soit*** avec un composé de formule **(III/B)** : 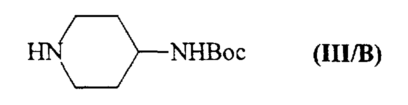 dans laquelle Boc représente un groupement *t*-butyloxycarbonyle,
pour conduire aux composés de formule **(VII)** : 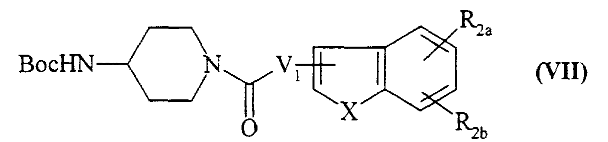 dans laquelle Boc, R₂ₐ, R_{2b}, X et V₁ sont tels que définis précédemment, composés de formule (VII) dont on déprotège la fonction amine terminale pour conduire aux composés de formule **(VI/B)** : 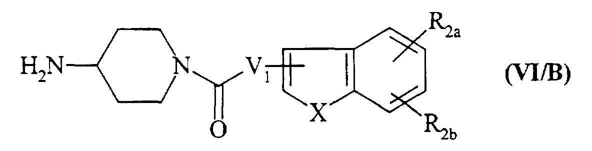 dans laquelle R₂ₐ, R_{2b}, V₁ et X sont tels que définis précédemment,
l'ensemble des composés de formule (VI/A) et (VI/B) forment les composés de formule **(VI)** : 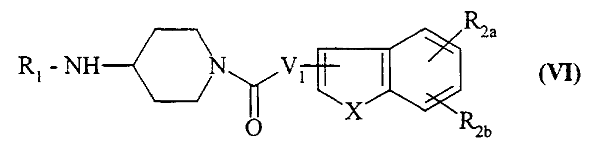 dans laquelle R₁ est tel que défini dans la formule (I) et R₂ₐ, R_{2b}, X et V₁ sont tels que définis précédemment,
composés de formule (VI) que l'on traite par l'un des agents réducteurs utilisés couramment en synthèse organique pour réduire les fonctions amides, pour conduire aux composés de formule **(VIII)** : 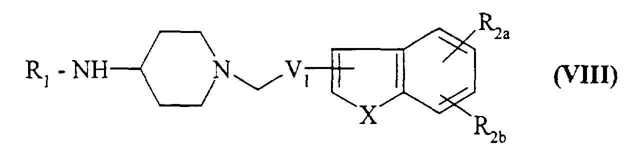 dans laquelle R₁, V₁, X, R₂ₐ et R_{2b} sont tels que définis précédemment,
composés de formule (VIII) que l'on place en présence d'un composé de formule **(IX)** :
W - SO₂Cl **(IX)**
dans laquelle W a la même signification que dans la formule (I), pour conduire aux composés de formule **(I/a)**, cas particulier des composés de formule (I) : 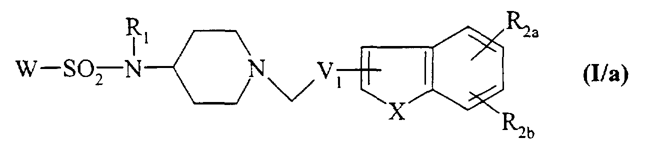 dans laquelle W, R₁, V₁, X, R₂ₐ et R_{2b} sont tels que définis précédemment,
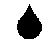 ***soit*** avec un composé de formule **(III/C)** : 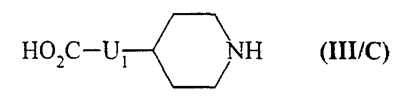 dans laquelle U₁ représente une liaison ou une chaîne alkylène (C₁-C₅) linéaire ou ramifiée, pour conduire aux composés de formule **(X)** : 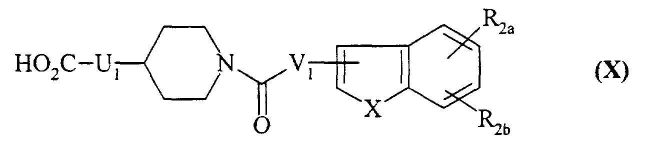 dans laquelle U₁, V₁, X, R₂ₐ et R_{2b} sont tels que définis précédemment,
composés de formule (X) que l'on traite avec de l'ammoniaque ou avec un dérivé de formule (V) telle que définie précédemment en présence d'un agent de couplage tel que du dicyclohexylcarbodiimide, du carbonyldiimidazole ou du 1,1,1,3,3,3-hexaméthyl-disylazane, pour conduire aux composés de formule **(XI)** : 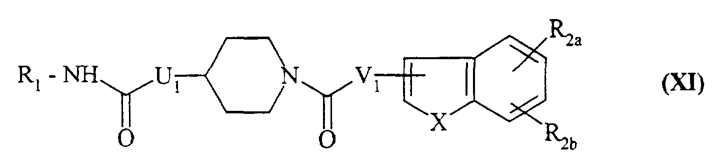 dans laquelle R₁ est tel que défini dans la formule (I), U₁, V₁, X, R₂ₐ et R_{2b} sont tels que définis précédemment,
composés de formule (XI) que l'on réduit selon des conditions classiques de la synthèse organique, pour conduire aux composés de formule **(XII)** : 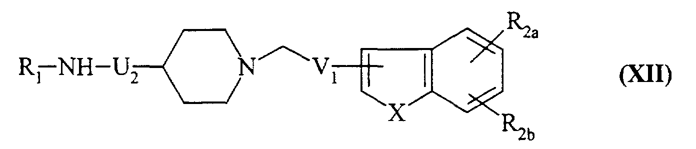 dans laquelle U₂ représente une chaîne alkylène (C₁-C₆) linéaire ou ramifié, R₁, V₁, X, R₂ₐ et R_{2b} sont tels que définis précédemment,
composés de formule (XII) qui est mis à réagir avec un composé de formule (IX), tel que décrit précédemment, pour conduire aux composés de formule **(I/b)**, cas particulier des composés de formule (I) : 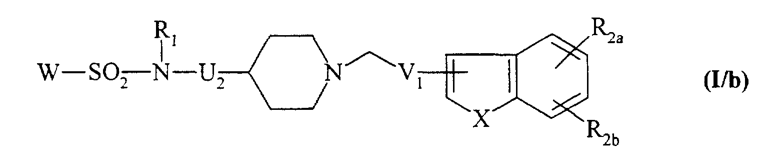 dans laquelle W, R₁, U₂, V₁, X, R₂ₐ et R_{2b} sont tels que définis précédemment,
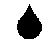 ***ou*** composés de formule (II) dont on réduit le groupement carbonyle terminal substitué par un groupement R'₅ prenant les définitions de R₅ tel que défini précédemment, exceptées la valeur atome de chlore, puis dont on substitue le groupement hydroxy obtenu, par un atome d'halogène tel que Cl, Br, ou I, en utilisant les techniques usuelles de la chimie organique, pour conduire aux composés de formule **(II/B)** : 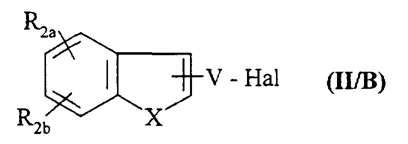 dans laquelle X et V sont tels que définis dans la formule (I) et Hal représente un atome d'halogène,
composé de formule (II/B) que l'on additionne sur un composé de formule **(III/D)** : 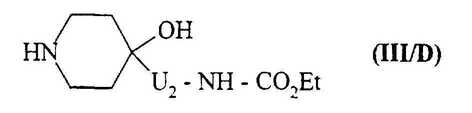 dans laquelle U₂ est tel que défini précédemment,
pour conduire aux composés de formule **(XIII)** : 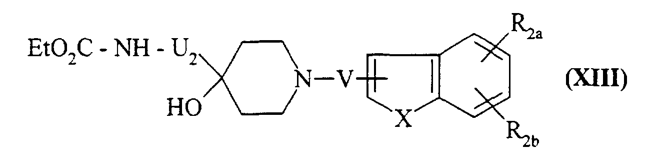 dans laquelle U₂, V, X, R₂ₐ et R_{2b} ont les mêmes significations que précédemment,
composés de formule (XIII) que l'on traite soit par un agent réducteur, soit par une base forte alcaline, pour conduire aux composés de formule **(XIV)** : 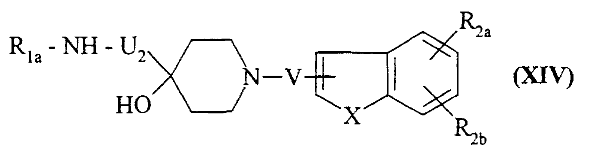 dans laquelle R₁ₐ représente un atome d'hydrogène ou un groupement méthyle, U₂, V, X, R₂ₐ et R_{2b} sont tels que définis précédemment,
composés de formule (XIV) que l'on met en présence d'un composé de formule (IX), pour conduire aux composés de formule **(I/c),** cas particulier des composés de formule (I) : 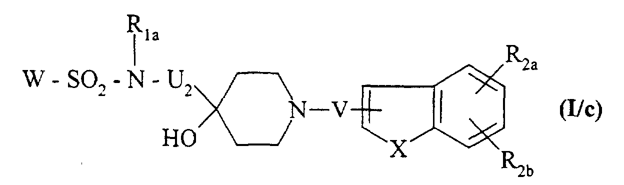 dans laquelle W, R₁ₐ, U₂, V, X, R₂ₐ et R_{2b} ont les mêmes significations que précédemment,
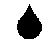 ***ou*** composé de formule (II), dans le cas particulier où R₅ représente un atome d'hydrogène et V₁ prend la valeur V'₁ représentant une chaîne alkylène (C₁-C₄) linéaire ou ramifiée, que l'on traite par de l'iodure de triméthylsulfoxonium en présence d'hydrure de sodium dans le diméthylsulfoxyde, pour conduire aux composés de formule **(II/C)** : 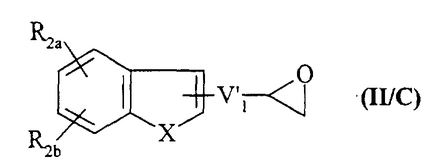 dans laquelle R₂ₐ, R_{2b}, X et V'₁ sont tels que définis précédemment,
composés de formule (II/C) que l'on fait réagir avec l'un quelconque des composés de formule (III/A), (III/B) ou (III/C), les intermédiaires obtenus étant alors déprotégés et fonctionnalisés selon les méthodes respectives décrites précédemment, pour conduire aux composés de formule **(XV)** : 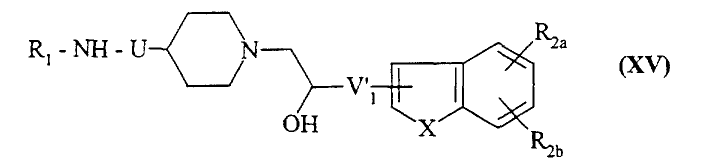 dans laquelle R₁, U, X, R₂ₐ et R_{2b} sont tels que définis précédemment et V'₁ prend la signification décrite précédemment,
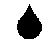 ***ou*** composé de formule (II), dans le cas particulier où il représente un composé de formule **(II/D)** : 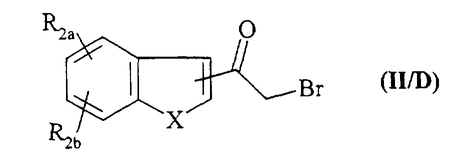 dans laquelle R₂ₐ, R_{2b} et X sont tels que définis dans la formule (I),
que l'on fait réagir avec l'un quelconque des composés de formule (III/A), (III/B) ou (III/C), les intermédiaires obtenus étant alors déprotégés et fonctionnalisés selon les méthodes respectives décrites précédemment,
pour conduire aux composés de formule **(XVI)** : 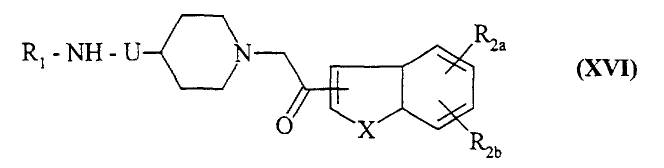 dans laquelle R₁, U, X, R₂ₐ et R_{2b} sont tels que définis précédemment,
composés de formule (XVI) que l'on réduit de façon classique pour conduire aux composés de formule **(XVII)** : 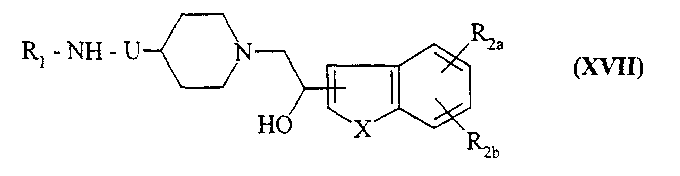 l'ensemble des composés de formule (XV) et (XVII) formant les composés de formule **(XVIII)** : 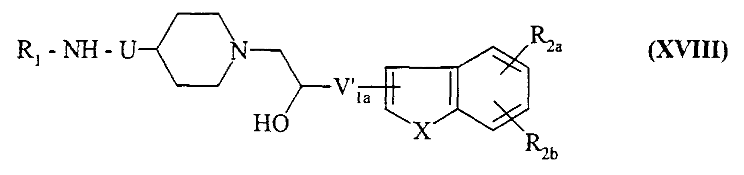 dans laquelle R₁, U, X, R₂ₐ et R_{2b} sont tels que définis précédemment, et V'₁ₐ représente une liaison ou une chaîne alkylène (C₁-C₄) linéaire ou ramifiée,
composés de formule (XVIII) dont on substitue, si on le souhaite la fonction hydroxy par un atome d'halogène, selon des conditions classiques de la synthèse organique, pour conduire aux composés de formule (XIX) : 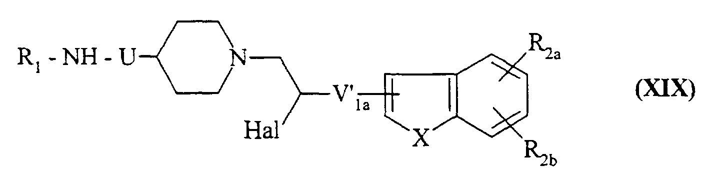 dans laquelle Hal représente un atome d'halogène, et R₁, U, V'₁ₐ, X, R₂ₐ et R_{2b} sont tels que définis précédemment,
l'ensemble des composés de formule (XVIII) et (XIX) étant mis à réagir avec un composé de formule (IX) tel que décrit précédemment, pour conduire aux composés de formule **(I/d),** cas particulier des composés de formule (I) : 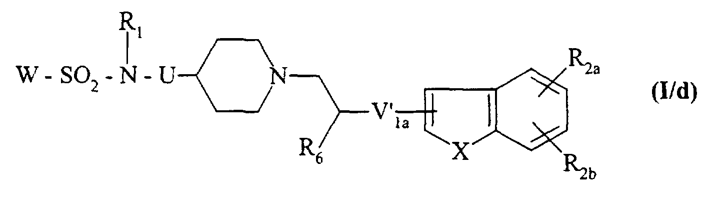 dans laquelle W, R₁, U, X, R₂ₐ et R_{2b} sont tels que définis dans la formule (I), V'₁ₐ est tel que défini précédemment et R₆ représente un groupement hydroxy ou un atome d'halogène,
composés de formule (I/d), dans le cas particulier où R₆ représente un groupement hydroxy, que l'on soumet à l'action d'un agent alkylant selon des conditions classiques de la chimie organique, pour conduire aux composés de formule (I/e), cas particulier des composés de formule (I): 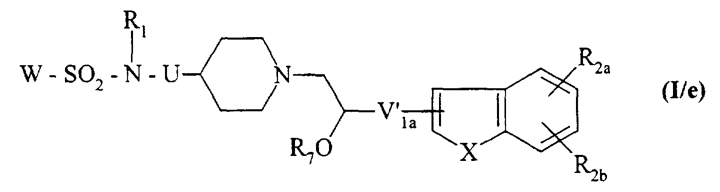 dans laquelle W, R₁, U, V'₁ₐ, X, R₂ₐ et R_{2b} sont tels que définis précédemment et R₇ représente un groupement alkyle (C₁-C₆) linéaire ou ramifié,
les composés de formule (I/a) à (I/e) formant l'ensemble des composés de l'invention, que l'on purifie, le cas échéant, selon des techniques classiques de purification, qui peuvent, si on le désire, être séparés en leurs différents isomères, selon une technique classique de séparation, dont on module les substituants R₂ₐ, R_{2b} et ceux du groupement W selon des méthodes classiques de la synthèse organique utilisées dans le domaine de la chimie aromatique dont on réduit, si on le souhaite, le groupement W dans le cas où celui-ci est un système bicyclique, pour conduire à des groupements W bicycliques dont l'un des cycles est partiellement hydrogéné, celui-ci pouvant ensuite être substitué selon des conditions classiques de la chimie organique, et que l'on transforme, si on le souhaite, en leurs sels d'addition à un acide ou à une base pharmaceutiquement acceptable, et qui peuvent exister éventuellement sous formes d'hydrates ou de solvates.

11. Compositions pharmaceutiques contenant comme principe actif au moins un composé de formule (I) selon l'une quelconque des revendications 1 à 9, seul ou en combinaison avec un ou plusieurs excipients ou véhicules inertes, non toxiques, pharmaceutiquement acceptables.

12. Compositions pharmaceutiques selon la revendication 11 contenant au moins un principe actif selon l'une quelconque des revendications 1 à 9, utiles en tant que médicament pour le traitement des maladies ou des conditions pathologiques dans lesquelles une dysfonction endothéliale est connue.

13. Compositions pharmaceutiques selon la revendication 11 contenant au moins un principe actif selon l'une quelconque des revendications 1 à 9, utiles en tant que médicament pour prévenir le développement, l'extension et les complications des lésions athéroscléreuses, pour prévenir les complications vasculaires après pontage vasculaire, dilatation vasculaire, reperméabilisation vasculaire et transplantation cardiaque, ou pour traiter l'ischémie myocardique ou périphérique, l'insuffisance cardiaque et l'hypertension artérielle pulmonaire.

## Patentansprüche

1. Verbindungen der Formel (I): 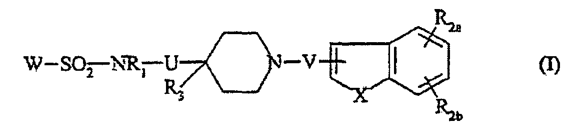 in der:
**R**_{**1**} ein Wasserstoffatom oder eine geradkettige oder verzweigte (C₁-C₆)-Alkylgruppe darstellt,
**R**_{**2a**} und **R**_{**2b**}**,** die gleichartig oder verschieden sind, unabhängig voneinander jeweils eine Gruppe bedeuten ausgewählt aus Wasserstoff- oder Halogenatomen, geradkettigen oder verzweigten (C₁-C₆)-Alkyl-, Hydroxy-, geradkettigen oder verzweigten (C₁-C₆)-Alkoxy-, geradkettigen oder verzweigten (C₁-C₆)-Tri-halogenalkyl-, Cyano-, Nitro-, Amino-, geradkettigen oder verzweigten (C₁-C₆)-Alkylamino- und geradkettigen oder verzweigten Di-(C₁-C₆)-alkylaminogruppen,
**R**_{**3**} ein Wasserstoffatom oder eine Hydroxygruppe bedeutet,
**X** ein Sauerstoffatom oder eine Methylengruppe bedeutet,
**V** eine geradkettige oder verzweigte (C₁-C₆)-Alkylenkette bedeutet, die gegebenenfalls eine oder mehrere Unsättigungen aufweist und gegebenenfalls durch eine oder mehrere gleichartige oder verschiedenartige Gruppen ausgewählt aus Halogenatomen, Hydroxy- und geradkettigen oder verzweigten (C₁-C₆)-Alkoxygruppen substituiert ist,
**U** eine Bindung oder eine geradkettige oder verzweigte (C₁-C₆)-Alkylenkette bedeutet,
**W** eine Gruppe bedeutet ausgewählt aus Aryl und Heteroaryl, wobei jede dieser Gruppen gegebenenfalls substituiert ist durch eine oder mehrere gleichartige oder verschiedenartige Gruppen ausgewählt aus Halogenatomen, geradkettigen oder verzweigten (C₁-C₆)-Alkyl-, Hydroxy-, geradkettigen oder verzweigten (C₁-C₆)-Alkoxy-, geradkettigen oder verzweigten (C₁-C₆)-Trihalogenalkyl-, Oxo-, Cyano-, Nitro-, Amino-, geradkettigen oder verzweigten (C₁-C₆)-Alkyl-amino-, geradkettigen oder verzweigten Di-(C₁-C₆)-alkylamino-, Pyridinyl-, geradkettigen oder verzweigten (C₁-C₆)-Alkylcarbonyl-, Aminocarbonyl- (wobei der Aminorest gegebenenfalls durch eine oder zwei gleichartige oder verschiedenartige, geradkettige oder verzweigte (C₁-C₆)-Alkylgruppen substituiert ist), geradkettigen oder verzweigten (C₁-C₆)-Alkoxycarbonyl-, geradkettigen oder verzweigten (C₁-C₆)-Trihalogenalkylcarbonyl-, geradkettigen oder verzweigten (C₁-C₆)-Alkylsulfonyl- und geradkettigen oder verzweigten (C₁-C₆)-Trihalogenalkylsulfonylgruppen,
deren Isomere, deren Hydrate, deren Solvate sowie deren Additionssalze mit einer pharmazeutisch annehmbaren Säure oder Base, mit der Maßgabe, daß man:
- unter einer Arylgruppe eine Gruppe versteht ausgewählt aus Phenyl, Biphenyl, Naphthyl, Dihydronaphthyl, Tetrahydronaphthyl, Indanyl und Indenyl,
- unter einer Heteroarylgruppe ein monocyclisches aromatisches oder bicyclisches System mit 5 bis 12 Kettengliedern, welches 1 bis 3 gleichartige oder verschiedenartige Heteroatome ausgewählt aus Sauerstoff, Stickstoff und Schwefel versteht, wobei einer der Ringe im Fall eines bicyclischen Systems einen aromatischen Charakter aufweist, während der andere aromatisch oder teilweise hydriert sein kann.

2. Verbindungen der Formel (I) nach Anspruch 1, **dadurch gekennzeichnet, daß** X ein Sauerstoffatom bedeutet, deren Isomere, deren Hydrate, deren Solvate sowie deren Additionssalze mit einer pharmazeutisch annehmbaren Säure.

3. Verbindungen der Formel (I) nach Anspruch 1, **dadurch gekennzeichnet, daß** R₁ ein Wasserstoffatom oder die Methylgruppe bedeutet, deren Isomere, deren Hydrate, deren Solvate sowie deren Additionssalze mit einer pharmazeutisch annehmbaren Säure.

4. Verbindungen der Formel (I) nach einem der Ansprüche 1 bis 3, **dadurch gekennzeichnet, daß** sie die Verbindungen der Formel **(I/A)** sind: 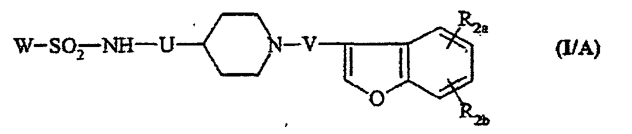 in der W, U, V, R₂ₐ und R_{2b} die bezüglich der Formel (I) angegebenen Bedeutungen besitzen, deren Isomere, deren Hydrate, deren Solvate sowie deren Additionssalze mit einer pharmazeutisch annehmbaren Säure.

5. Verbindungen der Formel (I) nach Anspruch 1, **dadurch gekennzeichnet, daß** W eine Isochinolin-5-yl-gruppe bedeutet, deren Isomere, deren Hydrate, deren Solvate sowie deren Additionssalze mit einer pharmazeutisch annehmbaren Säure.

6. Verbindungen der Formel (I) nach Anspruch 1, **dadurch gekennzeichnet, daß** W eine 1,2,3,4-Tetrahydro-isochinolin-5-yl-gruppe bedeutet, die gegebenenfalls in der 2-Stellung durch eine Gruppe der Formel -C(O)-A substituiert ist, in der A eine Gruppe bedeutet ausgewählt aus geradkettigem oder verzweigten (C₁-C₆)-Alkyl, Amino (welches seinerseits gegebenenfalls durch eine oder zwei gleichartige oder verschiedenartige, geradkettige oder verzweigte (C₁-C₆)-Alkylgruppen substituiert ist), geradkettigem oder verzweigtem (C₁-C₆)-Alkoxy, Trifluormethyl, geradkettigem oder verzweigtem (C₁-C₆)-Alkylsulfonyl und Trifluormethylsulfonyl, deren Isomere, deren Hydrate, deren Solvate sowie deren Additionssalze mit einer pharmazeutisch annehmbaren Säure.

7. Verbindungen der Formel (I) nach Anspruch 4, **dadurch gekennzeichnet, daß** sie die Verbindungen der Formel (I/A) sind, wie sie oben definiert worden ist, in der U, V, R₂ₐ und R_{2b} die bezüglich der Formel (I) angegebenen Bedeutungen besitzt und W eine Isochinolin-5-yl-gruppe bedeutet, deren Isomere, deren Hydrate, deren Solvate sowie deren Additionssalze mit einer pharmazeutisch annehmbaren Säure.

8. Verbindungen der Formel (I) nach Anspruch 4, **dadurch gekennzeichnet, daß** sie die Verbindungen der Formel (I/A) sind, wie sie oben definiert worden ist, in der U, V, R₂ₐ und R_{2b} die bezüglich der Formel (I) angegebenen Bedeutungen besitzen und W eine 1,2,3,4-Tetrahydroisochinolin-5-yl-gruppe bedeutet, die gegebenenfalls in der 2-Stellung durch eine Gruppe der Formel -C(O)A substituiert ist, in der A die oben angegebenen Bedeutungen besitzt, deren Isomere, deren Hydrate, deren Solvate sowie deren Additionssalze mit einer pharmazeutisch annehmbaren Säure.

9. Verbindungen der Formel (I) nach Anspruch 1, nämlich:
- *N*-{1-[2-(Benzofuran-3-yl)-ethyl]-4-piperidinyl}(isochinolin-5-yl)-sulfonamid,
- 5-{[({(1-[2-(Benzofuran-3-yl)-ethyl]-4-piperidinyl}-methyl)-(methyl)-amino]-sulfonyl}-3,4-dihydro-2-(1*H*)-isochinolincarbonsäureethylester,
- N-({2-[2-Benzofuran-3-yl)-ethyl]-4-hydroxy-4-piperidinyl}-ethyl)-5-isochinolinsulfonamid,
- *N*-({1-[2-(Benzofuran-3-yl)-ethyl]-4-hydroxy-4-piperidinyl}-methyl)-*N*-methyl-2,1,3-benzoxadiazol-4-sulfonamid und
- 5-{[({1-[2-(Benzofuran-3-yl)-ethyl]-4-piperidinyl}-methyl)-(methyl)-amino]-sulfonyl}-3,4-dihydro-2(1*H*)-isochinolincarbonsäure-isopropylester.

10. Verfahren zur Herstellung der Verbindungen der Formel (I) nach Anspruch 1, **dadurch gekennzeichnet, daß** man als Ausgangsprodukt eine Verbindung der Formel **(II)** verwendet: 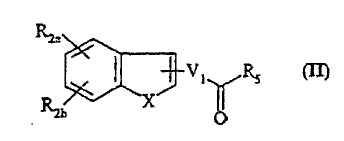 in der X, R₂ₐ und R_{2b} die bezüglich der Formel (I) angegebenen Bedeutungen besitzen, V₁ eine Bindung oder eine geradkettige oder verzweigte (C₁-C₅)-Alkylenkette, die gegebenenfalls eine oder mehrere Unsättigungen aufweist, und R₅ ein Wasserstoffatom, ein Chloratom, eine Hydroxygruppe oder eine geradkettige oder verzweigte (C₁-C₆)-Alkoxygruppe bedeuten,
welche Verbindungen der Formel (II) man:
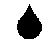 ***entweder*** mit einer Verbindung der Formel **(III/A)** umsetzt: 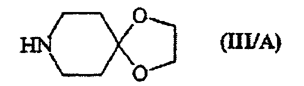 zur Bildung der Verbindungen der Formel **(IV)**: 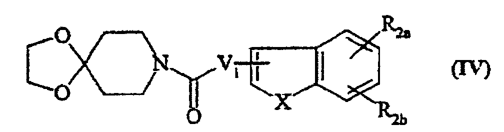 in der R₂ₐ, R_{2b}, X und V₁ die oben angegebenen Bedeutungen besitzen,
von welchen Verbindungen der Formel (IV) man die Ketalschutzgruppe unter Anwendung klassischer Methoden der organischen Synthese abspaltet und sie unter den Bedingungen der reduzierenden Aminierung mit einer Verbindung der Formel **(V)** umsetzt:
R'₁ - NH₂ **(V)**
in der R'₁ eine geradkettige oder verzweigte (C₁-C₆)-Alkylgruppe darstellt,
zur Bildung der Verbindungen der Formel **(VI/A)**: 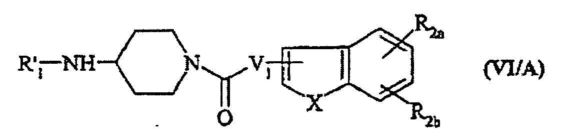 in der R'₁, R₂ₐ, R_{2b}, X und V₁ die oben angegebenen Bedeutungen besitzen,
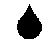 ***oder*** mit einer Verbindung der Formel **(III/B)** umsetzt: 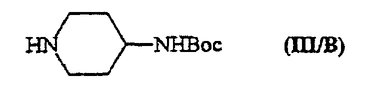 in der Boc eine *t*.-Butyloxycarbonylgruppe darstellt,
zur Bildung der Verbindungen der Formel **(VII):** 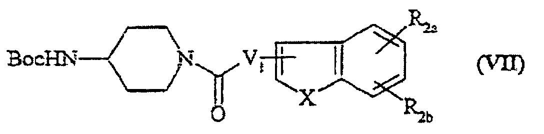 in der Boc, R₂ₐ, R_{2b}, X und V₁ die oben angegebenen Bedeutungen besitzen, von welchen Verbindungen der Formel (VII) man die Schutzgruppe der endständigen Aminfunktion abspaltet zur Bildung der Verbindungen der Formel **(VI/B)**: 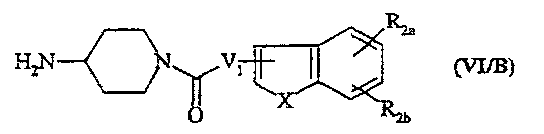 in der R₂ₐ, R_{2b}, V₁ und X die oben angegebenen Bedeutungen besitzen,
wobei die Gesamtheit der Verbindungen der Formel (VI/A) und (VI/B) die Verbindungen der Formel **(VI)** bildet: 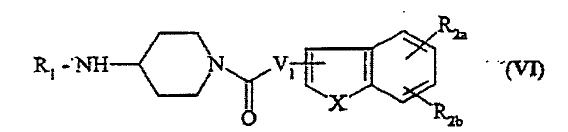 in der R₁ die bezüglich der Formel (I) angegebenen Bedeutungen besitzt und R₂ₐ, R_{2b}, X und V₁ die oben angegebenen Bedeutungen besitzen,
welche Verbindungen der Formel (VI) man mit einem der in dieser Weise in der organischen Synthese für die Reduktion von Amidfunktionen verwendeten Reduktionsmittel behandelt zur Bildung der Verbindungen der Formel **(VIII)**: 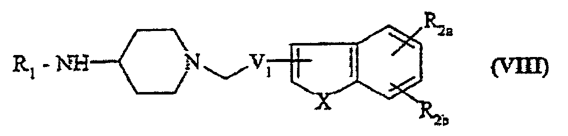 in der R₁, V₁, X, R₂ₐ und R_{2b} die oben angegebenen Bedeutungen besitzen,
welche Verbindungen der Formel (VIII) man mit einer Verbindung der Formel **(IX)** in Kontakt bringt:
W - SO₂Cl **(IX)**
in der W die bezüglich der Formel (I) angegebenen Bedeutungen besitzt, zur Bildung der Verbindungen der Formel **(I/a)**, einem Sonderfall der Verbindungen der Formel (I): 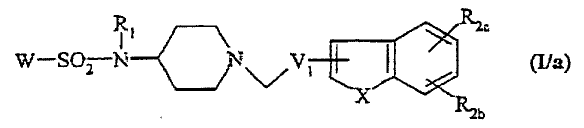 in der W, R₁, V₁, X, R₂ₐ und R_{2b} die oben angegebenen Bedeutungen besitzen,
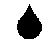 ***oder*** mit einer Verbindung der Formel **(III/C)** umsetzt: 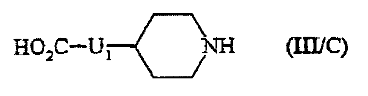 in der U₁ eine Bindung oder eine geradkettige oder verzweigte (C₁-C₅)-Alkylenkette bedeutet, zur Bildung der Verbindungen der Formel **(X)**: 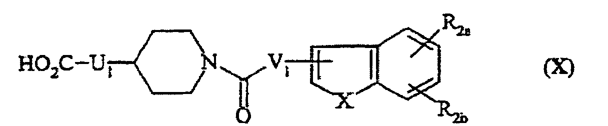 in der U₁, V₁, X, R₂ₐ und R_{2b} die oben angegebenen Bedeutungen besitzen,
welche Verbindungen der Formel (X) man mit Ammoniak oder einem Derivat der Formel (V), wie sie oben definiert worden ist, in Gegenwart eines Kupplungsmittels, wie Dicyclohexylcarbodiimid, Carbonyldiimidazol oder 1,1,1,3,3,3-Hexamethyldisilazan, behandelt zur Bildung der Verbindungen der Formel **(XI)**: 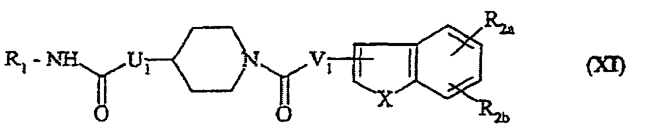 in der R₁ die bezüglich der Formel (I) angegebenen Bedeutungen besitzt und U₁, V₁, X, R₂ₐ und R_{2b} die oben angegebenen Bedeutungen besitzen,
welche Verbindungen der Formel (XI) man unter klassischen Bedingungen der organischen Synthese reduziert zur Bildung der Verbindungen der Formel **(XII)**: 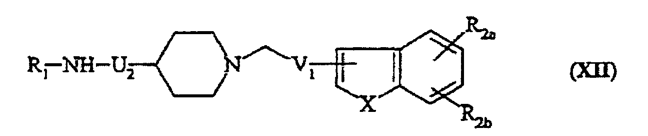 in der U₂ eine geradkettige oder verzweigte (C₁-C₆)-Alkylenkette bedeutet und R₁, V₁, X, R₂ₐ und R_{2b} die oben angegebenen Bedeutungen besitzen,
welche Verbindungen der Formel (XII) mit einer Verbindung der Formel (IX), wie sie oben beschrieben worden ist, umsetzt zur Bildung der Verbindungen der Formel **(I/b)**, einem Sonderfall der Verbindungen der Formel (I): 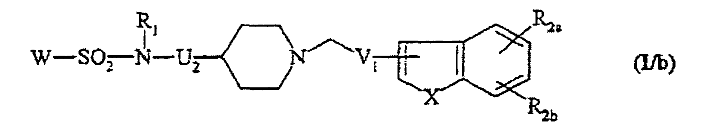 in der W, R₁, U₂, V₁, X, R₂ₐ und R_{2b} die oben angegebenen Bedeutungen besitzen,
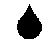 ***oder*** man die endständige Carbonylgruppe der Verbindungen der Formel (II), die durch eine Gruppe R'₅ substituiert ist, welche die Bedeutungen von R₅ mit Ausnahme des Chloratoms besitzt, reduziert und dann die erhaltene Hydroxygruppe mit Hilfe üblicher Methoden der organischen Synthese mit einem Halogenatom, wie Cl, Br oder I, substituiert, zur Bildung der Verbindungen der Formel **(II/B)**: 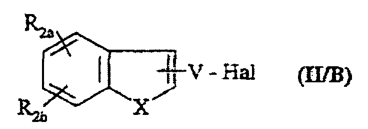 in der X und V die bezüglich der Formel (I) angegebenen Bedeutungen besitzen und Hal ein Halogenatom darstellt,
welche Verbindung der Formel (II/B) man zu einer Verbindung der Formel **(III/D)** gibt: 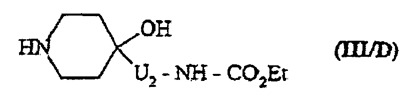 in der U₂ die oben angegebenen Bedeutungen besitzt,
zur Bildung der Verbindungen der Formel **(XIII)**: 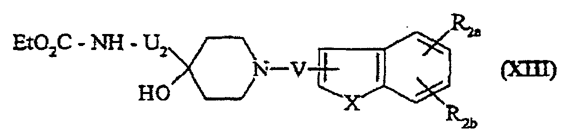 in der U₂, V, X, R₂ₐ und R_{2b} die oben angegebenen Bedeutungen besitzen,
welche Verbindungen der Formel (XIII) man entweder mit einem Reduktionsmittel oder mit einer stark alkalischen Base behandelt, zur Bildung der Verbindungen der Formel **(XIV)**: 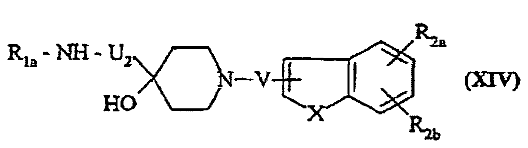 in der R₁ₐ ein Wasserstoffatom oder eine Methylgruppe bedeutet und U₂, V, X, R₂ₐ und R_{2b} die oben angegebenen Bedeutungen besitzen,
welche Verbindungen der Formel (XIV) man mit einer Verbindung der Formel (IX) in Kontakt bringt zur Bildung der Verbindungen der Formel **(I/c)**, einem Sonderfall der Verbindungen der Formel (I): 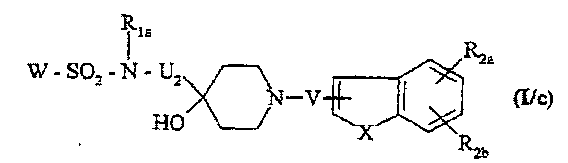 in der W, R₁ₐ, U₂, V, X, R₂ₐ und R_{2b} die oben angegebenen Bedeutungen besitzen,
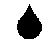 ***oder*** man die Verbindung der Formel (II) in dem Sonderfall, da R₅ ein Wasserstoffatom bedeutet und V₁ die Bedeutung V'₁, welche eine geradkettige oder verzweigte (C₁-C₄)-Alkylenkette darstellt, aufweist, mit Trimethylsulfoxoniumiodid in Gegenwart von Natriumhydrid in Dimethylsulfoxid behandelt zur Bildung der Verbindungen der Formel **(II/C)**: 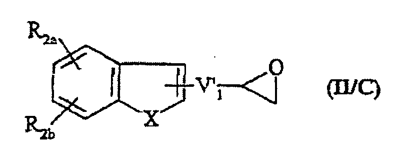 in der R₂ₐ, R_{2b}, X und V'₁ die oben angegebenen Bedeutungen besitzen,
welche Verbindungen der Formel (II/C) man mit irgendeiner der Verbindungen der Formeln (III/A), (III/B) oder (III/C) umsetzt, wobei die erhaltenen Zwischenprodukte mit Hilfe der oben beschriebenen Verfahren von ihren Schutzgruppen befreit befreit und funktionalisiert werden zur Bildung der Verbindungen der Formel **(XV)**: 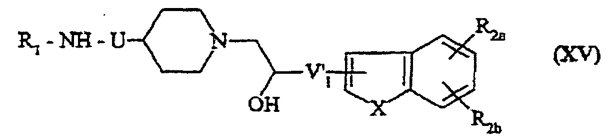 in der R₁, U, X, R₂ₐ und R_{2b} die oben angegebenen Bedeutungen besitzen und V'₁ die oben beschriebene Bedeutung aufweist,
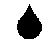 ***oder*** man die Verbindung der Formel (II) in dem Sonderfall, da sie eine Verbindung der Formel **(II/D)** darstellt: 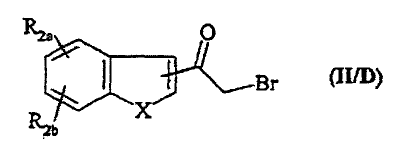 in der R₂ₐ, R_{2b} und X die bezüglich der Formel (I) angegebenen Bedeutungen besitzen,
mit irgendeiner der Verbindungen der Formeln (III/A), (III/B) oder (III/C) umsetzt, wobei die erhaltenen Zwischenprodukte mit Hilfe der oben beschriebenen Verfahren von ihren Schutzgruppen befreit und funktionalisiert werden,
zur Bildung der Verbindungen der Formel **(XVI)**: 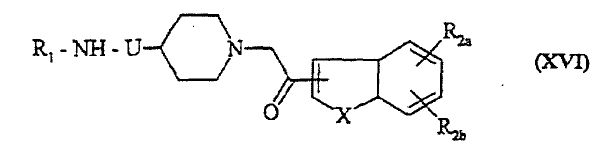 in der R₁, U, X, R₂ₐ und R_{2b} die oben angegebenen Bedeutungen besitzen,
welche Verbindungen der Formel (XVI) man in klassischer Weise reduziert zur Bildung der Verbindungen der Formel **(XVII)**: 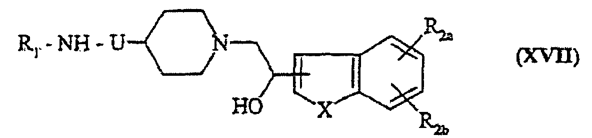 wobei die Gesamtheit der Verbindungen der Formeln (XV) und (XVII) die Verbindungen der Formel **(XVIII)** bildet: 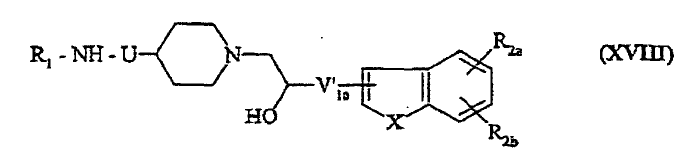 in der R₁, U, X, R₂ₐ und R_{2b} die oben angegebenen Bedeutungen besitzen und V'₁ₐ eine Bindung oder eine geradkettige oder verzweigt (C₁-C₄)-Alkylenkette darstellt, bei welchen Verbindungen der Formel (XVIII) man gewünschtenfalls die Hydroxyfunktion unter Anwendung klassischer Bedingungen der organischen Synthese durch ein Halogenatom substituiert, zur Bildung der Verbindungen der Formel **(XIX)**: 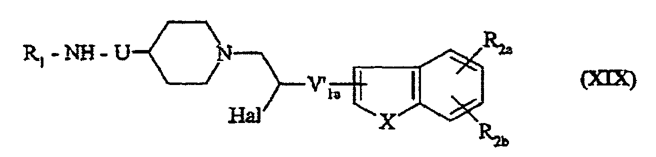 in der Hal ein Halogenatom darstellt und R₁, U, V'₁ₐ, X, R₂ₐ und R_{2b} die oben angegebenen Bedeutungen besitzen,
welche Gesamtheit der Verbindungen der Formeln (XVIII) und (XIX) man mit einer Verbindung der oben beschriebenen Formel (IX) umsetzt, zur Bildung der Verbindungen der Formel **(I/d),** einem Sonderfall der Verbindungen der Formel (I): 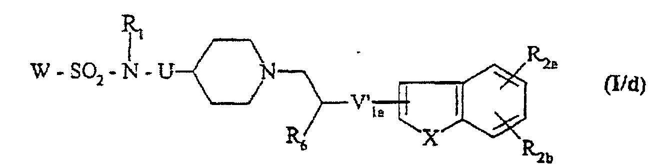 in der W, R₁, U, X, R₂ₐ und R_{2b} die bezüglich der Formel (I) angegebenen Bedeutungen besitzen, V'₁ₐ die oben angegebenen Bedeutungen aufweist und R₆ eine Hydroxygruppe oder ein Halogenatom darstellt,
welche Verbindungen der Formel (I/d) man in dem besonderen Fall, da R₆ eine Hydroxygruppe darstellt, der Einwirkung eines Alkylierungsmittels unter klassischen Bedingungen der organischen Chemie unterwirft, zur Bildung der Verbindungen der Formel (I/e), einem Sonderfall der Verbindungen der Formel (I): 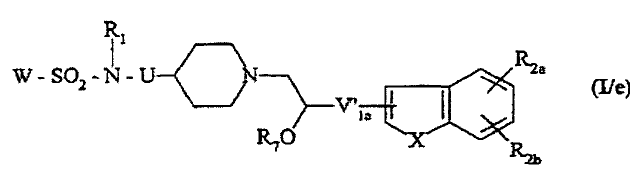 in der W, R₁, U, V'₁ₐ, X, R₂ₐ und R_{2b} die oben angegebenen Bedeutungen besitzen und R₇ eine geradkettige oder verzweigte (C₁-C₆)-Alkylgruppe bedeutet,
welche Verbindungen der Formeln (I/a) bis (I/e), welche die Gesamtheit der erfindungsgemäßen Verbindungen bilden, man gegebenenfalls mit Hilfe klassischer Reinigungsmethoden reinigt, welche gewünschtenfalls mit Hilfe einer klassischen Trennmethode in ihre verschiedenen Isomeren aufgetrennt werden können, bei welchen man die Substituenten R₂ₐ, R_{2b} und jene der Gruppe W unter Anwendung klassischer Methoden der organischen Synthese, wie sie im Bereich der Aromaten-Chemie angewandt werden, moduliert und gewünschtenfalls die Gruppe W in dem Fall, da diese ein bicyclisches System darstellt, reduziert zur Bildung von bicyclischen Gruppen W, bei denen einer der Ringe teilweise hydriert ist, welche Gruppen anschließend unter Anwendung klassischer Bedingungen der organischen Chemie substituiert werden können und welche man gewünschtenfalls in ihre Additionssalze mit einer pharmazeutisch annehmbaren Säure oder Base überführt und welche gegebenenfalls in Form der Hydrate oder Solvate vorliegen können.

11. Pharmazeutische Zubereitungen, enthaltend als Wirkstoff mindestens eine Verbindung der Formel (I) nach einem der Ansprüche 1 bis 9 allein oder in Kombination mit einem oder mehreren inerten, nichttoxischen, pharmazeutisch annehmbaren Trägermaterialien oder Bindemitteln.

12. Pharmazeutische Zubereitungen nach Anspruch 11, enthaltend mindestens einen Wirkstoff nach einem der Ansprüche 1 bis 9, nützlich als Arzneimittel für die Behandlung von Erkrankungen oder pathologischen Zuständen, bei denen eine endotheliale Dysfunktion bekannt ist.

13. Pharmazeutische Zubereitungen nach Anspruch 11, enthaltend mindestens einen Wirkstoff nach einem der Ansprüche 1 bis 9, nützlich als Arzneimittel zur Vorbeugung der Entwicklung, der Ausbreitung oder von Komplikationen von atherosklerotischen Schädigungen, zur Vorbeugung von Gefäßkomplikationen nach der Anordnung von Bypassen, der Gefäßdilatation, der Gefäßrepermeabilisierung und von Herztransplantationen, oder zur Behandlung von myokardischer oder peripherer Ischämie, von Herzinsuffizienz und arterieller Lungenhypertension.

## Claims

1. Compounds of formula (I) : 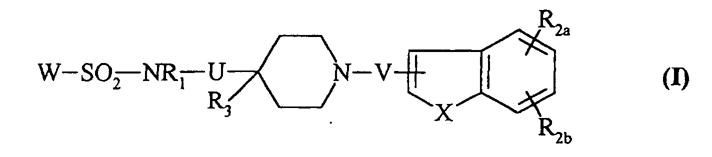 wherein :
**R**_{**1**} represents a hydrogen atom or a linear or branched (C₁-C₆)alkyl group,
**R**_{**2a**} and **R**_{**2b**}**,** which may be identical or different, each independently of the other represents a group selected from a hydrogen atom, a halogen atom, a linear or branched (C₁-C₆)alkyl group, a hydroxy group, a linear or branched (C₁-C₆)alkoxy group, a linear or branched (C₁-C₆)trihaloalkyl group, a cyano group, a nitro group, an amino group, a linear or branched (C₁-C₆)alkylamino group, and a di-(C₁-C₆)alkyl-amino group in which each alkyl moiety is linear or branched,
**R**_{**3**} represents a hydrogen atom or a hydroxy group,
**X** represents an oxygen atom or a methylene group,
**V** represents a linear or branched (C₁-C₆)alkylene chain optionally containing one or more unsaturated bonds and being optionally substituted by one or more identical or different groups selected from halogen atoms, hydroxy groups and linear or branched (C₁-C₆)alkoxy groups,
**U** represents a bond or a linear or branched (C₁-C₆)alkylene chain,
**W** represents a group selected from aryl and heteroaryl, each of those groups being optionally substituted by one or more identical or different groups selected from halogen atoms, linear or branched (C₁-C₆)alkyl groups, hydroxy groups, linear or branched (C₁-C₆)alkoxy groups, linear or branched (C₁-C₆)trihaloalkyl groups, oxo groups, cyano groups, nitro groups, amino groups, linear or branched (C₁-C₆)-alkylamino groups, di-(C₁-C₆)alkylamino groups in which each alkyl moiety is linear or branched, pyridyl groups, linear or branched (C₁-C₆)alkylcarbonyl groups, aminocarbonyl groups (the amino moiety being optionally substituted by one or two identical or different linear or branched (C₁-C₆)alkyl groups), linear or branched (C₁-C₆)alkoxycarbonyl groups, linear or branched (C₁-C₆)trihaloalkylcarbonyl groups, linear or branched (C₁-C₆)alkylsulphonyl groups, and linear or branched (C₁-C₆)-trihaloalkylsulphonyl groups,
their isomers, their hydrates, their solvates and addition salts thereof with a pharmaceutically acceptable acid or base,
it being understood that :
- "aryl group" is understood to mean a group selected from phenyl, biphenyl, naphthyl, dihydronaphthyl, tetrahydronaphthyl, indanyl and indenyl,
- "heteroaryl group" is understood to mean a monocyclic aromatic or bicyclic system having from 5 to 12 ring members and containing from 1 to 3 identical or different hetero atoms selected from oxygen, nitrogen and sulphur, and in the case of a bicyclic system one of the rings has an aromatic character, it being possible for the other ring to be aromatic or partially hydrogenated.

2. Compounds of formula (I) according to claim 1, **characterised in that** X represents an oxygen atom, their isomers, their hydrates, their solvates and addition salts thereof with a pharmaceutically acceptable acid.

3. Compounds of formula (I) according to claim 1, **characterised in that** R₁ represents a hydrogen atom or a methyl group, their isomers, their hydrates, their solvates and addition salts thereof with a pharmaceutically acceptable acid.

4. Compounds of formula (I) according to any one of claims 1 to 3, **characterised in that** they represent compounds of formula **(I/A)** : 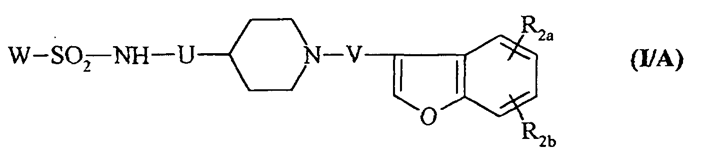 wherein W, U, V, R₂ₐ and R_{2b} are as defined for formula (I), their isomers, their hydrates, their solvates and addition salts thereof with a pharmaceutically acceptable acid.

5. Compounds of formula (I) according to claim 1, **characterised in that** W represents an isoquinolin-5-yl group, their isomers, their hydrates, their solvates and addition salts thereof with a pharmaceutically acceptable acid.

6. Compounds of formula (I) according to claim 1, **characterised in that** W represents a 1,2,3,4-tetrahydro-isoquinolin-5-yl group optionally substituted in the 2-position by a group of formula -C(O)-A wherein A represents a group selected from linear or branched (C₁-C₆)alkyl, amino (itself optionally substituted by one or two identical or different linear or branched (C₁-C₆)alkyl groups), linear or branched (C₁-C₆)alkoxy, trifluoromethyl, linear or branched (C₁-C₆)alkylsulphonyl, and trifluoromethylsulphonyl, their isomers, their hydrates, their solvates and addition salts thereof with a pharmaceutically acceptable acid.

7. Compounds of formula (I) according to claim 4, **characterised in that** they represent compounds of formula (I/A) as defined hereinbefore wherein U, V, R₂ₐ and R_{2b} are as defined for formula (I) and W represents an isoquinolin-5-yl group, their isomers, their hydrates, their solvates and addition salts thereof with a pharmaceutically acceptable acid.

8. Compounds of formula (I) according to claim 4, **characterised in that** they represent compounds of formula (I/A) as defined hereinbefore wherein U, V, R₂ₐ and R_{2b} are as defined for formula (I) and W represents a 1,2,3,4-tetrahydroisoquinolin-5-yl group optionally substituted in the 2-position by a group of formula -C(O)A wherein A is as defined hereinbefore, their isomers, their hydrates, their solvates and addition salts thereof with a pharmaceutically acceptable acid.

9. Compounds of formula (I) according to claim 1 which are :
- *N*-{1-[2-(benzofuran-3-yl)ethyl]-4-piperidyl}-(isoquinolin-5-yl)sulphonamide,
- ethyl 5-{[({(1-[2-(benzofuran-3-yl)ethyl]-4-piperidyl}methyl)-(methyl)amino]-sulphonyl}-3,4-dihydro-2(1*H*)-isoquinolinecarboxylate,
- *N*-({2-[2-benzofuran-3-yl)ethyl]-4-hydroxy-4-piperidyl}ethyl)-5-isoquinoline-sulphonamide,
- *N*-({1-[2-(benzofuran-3-yl)ethyl]-4-hydroxy-4-piperidyl}methyl)-*N*-methyl-2,1,3-benzoxadiazole-4-sulphonamide, and
- isopropyl 5-{[({1-[2-(benzofuran-3-yl)ethyl]-4-piperidyl}methyl)-(methyl)amino]-sulphonyl}-3,4-dihydro-2(1*H*)-isoquinolinecarboxylate.

10. Process for the preparation of compounds of formula (I) according to claim 1, **characterised in that** there is used as starting material a compound of formula **(II)** : 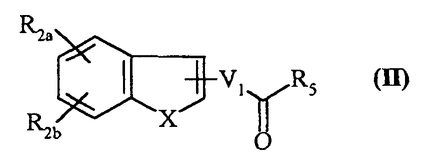 wherein X, R₂ₐ and R_{2b} are as defined for formula (I), V₁ represents a bond or a linear or branched (C₁-C₅)alkylene chain optionally containing one or more unsaturated bonds, R₅ represents a hydrogen atom, a chlorine atom, a hydroxy group or a linear or branched (C₁-C₆)alkoxy group,
which compounds of formula (II) are reacted :
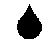 ***either*** with a compound of formula **(III/A)** : 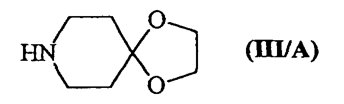 to yield compounds of formula **(IV)** : 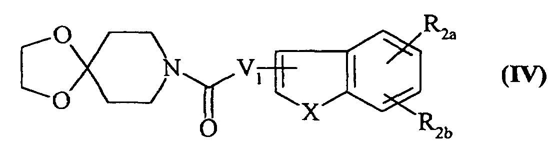 wherein R₂ₐ, R_{2b}, X and V₁ are as defined hereinbefore,
the ketal function of which compounds of formula (IV) is deprotected in accordance with conventional techniques of organic synthesis, and which are then reacted under conditions of reductive amination with a compound of formula **(V)** :
R'₁ - NH₂ **(V)**
wherein R'₁ represents a linear or branched (C₁-C₆)alkyl group,
to yield compounds of formula **(VI/A)** : 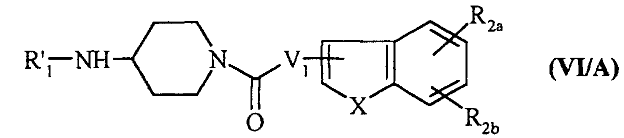 wherein R'₁, R₂ₐ, R_{2b}, X and V₁ are as defined hereinbefore,
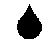 ***or*** with a compound of formula **(III/B)** : 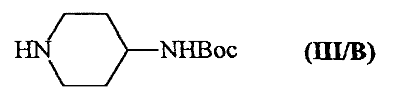 wherein Boc represents a *tert*-butoxycarbonyl group,
to yield compounds of formula **(VII)** : 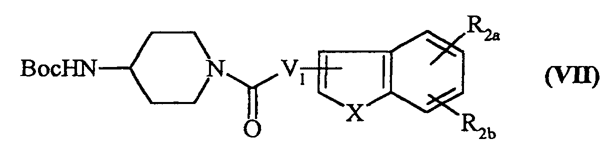 wherein Boc, R₂ₐ, R_{2b}, X and V₁ are as defined hereinbefore, the terminal amine function of which compounds of formula (VII) is deprotected to yield compounds of formula **(VI/B)** : 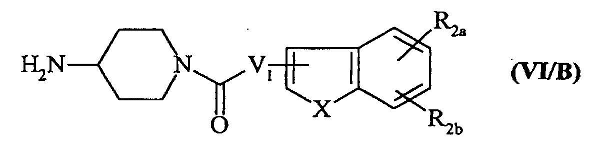 wherein R₂ₐ, R_{2b}, V₁ and X are as defined hereinbefore,
the totality of the compounds of formulae (VI/A) and (VI/B) constituting the compounds of formula **(VI)** : 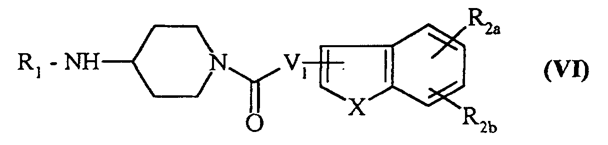 wherein R₁ is as defined for formula (I) and R₂ₐ, R_{2b}, X and V₁ are as defined hereinbefore,
which compounds of formula (VI) are treated with one of the reducing agents currently used in organic synthesis to reduce amide functions, to yield compounds of formula **(VIII) :** 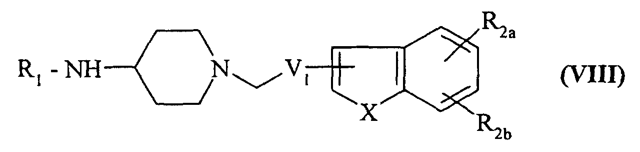 wherein R₁, V₁, X, R₂ₐ and R_{2b} are as defined hereinbefore,
which compounds of formula (VIII) are treated with a compound of formula **(IX)** :
W - SO₂Cl **(IX)**
wherein W is as defined for formula (I), to yield compounds of formula **(I/a)**, a particular case of the compounds of formula (I) : 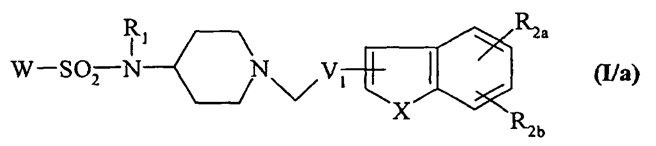 wherein W, R₁, V₁, X, R₂ₐ and R_{2b} are as defined hereinbefore,
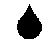 ***or*** with a compound of formula **(III/C)** : 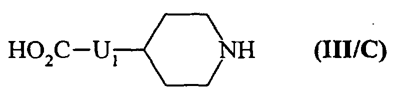 wherein U₁ represents a bond or a linear or branched (C₁-C₅)alkylene chain,
to yield compounds of formula **(X)** : 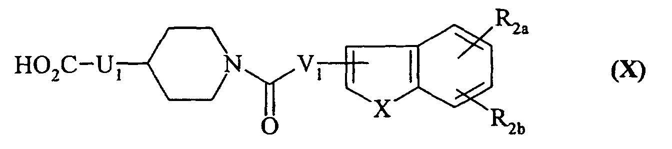 wherein U₁, V₁, X, R₂ₐ and R_{2b} are as defined hereinbefore,
which compounds of formula (X) are treated with ammonium hydroxide or with a compound of formula (V) as defined hereinbefore in the presence of a coupling agent, such as dicyclo-hexylcarbodiimide, carbonyldiimidazole or 1,1,1,3,3,3-hexamethyldisylazane, to yield compounds of formula **(XI)** : 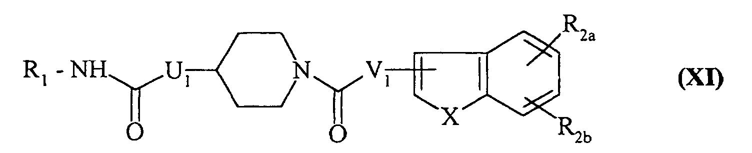 wherein R₁ is as defined for formula (I), and U₁, V₁, X, R₂ₐ and R_{2b} are as defined hereinbefore,
which compounds of formula (XI) are reduced in accordance with conventional conditions of organic synthesis, to yield compounds of formula **(XII)** : 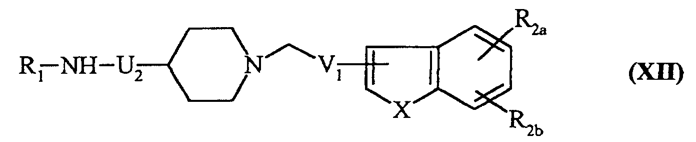 wherein U₂ represents a linear or branched (C₁-C₆)alkylene chain, and R₁, V₁, X, R₂ₐ and R_{2b} are as defined hereinbefore,
which compounds of formula (XII) are reacted with a compound of formula (IX), as described hereinbefore, to yield compounds of formula **(I/b)**, a particular case of the compounds of formula (I) : 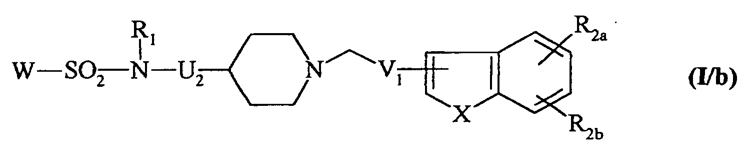 wherein W, R₁, U₂, V₁, X, R₂ₐ and R_{2b} are as defined hereinbefore,
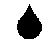 ***or*** the terminal carbonyl group of which compounds of formula (II), which group is substituted by a group R'₅ having the meanings of R₅ as defined hereinbefore with the exception of the meaning "chorine atom", is reduced, the resulting hydroxy group of which is then replaced by a halogen atom, such as Cl, Br or I, using customary techniques of organic chemistry, to yield compounds of formula **(II/B)** : 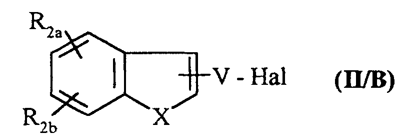 wherein X and V are as defined for formula (I) and Hal represents a halogen atom,
which compound of formula (II/B) is added to a compound of formula **(III/D)** : 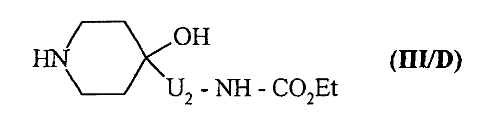 wherein U₂ is as defined hereinbefore,
to yield compounds of formula **(XIII)** : 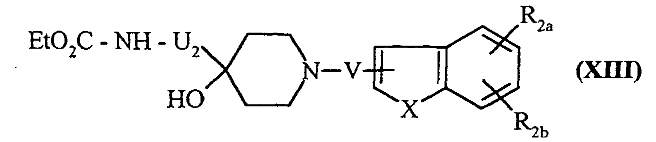 wherein U₂, V, X, R₂ₐ and R_{2b} are as defined hereinbefore,
which compounds of formula (XIII) are treated with a reducing agent or with a strong alkaline base, to yield compounds of formula **(XIV)** : 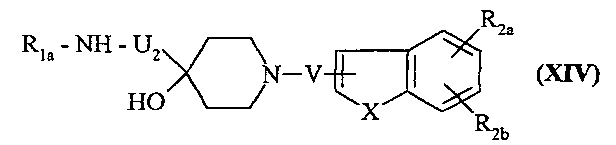 wherein R₁ₐ represents a hydrogen atom or a methyl group, and U₂, V, X, R₂ₐ and R_{2b} are as defined hereinbefore,
which compounds of formula (XIV) are treated with a compound of formula (IX), to yield compounds of formula **(I/c)**, a particular case of the compounds of formula (I) : 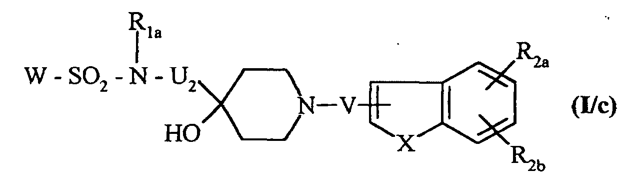 wherein W, R₁ₐ, U₂, V, X, R₂ₐ and R_{2b} are as defined hereinbefore,
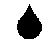 ***or*** which compound of formula (II), in the particular case when R₅ represents a hydrogen atom and V₁ has the meaning V'₁ representing a linear or branched (C₁-C₄)alkylene chain, is treated with trimethylsulphoxonium iodide in the presence of sodium hydride in dimethyl sulphoxide, to yield compounds of formula **(II/C)** : 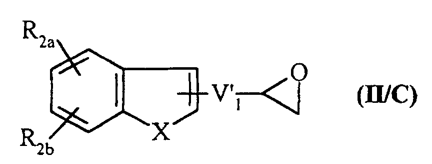 wherein R₂ₐ, R_{2b}, X and V'₁ are as defined hereinbefore,
which compounds of formula (II/C) are reacted with any one of the compounds of formulae (III/A), (III/B) and (III/C), the resulting intermediates then being deprotected and functionalised in accordance with the respective methods described hereinbefore, to yield compounds of formula **(XV)** : 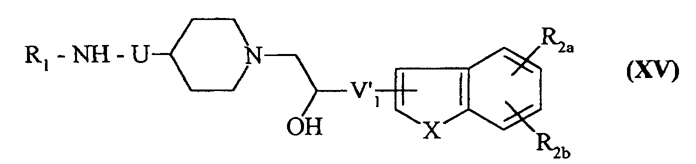 wherein R₁, U, X, R₂ₐ and R_{2b} are as defined hereinbefore and V'₁ is as defined hereinbefore,
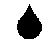 ***or*** which compound of formula (II), in the particular case when it represents a compound of formula **(II/D)** : 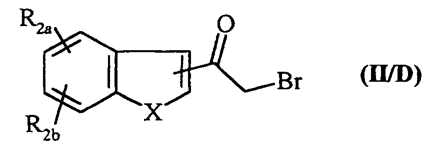 wherein R₂ₐ, R_{2b} and X are as defined for formula (I),
is reacted with any one of the compounds of formulae (III/A), (III/B) and (III/C), the resulting intermediates then being deprotected and functionalised in accordance with the respective methods described hereinbefore,
to yield compounds of formula **(XVI)** : 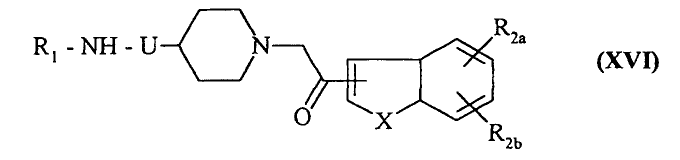 wherein R₁, U, X, R₂ₐ and R_{2b} are as defined hereinbefore,
which compounds of formula (XVI) are reduced in conventional manner to yield compounds of formula **(XVII)** : 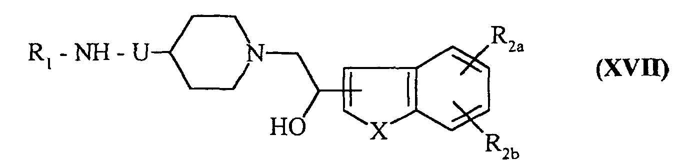 the totality of the compounds of formulae (XV) and (XVII) constituting the compounds of formula **(XVIII)** : 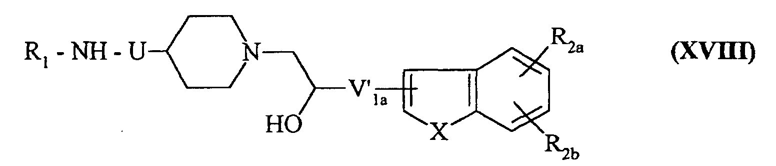 wherein R₁, U, X, R₂ₐ and R_{2b} are as defined hereinbefore, and V'₁ₐ represents a bond or a linear or branched (C₁-C₄)alkylene chain,
the hydroxy function of which compounds of formula (XVIII) is replaced, if desired, by a halogen atom, in accordance with conventional conditions of organic synthesis, to yield compounds of formula (XIX) : 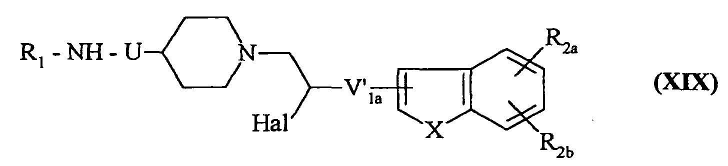 wherein Hal represents a halogen atom, and R₁, U, V'₁ₐ, X, R₂ₐ and R_{2b} are as defined hereinbefore,
the totality of the compounds of formulae (XVIII) and (XIX) being reacted with a compound of formula (IX), as described hereinbefore, to yield compounds of formula **(I/d)**, a particular case of the compounds of formula (I) . 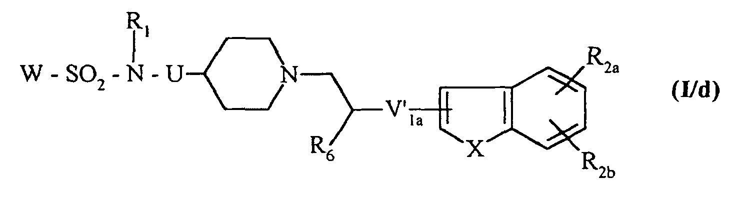 wherein W, R₁, U, X, R₂ₐ and R_{2b} are as defined for formula (I), V'₁ₐ is as defined hereinbefore and R₆ represents a hydroxy group or a halogen atom,
which compounds of formula (I/d), in the particular case when R₆ represents a hydroxy group, are subjected to the action of an alkylating agent in accordance with conventional conditions of organic chemistry, to yield compounds of formula (I/e), a particular case of the compounds of formula (I) : 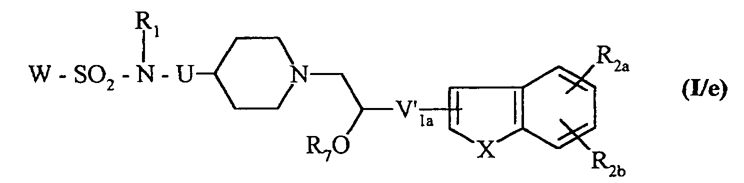 wherein W, R₁, U, V'₁ₐ, X, R₂ₐ and R_{2b} are as defined hereinbefore and R₇ represents a linear or branched (C₁-C₆)alkyl group,
which compounds of formulae (I/a) to (I/e) constitute the totality of the compounds of the invention, which are purified, if necessary, in accordance with conventional purification techniques, which may be separated, if desired, into their different isomers in accordance with a conventional separation technique, the substituents R₂ₐ and R_{2b} of which and those of the group W being transformed in accordance with conventional methods of organic synthesis used in the field of aromatic chemistry, the W group of which, when it is a bicyclic system, is reduced, if desired, to yield bicyclic W groups, one of whose rings is partially hydrogenated and which may then be substituted in accordance with conventional conditions of organic chemistry, and which are converted, if desired, into their addition salts with a pharmaceutically acceptable acid or base, and which may be in the form of hydrates or solvates.

11. Pharmaceutical compositions comprising as active ingredient at least one compound of formula (I) according to any one of claims 1 to 9, alone or in combination with one or more inert non-toxic pharmaceutically acceptable excipients or carriers.

12. Pharmaceutical compositions according to claim 11 comprising at least one active ingredient according to any one of claims 1 to 9, for use as a medicament for the treatment of diseases or pathological conditions in which an endothelial dysfunction is known.

13. Pharmaceutical compositions according to claim 11 comprising at least one active ingredient according to any one of claims 1 to 9, for use as a medicament for preventing the development, extension and complications of atherosclerotic lesions, for preventing vascular complications after vascular bypass, vascular dilatation, vascular repermeabilisation and heart transplantation, or for treating myocardial or peripheral ischaemia, cardiac insufficiency and pulmonary arterial hypertension.
